# EUROPEAN PATENT APPLICATION

(11) **EP 1 489 081 A1**
(43) Date of publication of application: **22.12.2004**
(21) Application number: 03715376.4
(22) Date of filing: 19.03.2003
(51) Int. Cl.: C07D 471/10, A61K 31/438, A61P 1/04, A61P 11/02, A61P 11/06, A61P 13/12, A61P 17/00, A61P 19/02, A61P 25/00, A61P 29/00, A61P 37/02, A61P 37/08, A61P 43/00

(54) **SPIRO DERIVATIVES AND ADHESION MOLECULE INHIBITORS COMPRISING THE SAME AS THE ACTIVE INGREDIENT**

(30) Priority: 22.03.2002 JP 2002080697
(71) Applicant: TORAY INDUSTRIES, INC., Tokyo 103-8666 (JP)
(72) Inventor: ISHIGAKI, Takeshi, Kamakura-shi, Kanagawa 248-0031 (JP); TANIGUCHI, Koji, Kamakura-shi, Kanagawa 248-0034 (JP); KAINOH, Mie, Fujisawa-shi, Kanagawa 251-0052 (JP); MEGURO, Hiroyuki, Kamakura-shi, Kanagawa 248-0036 (JP); ISHIZAKA, Yoko, Yokohama-shi, Kanagawa 233-0015 (JP)
(74) Representative: Kador & Partner
(86) International application number: PCT/JP2003/003324
(87) International publication number: WO 2003/080611

(57) **Abstract**

Disclosed is the use of an adhesion molecule inhibitor that is effective in the prevention and treatment of inflammatory diseases caused by infiltration of leukocytes such as monocytes, lymphocytes and eosinophils, by inhibiting cell infiltration which mediates adhesion molecules, especially adhesion molecule VLA-4.

Since the spiro acid derivatives according to the present invention are excellent in the effect of inhibiting cell adhesion via adhesion molecules, especially adhesion molecule VLA-4, they are useful as therapeutic drugs against various inflammatory diseases. For example, provided are the spiro derivative and the adhesion molecule inhibitor which includes as an active ingredient the spiro derivative as shown by the below formula (18).

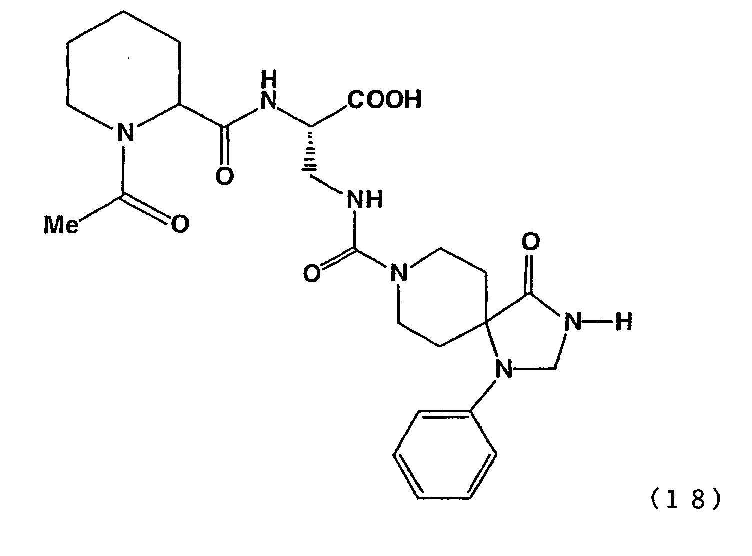

## Description

### Technical Field

The present invention relates to a novel spiro derivative or a pharmaceutically acceptable salt thereof, useful as an adhesion molecule inhibitor, especially VLA-4 inhibitor, to an adhesion molecule inhibitor, especially VLA-4 inhibitor, containing the same as an active ingredient, and to a therapeutic agent against inflammatory diseases containing the same as an active ingredient.

### Background Art

Adhesion molecules are involved in adhesion between cells and between cells and intercellular matrix and migration and activation of cells. Adhesion molecules include a number of families such as integrin family and immunoglobulin superfamily. The adhesion molecules belonging to the integrin family are those expressed on leukocytes such as lymphocytes, monocytes, basophils and eosinophils. These adhesion molecules have a heterodimer structure, in which an α chain and a β chain are non-covalently bound, and are classified into several subfamilies depending on the molecular species of the β chain (Cell, 76, 301 (1994)). VLA-4 (very late antigen-4) also called α4β1, CD49d/CD29, a member of the integrin family, is expressed on lymphocytes, monocytes, eosinophils and mast cells (Ann. Rev. Immunol., 8, 365 (1990)). Both VCAM-1 (vascular cell adhesion molecule-1) present on vascular endothelial cells and the CS-1 region in the type III connective segment of the extracellular matrix fibronectin are known as VLA-4 ligands (Immunol. Today, 14, 506 (1993); Cell, 60, 577 (1990)). It is known that VLA-4 on the leucocytes interacts with these ligands, participating in cell functions such as cell adhesion, extravascular migration or infiltration, differentiation and proliferation (Springer Semin. Immunopathol., 16, 379 (1995)).

The fact that the interaction between VLA-4 and ligands plays an important role in inflammation and immune reaction has been suggested from research using anti-VLA-4 monoclonal antibodies that have function inhibitory activity (Ciba Foundation Symposium, 189, 79 (1995)). Examples of this include investigations which used animal models, such as experimental autoimmune encephalomyelitis (Nature, 356, 63 (1992)), colitis (J. Clin. Invest., 92, 372 (1993)), contact hypersensitivity and delayed hypersensitivity reactions (J. Immunol., 150, 1172 (1993); Eur. J. Immunol., 23 , 682 (1993)), arthritis (J. Clin. Invest., 89, 1445 (1992)), graft versus host disease (J. Immunol., 153, 5810 (1994)), asthma (J. Exp. Med., 180, 795 (1994), nephritis (J. Clin. Invest., 91, 577 (1993)) and immunocomplex-induced pulmonary injury (J. Immunol., 150, 2401 (1993)). Effects from inhibiting VLA-4 were reported in these examples.

It has been reported that the CS-1 region amino acid sequence of the binding site with VLA-4 is 3 amino acid residues (LDV) of Leucine (Leu)-Aspartic acid (Asp)-Valine (Val) (J. Cell Biol., 124, 601 (1994)). It has been reported that the CS-1 peptide or LDV derivative are effective against asthma or arthritis models by inhibiting ligands binding with VLA-4 in the same manner as that of the above-described antibodies (J. Clin. Invest., 94, 655 (1994); Proc. Natl. Acad. Sci. USA, 88, 9072 (1991)). Further, it has been revealed from the results of mutation of VCAM-1 that the binding sites on VCAM-1 for binding to VLA-4 are present in domains 1 and 4, of which it is clear that the glutamine (Gln)-isolucine (IIe)-aspartic acid (Asp)-serine (Ser)-proline (Pro)-leucine (Leu) amino acid sequence on the CD loop is important for the binding to VLA-4 (J. Cell Biol., 125, 1395 (1994); J. Cell Biol., 124, 601 (1994); J. Cell Biol., 125, 215 (1994); J. Cell Science, 107, 2127 (1994)). J. H. Wang et al. have reported a cyclic peptide Cys*GlnIIeAspSerProCys* (wherein Cys*Cys* represents disulfide bond) which has adhesion inhibitory activity against VLA-4, which cyclic peptide is based on the glutamine-isolucine-aspartic acid-serine-proline (Proc. Natl. Acad. Sci. USA, 92, 5714 (1995)). However, generally peptide mimetics are known to be unstable in vivo, making a non-peptide low molecular weight VLA-4 inhibition compound desirable.

In addition, several reports have been made regarding low molecular weight compounds showing VLA-4 inhibitory activity (for example, WO 01/68586, WO 01/56994, WO 01/55121, JP-A-2001-163802, JP-A-2001-89368).

However, known low molecular weight compounds showing VLA-4 inhibitory activity do not have sufficient activity, making a compound which has a novel skeleton and shows high VLA-4 inhibitory activity desirable.

### Disclosure of the Invention

It has been proved that the cause of development of chronic inflammatory diseases such as allergic inflammation and rheumatoid arthritis is the repetition of excessive accumulation of leukocytes at the inflammatory site. However, conventional therapy for these diseases uses drugs that have an inhibitory effect on the action of chemical mediators, drugs that have a suppressing effect on the production of chemical mediators, or drugs that have an inhibitory effect on the production of active oxygen. Drugs are also used that suppress activation of leukocytes, such as steroids. Since these drugs do not have an effect to suppress the process of accumulation of leukocytes to the inflammatory site as their main action, they cannot inhibit progress of inflammation. In contrast, since adhesion molecules VLA-4 and VCAM-1 mainly participate in the process of accumulation of leukocytes to an inflammatory site, a novel compound having an activity to inhibit the adhesion of VLA-4 and VCAM-1 is thought to inhibit accumulation of leukocytes to an inflammatory site. Thus, the probability that such a compound is an effective therapeutic drug against the above-mentioned diseases is high.

An object of the present invention is to discover a compound which inhibits cell infiltration via adhesion molecules, especially, adhesion molecule VLA-4, thereby making it possible to prevent and treat inflammatory diseases caused by infiltration of leukocytes such as monocytes, lymphocytes and eosinophils.

As a result of intensive study, the present inventors discovered that specific novel spiro derivatives and pharmaceutically acceptable salts thereof have activities to inhibit cell adhesion via adhesion molecules, especially adhesion molecule VLA-4, thereby completing the present invention.

That is, the present invention encompasses the following invention.
(1) A spire derivative or a pharmaceutically acceptable salt thereof represented by Formula I, 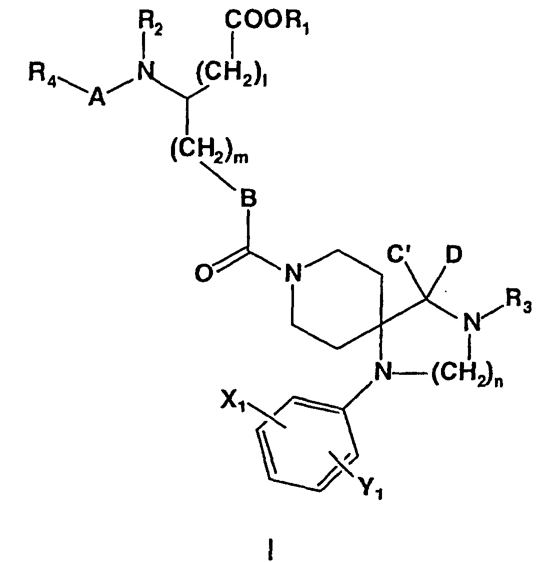 wherein l and m each independently represent an integer of 0 to 2;
   n represents an integer of 1 to 3;
   A represents -C(O)- or -S(O)₂-;
   B represents -CH₂- or -NH-;
   C' and D both represent a hydrogen atom, or C' and D represent together =O;
   X₁ and Y₁ independently represent hydrogen, halogen, C₁₋₈ alkyl, trifluoromethyl, C₁₋₈ alkoxy, cyano, nitro, hydroxyl, amino, or tetrazolyl;
   R₁ represents hydrogen, C₁₋₆ linear alkyl, C₃₋₈ branched alkyl, benzyl or -CH₂OC(O)C(CH₃)₃;
   R₂ represents hydrogen or C₁₋₆ linear alkyl;
   R₃ represents hydrogen, C₁₋₆ linear alkyl, C₃₋₈ branched alkyl; allyl, homoallyl, C₃₋₈ cycloalkyl-C₁₋₈ alkyl, unsubstituted phenyl or phenyl substituted with at least one substituent of substituent group E, unsubstituted benzyl or benzyl substituted with at least one substituent of substituent group E, unsubstituted phenethyl or phenethyl substituted with at least one substituent of substituent group E, unsubstituted styryl or styryl substituted with at least one substituent of substituent group E, unsubstituted naphthyl or naphthyl substituted with at least one substituent of substituent group E, or unsubstituted naphthylmethyl or naphthylmethyl substituted with at least one substituent of substituent group E (wherein substituent group E consists of halogen, C₁₋₈ alkyl, C₁₋₈ alkoxy, trifluoromethyl, trifluoromethoxy, C₁₋₈ alkylthio, cyano, nitro, hydroxyl, amino, C₁₋₈ alkylacyl, C₁₋₈ alkylacylamino and tetrazolyl);
   R₄ represents C₁₋₈ alkyl, C₂₋₈ alkenyl, C₂₋₈ alkynyl, Cy, Cy-C₁₋₈ alkyl, Cy-C₂₋₈ alkenyl, Cy-C₂₋₈ alkynyl, Ar, Ar-C₁₋₈ alkyl, Ar-C₂₋₈ alkenyl or Ar-C₂₋₈ alkynyl, wherein the alkyl, alkenyl and alkynyl may be linear or branched, and may be substituted with 1 to 4 independently selected substituents of R₅, wherein
   Cy is C₃₋₈ cycloalkyl that may be substituted with 1 to 4 substituents of R₆ or 3- to 8-membered monocyclic or bicyclic heterocycle that includes 1 to 4 nitrogen atoms, oxygen atoms or sulfur atoms independently selected, which heterocycle may be substituted with 1 to 4 substituents of R₆ (with the proviso that the hetero atoms do not bond directly with A),
   Ar is phenyl that may be substituted with 1 to 5 substituents of R₇, naphthyl that may be substituted with 1 to 5 substituents of R₇, or 5- to 8-membered monocyclic or bicyclic heteroaryl that includes 1 to 4 nitrogen atoms, oxygen atoms or sulfur atoms independently selected, which heterocycle may be substituted with 1 to 5 substituents of R₇ (wherein the hetero atoms do not directly bond with A),
   R₅ is halogen, trifluoromethyl, -OR^{a} or -SR^{a},
   R^{a} is hydrogen, C₁₋₈ alkyl, allyl, homoallyl, trifluoromethyl, phenyl or benzyl,
   R₆ and R₇ are, each independently, R₅, C₁₋₈ alkyl, C₂₋₈ alkenyl, C₂₋₈ alkynyl, C₃₋₈ cycloalkyl-C₁₋₈ alkyl, cyano, nitro, =O, -SO₂R^{b}, -SO₂NR^{c}R^{d}, -C(O)R^{b}, -C(O)OR^{b}, -C(O)NR^{c}R^{d}, -NR^{c}R^{d}, -NR^{c}C(O)R^{b}, -NR^{c}SO₂R^{b}, unsubstituted phenyl or phenyl substituted with at least one substituent of substituent group E or unsubstituted benzyl or benzyl substituted with at least one substituent of substituent group E,
   R^{b}, R^{c} and R^{d} are each independent, and are hydrogen, C₁₋₈ alkyl, C₃₋₈ cycloalkyl, C₃₋₈ cycloalkyl-C₁₋₈ alkyl, unsubstituted phenyl or phenyl substituted with at least one substituent of substituent group E, unsubstituted benzyl or benzyl substituted with at least one substituent of substituent group E, unsubstituted phenethyl or phenethyl substituted with at least one substituent of substituent group E, unsubstituted styryl or styryl substituted with at least one substituent of substituent group E, unsubstituted naphthyl or naphthyl substituted with at least one substituent of substituent group E, or unsubstituted naphthylmethyl or naphthylmethyl substituted with at least one substituent of substituent group E
   (with the proviso that excluded are the compounds in which, when A is -C(O)-, R₄ is C₁₋₇ linear alkyl, C₃₋₉ branched alkyl, adamantyl, benzyl or phenethyl substituted with 0 to 2 substituents of halogen, C₁₋₈ alkyl, C₁₋₈ alkoxy, cyano, nitro, hydroxyl, amino, or tetrazolyl;
   Formula II, 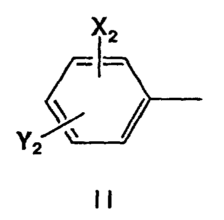 wherein X₂ and Y₂ each independently represent hydrogen, halogen, alkyl containing 1 to 8 carbon atoms, alkoxy containing 1 to 8 carbon atoms, cyano, nitro, hydroxyl, amino, or tetrazolyl; or
   Formula III 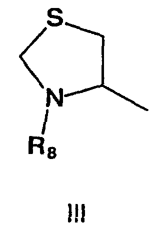 wherein R₈ represents linear alkyl containing 1 to 6 carbon atoms, branched alkyl containing 3 to 8 carbon atoms, linear alkylacyl containing 1 to 6 carbon atoms, branched alkylacyl containing 3 to 8 carbon atoms, cycloalkylacyl containing 5 to 7 carbon atoms, linear alkylsulfonyl containing to 6 carbon atoms or branched alkylsulfonyl containing 3 to 8 carbon atoms, or
   benzoyl substituted with 0 to 2 substituents of halogen, alkyl containing 1 to 8 carbon atoms, alkoxy containing 1 to 8 carbon atoms, cyano, nitro, hydroxyl, amino or tetrazolyl, phenylsulfonyl substituted with 0 to 2 substituents of halogen, alkyl containing 1 to 8 carbon atoms, alkoxy containing 1 to 8 carbon atoms, cyano, nitro, hydroxyl, amino or tetrazolyl, benzyl substituted with 0 to 2 substituents of halogen, alkyl containing 1 to 8 carbon atoms, alkoxy containing 1 to 8 carbon atoms, cyano, nitro, hydroxyl, amino or tetrazolyl).
(2) A spiro derivative or a pharmaceutically acceptable salt thereof represented by Formula I, 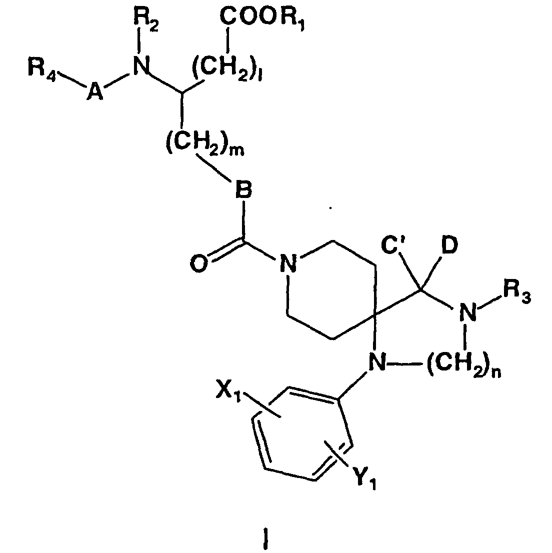 wherein I and m each independently represent an integer of 0 to 2;
   n represents an integer of 1 to 3;
   A represents -C(O)- or -S(O)₂-;
   B represents -CH₂- or -NH-;
   C' and D both represent a hydrogen atom, or C' and D represent together =O;
   X₁ and Y₁ independently represent hydrogen, halogen, C₁₋₈ alkyl, trifluoromethyl, C₁₋₈ alkoxy, cyano, nitro, hydroxyl, amino, or tetrazolyl;
   R₁ represents hydrogen, C₁₋₆ linear alkyl, C₃₋₈ branched alkyl, benzyl or -CH₂OC(O)C(CH₃)₃;
   R₂ represents hydrogen or C₁₋₆ linear alkyl;
   R₃ represents hydrogen, C₁₋₆ linear alkyl, C₃₋₈ branched alkyl, allyl, homoallyl, C₆₋₁₀ cycloalkylalkyl, unsubstituted phenyl or phenyl substituted with at least one substituent of substituent group E, unsubstituted benzyl or benzyl substituted with at least one substituent of substituent group E, unsubstituted phenethyl or phenethyl substituted with at least one substituent of substituent group E, unsubstituted styryl or styryl substituted with at least one substituent of substituent group E, or unsubstituted naphthylmethyl or naphthylmethyl substituted with at least one substituent of substituent group E (wherein substituent group E consists of halogen, C₁₋₈ alkyl, C₁₋₈ alkoxy, trifluoromethyl, trifluoromethoxy, C₁₋₈ alkylthio, cyano, nitro, hydroxyl, amino, C₁₋₈ alkylacyl, C₁₋₈ alkylacylamino and tetrazolyl);
   R₄ represents C₃₋₈ cycloalkyl that may be substituted with 1 to 4 substituents of R₆ or 3- to 8-membered monocyclic or bicyclic heterocycle that includes 1 to 4 nitrogen atoms or oxygen atoms independently selected, which heterocycle may be substituted with 1 to 4 substituents of R₆ (with the proviso that the hetero atoms do not bond directly with A), tetrahydrothiophene or tetrahydrothiopyran that may be substituted with 1 to 4 substituents of R₆, phenyl that may be substituted with 3 to 5 substituents of R₇, naphthyl that may be substituted with 1 to 4 substituents of R₇, 5- to 8-membered monocyclic or bicyclic heteroaryl that includes 1 to 4 nitrogen atoms, oxygen atoms or sulfur atoms independently selected, which heterocycle may be substituted with 1 to 4 substituents of R₇, C₃₋₈ cycloalkyl-C₁₋₈ alkyl, or 3- to 8-membered monocyclic heterocycle-C₁₋₈ alkyl, the heterocycle including 1 to 4 nitrogen atoms or oxygen atoms independently selected and which may be substituted with 1 to 4 substituents of R₆, wherein
   R₆ and R₇ are, independently, halogen, trifluoromethyl, -OR^{a}, -SR^{a}, C₁₋₈ alkyl, C₂₋₈ alkenyl, C₂₋₈ alkynyl, C₃₋₈ cycloalkyl-C₁₋₈ alkyl, cyano, nitro, =O, -SO₂R^{b}, -SO₂NR^{c}R^{d}, -C(O)R^{b}, -C(O)OR^{b}, -C(O)NR^{c}R^{d}, -NR^{c}R^{d}, -NR^{c}C(O)R^{b}, -NR^{c}SO₂R^{b}, unsubstituted phenyl or phenyl substituted with at least one substituent of substituent group E, or unsubstituted benzyl or benzyl substituted with at least one substituent of substituent group E,
   R^{a} is hydrogen, C₁₋₈ alkyl, allyl, homoallyl, trifluoromethyl, phenyl or benzyl,
   R^{b}, R^{c} and R^{d} are each independent, and are hydrogen, C₁₋₈ alkyl, C₃₋₈ cycloalkyl, C₃₋₈ cycloalkyl-C₁₋₈ alkyl, unsubstituted phenyl or phenyl substituted with at least one substituent of substituent group E, unsubstituted benzyl or benzyl substituted with at least one substituent of substituent group E, unsubstituted phenethyl or phenethyl substituted with at least one substituent of substituent group E, unsubstituted styryl or styryl substituted with at least one substituent of substituent group E, unsubstituted naphthyl or naphthyl substituted with at least one substituent of substituent group E, or unsubstituted naphthylmethyl or naphthylmethyl substituted with at least one substituent of substituent group E.
(3) A spiro derivative or a pharmaceutically acceptable salt thereof, wherein in the above-described Formula I,
   l, m, n, B, C', D, X₁, Y₁, R₁, R₂, and R₃ are defined in the same manner as in the above-described (2),
   A represents -C(O)- or -S(O)₂-,
   R₄ represents C₃₋₈ cycloalkyl that may be substituted with 1 to 4 substituents of R₆ (wherein R₆ is defined in the same manner as in the above-described (2)), phenyl that may be substituted with 3 to 5 substituents of R₇ (wherein R₇ is defined in the same manner as in the above-described (2)), naphthyl that may be substituted with 1 to 4 substituents of R₇ (wherein R₇ is defined in the same manner as in the above-described (2)), Formula IV that may be substituted with 1 to 4 substituents of R₆, 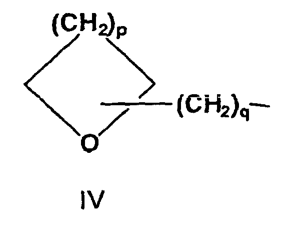 (wherein R₆ is defined in the same manner as in the above-described (2), and p represents an integer of 0 to 5 and q represents an integer of 0 to 2), Formula V that may be substituted with 1 to 4 substituents of R₆, 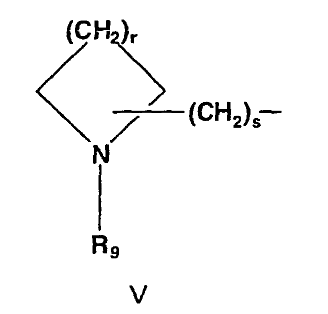 (wherein R₆ is defined in the same manner as in the above-described (2), r represents an integer of 0 to 5 and s represents an integer of 0 to 2, R₉ represents hydrogen, C₁₋₈ alkyl, -SO₂R^{b}, -SO₂NR^{c}R^{d}, -C(O)R^{b}, -C(O)OR^{b}, -C(O)NR^{c}R^{d}*,* unsubstituted phenyl or phenyl substituted with at least one substituent of substituent group E, unsubstituted benzyl or benzyl substituted with at least one substituent of substituent group E, unsubstituted phenethyl or phenethyl substituted with at least one substituent of substituent group E, unsubstituted styryl or styryl substituted with at least one substituent of substituent group E, unsubstituted naphthyl or naphthyl substituted with at least one substituent of substituent group E, or unsubstituted naphthylmethyl or naphthylmethyl substituted with at least one substituent of substituent group E, and R^{b}, R^{c}, R^{d} and substituent group E are defined in the same manner as in the above-described (2)), or Formula VI that may be substituted with 1 to 4 substituents of R₆, 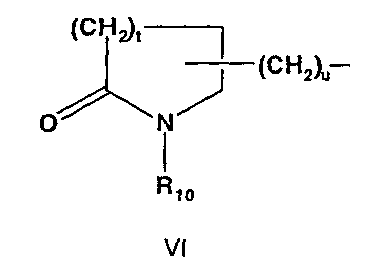 (wherein R₆ is defined in the same manner as in the above-described (2), t represents an integer of 0 to 4 and u represents an integer of 0 to 2, R₁₀ represents hydrogen, C₁₋₈ alkyl, unsubstituted phenyl or phenyl substituted with at least one substituent of substituent group E, unsubstituted benzyl or benzyl substituted with at least one substituent of substituent group E, unsubstituted phenethyl or phenethyl substituted with at least one substituent of substituent group E, unsubstituted styryl or styryl substituted with at least one substituent of substituent group E, unsubstituted naphthyl or naphthyl substituted with at least one substituent of substituent group E, or unsubstituted naphthylmethyl or naphthylmethyl substituted with at least one substituent of substituent group E, and substituent group E is defined in the same manner as in the above-described (2)).
(4) A spire derivative or a pharmaceutically acceptable salt thereof, wherein in the Formula I, l, m, n, A, B, C', D, X₁, Y₁, R₁, R₂, and R₃ are defined in the same manner as in the above-described (2), and R₄ represents Formula IV, Formula V or Formula VI (wherein Formula IV, Formula V and Formula VI are defined in the same manner as in the above-described (3)).
(5) A spiro derivative or a pharmaceutically acceptable salt thereof, wherein in the Formula I,
   l, m, n, A, B, C', D, X₁, Y₁, R₁, R₂, and R₃ are defined in the same manner as in the above-described (2), and
   R₄ represents Formula IV (wherein p and q are defined in the same manner as in the above-described (3)), Formula V (wherein r and s are defined in the same manner as in the above-described (3), R₉ represents hydrogen, C₁₋₈ alkyl, -SO₂R^{b}, or -C(O)R^{b}, R^{b} represents C₁₋₈ alkyl, substituted phenyl or phenyl substituted with at least one substituent of substituent group E, substituted benzyl or benzyl substituted with at least one substituent of substituent group E, substituent group E being defined in the same manner as in the above-described (2)), or Formula VI (wherein t and u are defined in the same manner as in the above-described (3),
   R₁₀ represents hydrogen, C₁₋₈ alkyl, substituted phenyl or phenyl substituted with at least one substituent of substituent group E, substituted benzyl or benzyl substituted with at least one substituent of substituent group E, substituent group E being defined in the same manner as in the above-described (2)).
(6) The spiro derivative or pharmaceutically acceptable salt thereof according to any of the above-described (1) to (5), wherein in Formula I, A is -C(O)-.
(7) The spiro derivative or pharmaceutically acceptable salt thereof according to any of the above-described (1) to (6), wherein in Formula I, B is -NH-.
(8) The spiro derivative or pharmaceutically acceptable salt thereof according to any of the above-described (1) to (7), wherein in Formula I, C' and D represent together =O.
(9) The spiro derivative or pharmaceutically acceptable salt thereof according to any of the above-described (1) to (8), wherein in Formula I, X₁ and Y₁ are both hydrogen.
(10) The spiro derivative or pharmaceutically acceptable salt thereof according to any of the above-described (1) to (9), wherein in Formula I, n is 1.
(11) The spiro derivative or pharmaceutically acceptable salt thereof according to any of the above-described (1) to (10), wherein in Formula I, l is 0.
(12) The spiro derivative or pharmaceutically acceptable salt thereof according to any of the above-described (1) to (11), wherein in Formula I, m is 1 or 2.
(13) The spiro derivative or pharmaceutically acceptable salt thereof according to any of the above-described (1) to (12), wherein in Formula I, R₃ is hydrogen, C₁₋₆ linear alkyl, C₃₋₈ branched alkyl or benzyl.
(14) The spiro derivative or pharmaceutically acceptable salt thereof according to any of the above-described (1) to (13), wherein in Formula I, R₁ is hydrogen or C₁₋₆ linear alkyl.
(15) An adhesion molecule inhibitor comprising the spiro derivative or pharmaceutically acceptable salt thereof according to any of the above-described (1) to (14) as an active ingredient.
(16) The adhesion molecule inhibitor according to the above-described (15), wherein the adhesion molecule is integrin family.
(17) The adhesion molecule inhibitor according to the above-described (16), wherein the integrin family is VLA-4.
(18) A drug comprising the spiro derivative or pharmaceutically acceptable salt thereof according to any of the above-described (1) to (14) as an active ingredient.
(19) An inflammatory disease therapeutic agent comprising the spiro derivative or pharmaceutically acceptable salt thereof according to any of the above-described (1) to (14) as an active ingredient.
(20) The inflammatory disease therapeutic agent according to the above-described (19), wherein the inflammatory disease is an allergic disease or an autoimmune disease.
(21) The inflammatory disease therapeutic agent according to the above-described (20), wherein the allergic disease is asthma, rhinitis or dermatitis.
(22) The inflammatory disease therapeutic agent according to the above-described (20),
wherein the autoimmune disease is multiple sclerosis, ulcerative colitis, arthritis or nephritis.

Furthermore, the present invention provides a method for inhibiting an adhesion molecule, comprising administering an effective amount of the spiro derivative or a pharmaceutically acceptable salt thereof according to the present invention to a subject. The present invention further provides a use of the spiro derivative or a pharmaceutically acceptable salt thereof according to the present invention for the production of a pharmaceutical. The present invention still further provides a use of the spiro derivative or a pharmaceutically acceptable salt thereof according to the present invention for the production of an adhesion molecule inhibitor.

According to the present invention, a novel substance is provided which inhibits cell infiltration via adhesion molecules, especially, adhesion molecule VLA-4, thereby making it possible to prevent and treat inflammatory diseases caused by infiltration of leukocytes such as monocytes, lymphocytes and eosinophils.

As described above, the spiro derivative according to the present invention is represented by the general formula I.

l and m each independently represent an integer of 0 to 2.

n represents an integer of 1 to 3.

A represents -C(O)- or -S(O)₂-.

B represents -CH₂- or -NH-.

C' and D both represent a hydrogen atom, or C' and D represent together =O.

X₁ and Y₁ independently represent hydrogen, halogen (fluorine, chlorine, bromine or iodine), C₁₋₈ alkyl (methyl, ethyl, n-propyl, 1-methylethyl and the like), trifluoromethyl, C₁₋₈ alkoxy (methoxy, ethoxy, n-propoxy, 1-methylethoxy and the like), cyano, nitro, hydroxyl, amino, or tetrazolyl (for example, 5-tetrazolyl and 1-tetrazolyl).

R₁ represents hydrogen, C₁₋₆ linear alkyl (methyl, ethyl, n-propyl, n-butyl, n-pentyl or n-hexyl), C₃₋₈ branched alkyl (1-methylethyl, 1-methylpropyl, 2-methylpropyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 3,5-dimethylhexyl, 3,6-dimethylhexyl, 4,5-dimethylhexyl and the like), benzyl or -CH₂OC(O)C(CH₃)₃.

R₂ represents hydrogen or C₁₋₆ linear alkyl (methyl, ethyl, n-propyl, n-butyl, n-pentyl or n-hexyl).

R₃ represents hydrogen, C₁₋₆ linear alkyl (methyl, ethyl, n-propyl, n-butyl, n-pentyl or n-hexyl), C₃₋₈ branched alkyl (1-methylethyl, 1-methylpropyl, 2-methylpropyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 3,5-dimethylhexyl, 3,6-dimethylhexyl, 4,5-dimethylhexyl and the like), allyl, homoallyl, C₃₋₈ cycloalkyl-C₁₋₈ alkyl (cyclopropylmethyl, cyclopropylethyl, cyclopropylpropyl, cyclopropylbutyl, cyclopropylpentyl, cyclopropylhexyl, cyclobutylmethyl, cyclobutylethyl, cyclobutylpropyl, cyclobutylbutyl, cyclopentylmethyl, cyclopentylethyl, cyclopentylpropyl, cyclopentylbutyl, cyclohexylmethyl, cyclohexylethyl, cyclohexylpropyl, cyclohexylbutyl, cycloheptylmethyl, cyclooctylmethyl and the like), unsubstituted phenyl or phenyl substituted with at least one substituent of substituent group E (phenyl, 2-methylphenyl, 2-cyanophenyl, 2-hydroxyphenyl, 2-chlorophenyl, 2-nitrophenyl, 2-aminophenyl, 2-bromophenyl, 2-fluorophenyl, 2-tetrazolylphenyl, 2-trifluoromethylphenyl, 2-methylthiophenyl, 2-acetylphenyl, 2-acetylaminophenyl, 3-methylphenyl, 3-cyanophenyl, 3-hydroxyphenyl, 3-chlorophenyl, 3-nitrophenyl, 3-aminophenyl, 3-bromophenyl, 3-fluorophenyl, 3-tetrazolylphenyl, 3-trifluoromethylphenyl, 3-methylthiophenyl, 3-acetylphenyl, 3-acetylaminophenyl, 4-methylphenyl, 4-cyanophenyl, 4-hydroxyphenyl, 4-chlorophenyl, 4-nitrophenyl, 4-aminophenyl, 4-bromophenyl, 4-fluorophenyl, 4-tetrazolylphenyl, 4-trifluoromethylphenyl, 4-methylthiophenyl, 4-acetylphenyl, 4-acetylaminophenyl, 2,6-dihydroxyphenyl, 2,6-dimethoxyphenyl, 2,6-dichlorophenyl, 2,6-dinitrophenyl, 2,6-dimethylphenyl and the like), unsubstituted benzyl or benzyl substituted with at least one substituent of substituent group E (benzyl, 2-cyanobenzyl, 2-hydroxybenzyl, 2-chlorobenzyl, 2-nitrobenzyl, 2-aminobenzyl, 2-bromobenzyl, 2-fluorobenzyl, 2-tetrazolylbenzy], 2-trifluoromethylbenzyl, 2-methylthiobenzyl, 2-acetylbenzyl, 2-acetylaminobenzyl, 3-cyanobenzyl, 3-hydroxybenzyl, 3-chlorobenzyl, 3-nitrobenzyl, 3-aminobenzyl, 3-bromobenzyl, 3-fluorobenzyl, 3-tetrazolylbenzyl, 3-trifluoromethylbenzyl, 3-methylthiobenzyl, 3-acetylbenzyl, 3-acetylaminobenzyl, 4-cyanobenzyl, 4-hydroxybenzyl, 4-chlorobenzyl, 4-nitrobenzyl, 4-aminobenzyl, 4-bromobenzyl, 4-fluorobenzyl, 4-tetrazolylbenzyl, 4-trifluoromethylbenzyl, 4-methylthiobenzyl, 4-acetylbenzyl, 4-acetylaminobenzyl, 2,6-dihydroxybenzyl, 2,6-dimethoxybenzyl, 2,6-dichlorobenzyl, 2,6-dinitrobenzyl, 2,6-dimethylbenzyl and the like), unsubstituted phenethyl or phenethyl substituted with at least one substituent of substituent group E (phenethyl, 2-cyanophenethyl, 2-hydroxyphenethyl, 2-chlorophenethyl, 2-nitrophenethyl, 2-aminophenethyl, 2-bromophenethyl, 2-fluorophenethyl, 2-tetrazolylphenethyl, 2,6-dihydroxyphenethyl, 2,6-dimethoxyphenethyl, 2,6-dichlorophenethyl, 2,6-dinitrophenethyl, 2,6-dimethylphenethyl and the like), unsubstituted styryl or styryl substituted with at least one substituent of substituent group E (styryl, 2-cyanostyryl, 2-hydroxystyryl, 2-chlorostyryl, 2-nitrostyryl, 2-aminostyryl, 2-bromostyryl, 2-fluorostyryl, 2-tetrazolylstyryl, 2,6-dihydroxystyryl, 2,6-dimethoxystyryl, 2,6-dichlorostyryl, 2,6-dinitrostyryl, 2,6-dimethylstyryl and the like), unsubstituted naphthyl or naphthyl substituted with at least one substituent of substituent group E (naphthyl, 2-cyanonaphthyl, 2-hydroxynaphthyl, 2-chloronaphthyl, 2-nitronaphthyl, 2-aminonaphthyl, 2-bromonaphthyl, 2-fluoronaphthyl, 2-tetrazolylnaphthyl, 2,8-dihydroxynaphthyl, 2,8-dimethoxynaphthyl, 2,8-dichloronaphthyl, 2,8-dinitronaphthyl, 2,8-dimethylnaphthyl and the like), or unsubstituted naphthylmethyl or naphthylmethyl substituted with at least one substituent of substituent group E (naphthylmethyl, (2-cyanonaphthyl)methyl, (2-hydroxynaphthyl)methyl, (2-chloronaphthyl)methyl, (2-nitronaphthyl)methyl, (2-aminonaphthyl)methyl, (2-bromonaphthyl)methyl, (2-fluoronaphthyl)methyl, (2-tetrazolylnaphthyl)methyl, (2,8-dihydroxynaphthyl)methyl, (2,8-dimethoxynaphthyl)methyl, (2,8-dichloronaphthyl)methyl, (2,8-dinitronaphthyl)methyl, (2,8-dimethylnaphthyl)methyl and the like).

Substituent group E represents halogen, C₁₋₈ alkyl (methyl, ethyl, 1-methylethyl, 1,1-dimethylethyl, propyl, 1-methylpropyl, 2-methylpropyl, butyl and the like), C₁₋₈ alkoxy (methoxy, ethoxy, propoxy, butoxy, 1-methylethyl, 1,1-dimethylethyl, 1-methylpropoxy, 2-methylpropoxy and the like), trifluoromethyl, trifluoromethoxy, C₁₋₈ alkylthio (methylthio, ethylthio, propylthio and the like), cyano, nitro, hydroxyl, amino, C₁₋₈ alkylacyl (acetyl, propionyl, butyryl, isobutyryl, valeryl, isovaleryl, pivaloyl and the like), C₁₋₈ alkylacylamino (acetylamino, propionylamino, butyrylamino, isobutyrylamino, valerylamino, isovaleiylamino, pivaloylamino and the like), and tetrazolyl.

In the present specification, an alkylacyl means a group in which an alkyl group is bonded to a carbonyl group, wherein the number of carbon atoms of the alkylacyl is represented by the number of carbon atoms of the alkyl moiety.

A "C₁₋₈ alkyl" represented by R₄ may be linear or branched, and represents methyl, ethyl, n-propyl, n-butyl, n-pentyl, n-hexyl, n-heptyl, n-octyl, 1-methylethyl, 1-methylpropyl, 2-methylpropyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 1,1-dimethylethyl, 2,2-dimethylpropyl, 3,5-dimethylhexyl, 3,6-dimethylhexyl, 4,5-dimethylhexyl and the like, and may also be substituted with 1 to 4 substituents of R₅ selected independently.

A "C₂₋₈ alkenyl" represented by R₄ may be linear or branched, and represents vinyl, 1-propenyl, 1-butenyl, 1-pentenyl, 1-hexenyl, 1-heptenyl, 1-octenyl, allyl, 2-butenyl, 3-butenyl, 2-pentenyl, 3-pentenyl, 4-pentenyl, 1-methylvinyl, 1-methyl-1-propenyl, 2-methyl-1-propenyl, 1,2-dimethyl-1-propenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1,2-dimethyl-1-butenyl and the like, and may also be substituted with 1 to 4 substituents of R₅ selected independently.

A "C₂₋₈ alkynyl" represented by R₄ may be linear or branched, and represents ethynyl, 1-propynyl, 1-butynyl, 1-pentynyl, 1-hexynyl, 1-heptynyl, 1-octynyl, 3-methyl-1-butynyl, 3,3-dimethyl-1-butynyl, 3-methyl-1-pentynyl, 4-methyl-1-pentynyl, 3,3-dimethyl-1-pentynyl, 3,4-dimethyl-1-pentynyl, 3,3,4-trimethyl- 1-pentynyl and the like, and may also be substituted with 1 to 4 substituents of R₈ selected independently.

A "Cy" represented by R₄ may be C₃₋₈ cycloalkyl that may be substituted with 1 to 4 substituents of R₆ or 3- to 8-membered monocyclic or bicyclic heterocycle that includes I to 4 nitrogen atoms, oxygen atoms or sulfur atoms independently selected, which heterocycle may be substituted with I to 4 substituents of R₆ (with the proviso that the hetero atoms do not bond directly with A).

For R₄, the "C₃₋₈ cycloalkyl" represented by "Cy" represents cyclopropyl, cyclobutyl, cyclopentyl, cyclohexyl, cycloheptyl or cyclooctyl.

For R₄, the "3- to 8-membered monocyclic or bicyclic heterocycle that includes 1 to 4 nitrogen atoms, oxygen atoms or sulfur atoms independently selected" represented by "Cy" may be saturated or partially unsaturated, and represents oxirane, oxetane, dihydrofuran, tetrahydrofuran, dihydropyran, tetrahydropyran, oxepane, oxocane, thiirane, thietane, dihydrothiophene, tetrahydrothiophene, dihydrothiopyran, tetrahydrothiopyran, thiepane, thiocane, aziridine, azetidine, dihydropyrrole, pyrrolidine, dihydropyridine, tetrahydropyridine, piperidine, azepan, azocane, oxazolidine, thiazoline, imidazolidine, dihydrooxazole, dihydrothiazole, dihydroimidazole, dioxolane, dithiolane, oxathiolane, dioxane, dithiane, hexahydropyrimidine, piperazine, dihydrooxazine, dihydrothiazine, tetrahydropyrimidine, octahydroindole, decahydroquinoline, decahydroisoquinoline and the like.

An "Ar" represented by R₄ is phenyl that may be substituted with 1 to 5 substituents of R₇, naphthyl that may be substituted with 1 to 5 substituents of R₇, or 5- to 8-membered monocyclic or bicyclic heteroaryl that includes 1 to 4 nitrogen atoms, oxygen atoms or sulfur atoms independently selected, which heterocycle may be substituted with 1 to 5 substituents of R₇ (wherein the hetero atoms do not directly bond with A).

For R₄, the "5- to 8-membered monocyclic or bicyclic heteroaryl that includes 1 to 4 nitrogen atoms, oxygen atoms or sulfur atoms independently selected" represented by "Ar" represents furan, benzofuran, isobenzofuran, benzodioxane, thiophene, benzo[b]thiophene, benzo[c]thiophene, pyrrole, indole, isoindole, quinoline, isoquinoline, imidazole, pyrazole, indazole, benzimidazole, cinnoline, quinazoline, quinoxaline, oxazole, isoxazole, benzoxazine, thiazole, isothiazole, benzoxathiin, furazole, triazole, tetrazole and the like.

A "Cy-C₁₋₈ alkyl" represented by R₄ represents methyl, ethyl, n-propyl, n-butyl, n-pentyl, n-hexyl, n-heptyl, n-octyl, 1-methylethyl, 1-methylpropyl, 2-methylpropyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 1,1-dimethylethyl, 2,2-dimethylpropyl, 3,5-dimethylhexyl, 3,6-dimethylhexyl, 4,5-dimethylhexyl and the like substituted with "Cy".

A "Cy-C₁₋₈ alkenyl" represented by R₄ represents vinyl, 1-propenyl, 1-butenyl, 1-pentenyl, 1-hexenyl, 1-heptenyl, 1-octenyl, allyl, 2-butenyl, 3-butenyl, 2-pentenyl, 3-pentenyl, 4-pentenyl, 1-methylvinyl, 1-meihyl-1-propenyl, 2-methyl-1-propenyl, 1,2-dimethyl-1-propenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1,2-dimethyl-1-butenyl and the like substituted with "Cy".

A "Cy-C₁₋₈ alkynyl" represented by R₄ represents ethynyl, 1-propynyl, 1-butynyl, 1-pentynyl, 1-hexynyl, 1-heptynyl, 1-octynyl, 3-methyl-1-butynyl, 3,3-dimethyl-1-butynyl, 3-methyl-1-pentynyl, 4-methyl-1-pentynyl, 3,3-dimethyl-1-pentynyl, 3,4-dimethyl-1-pentynyl, 3,3,4-trimethyl-1-pentynyl and the like substituted with "Cy".

An "Ar-C₁₋₈ alkyl" represented by R₄ represents methyl, ethyl, n-propyl, n-butyl, n-pentyl, n-hexyl, n-heptyl, n-octyl, 1-methylethyl, 1-methylpropyl, 2-methylpropyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 1,1-dimethylethyl, 2,2-dimethylpropyl, 3,5-dimethylhexyl, 3,6-dimethylhexyl, 4,5-dimethylhexyl and the like substituted with "Ar".

An "Ar-C₁₋₈ alkenyl" represented by R₄ represents vinyl, 1-propenyl, 1-butenyl, 1-pentenyl, 1-hexenyl, 1-heptenyl, 1-octenyl, allyl, 2-butenyl, 3-butenyl, 2-pentenyl, 3-pentenyl, 4-pentenyl, 1-methylvinyl, 1-methyl-1-propenyl, 2-methyl-1-prop 1,2-dimethyl-1-propenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1,2-dimethyl-1-butenyl and the like substituted with "Ar".

An "Ar-C₁₋₈ alkynyl" represented by R₄ represents 1-propynyl, 1-butynyl, 1-pentynyl, 1-hexynyl, 1-heptynyl, 1-octynyl, 3-methyl-1-butynyl, 3,3-dimethyl-1-butynyl, 3-methyl-1-pentynyl, 4-methyl-1-pentynyl, 3,3-dimethyl-1-pentynyl, 3,4-dimethyl-1-pentynyl, 3,3,4-trimethyl-1-pentynyl and the like substituted with "Ar".

A "C₁₋₈ alkyl" represented by R₆, R₇, R^{a}, R^{b}, R^{c} and R^{d} represents methyl, ethyl, n-propyl, n-butyl, n-pentyl, n-hexyl, n-heptyl, n-octyl, 1-methylethyl, 1-methylpropyl, 2-methylpropyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 1,1-dimethylethyl, 2,2-dimethylpropyl, 3,5-dimethylhexyl, 3,6-dimethylhexyl, 4,5-dimethylhexyl and the like.

A "C₂₋₈ alkenyl" represented by R₆ and R₇ represents vinyl, 1-propenyl, 1-butenyl, 1-pentenyl, 1-hexenyl, 1-heptenyl, 1-octenyl, allyl, 2-butenyl, 3-butenyl, 2-pentenyl, 3-pentenyl, 4-pentenyl, 1-methylvinyl, 1-methyl-1-propenyl, 2-methyl-1-propenyl, 1,2-dimethyl-1-propenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1,2-dimethyl-1-butenyl and the like.

A "C₂₋₈ alkynyl" represented by R₆ and R₇ may be linear or branched, and represents ethynyl, 1-propynyl, 1-butynyl, 1-pentynyl, 1-hexynyl, 1-heptynyl, 1-octynyl, 3-methyl-I-butynyl, 3,3-dimethyl-1-butynyl, 3-methyl-1-pentynyl, 4-methyl-1-pentynyl, 3,3-dimethyl-1-pentynyl, 3,4-dimethyl-1-pentynyl, 3,3,4-trimethyl-1-pentynyl and the like.

A "C₃₋₈ cycloalkyl" represented by R^{b}, R^{c} and R^{d} represents cyclopropyl, cyclobutyl, cyclopentyl, cyclohexyl, cycloheptyl or cyclooctyl.

A "C₃₋₈ cycloalkyl-C₁₋₈ alkyl" represented by R₆, R₇, R^{b}, R^{c} and R^{d} represents cyclopropylmethyl, cyclobutylmethyl, cyclopentylmethyl, cyclohexylmethyl, cycloheptylmethyl, cyclooctylmethyl, cyclopropylethyl, cyclobutylethyl, cyclopentylethyl, cyclohexylethyl, cycloheptylethyl, cyclooctylethyl, cyclopropylpropyl, cyclobutylpropyl, cyclopentylpropyl, cyclohexylpropyl, cycloheptylpropyl, cyclooctylpropyl, cyclopropylbutyl, cyclobutylbutyl, cyclopentylbutyl, cyclohexylbutyl, cycloheptylbutyl, cyclooctylbutyl and the like.

An "unsubstituted phenyl or phenyl substituted with at least one substituent of substituent group E" represented by R₆, R₇, R^{b}, R^{c} and R^{d} represents phenyl, 2-fluorophenyl, 2-chlorophenyl, 2-bromophenyl, 2-methylphenyl, 2-methoxyphenyl, 2-trifluoromethylphenyl, 2-methylthiophenyl, 2-cyanophenyl, 2-nitrophenyl, 2-hydroxyphenyl, 2-aminophenyl, 2-acetylphenyl, 2-acetylaminophenyl, 2-tetrazolylphenyl, 3-fluorophenyl, 3-chlorophenyl, 3-bromophenyl, 3-methylphenyl, 3-methoxyphenyl, 3-trifluoromethylphenyl, 3-methylthiophenyl, 3-cyanophenyl, 3-nitrophenyl, 3-hydroxyphenyl, 3-aminophenyl, 3-acetylphenyl, 3-acetylaminophenyl, 3-tetrazolylphenyl, 4-fluorophenyl, 4-chlorophenyl, 4-bromophenyl, 4-methylphenyl, 4-methoxyphenyl, 4-trifluoromethylphenyl, 4-methylthiophenyl, 4-cyanophenyl, 4-nitrophenyl, 4-hydroxyphenyl, 4-aminophenyl, 4-acetylphenyl, 4-acetylaminophenyl, 4-tetrazolylphenyl, 2,6-difluorophenyl, 2,6-dichlorophenyl, 2,6-dibromophenyl, 2,6-dimethylphenyl, 2,6-dimethoxyphenyl, 2,6-ditrifluoromethylphenyl, 2,6-dinitrophenyl, 2,6-dihydroxyphenyl, 2-bromo-6-methylphenyl, 2-methyl-5-nitrophenyl, 3,5-dichlorophenyl, 2,4,6-trichlorophenyl and the like.

An "unsubstituted benzyl or benzyl substituted with at least one substituent of substituent group E" represented by R₆, R₇, R^{b}, R^{c} and R^{d} represents benzyl, 2-fluorobenzyl, 2-chlorobenzyl, 2-bromobenzyl, 2-methylbenzyl, 2-methoxybenzyl, 2-trifluoromethylbenzyl, 2-methylthiobenzyl, 2-cyanobenzyl, 2-nitrobenzyl, 2-hydroxybenzyl, 2-aminobenzyl, 2-acetylbenzyl, 2-acetylaminobenzyl, 2-tetrazolylbenzyl, 3-fluorobenzyl, 3-chlorobenzyl, 3-bromobenzyl, 3-methylbenzyl, 3-methoxybenzyl, 3-trifluoromethylbenzyl, 3-methylthiobenzyl, 3-cyanobenzyl, 3-nitrobenzyl, 3-hydroxybenzyl, 3-aminobenzyl, 3-acetylbenzyl, 3-acetylaminobenzyl, 3-tetrazolylbenzyl, 4-fluorobenzyl, 4-chlorobenzyl, 4-bromobenzyl, 4-methylbenzyl, 4-methoxybenzyl, 4-trifluoromethylbenzyl, 4-methylthiobenzyl, 4-cyanobenzyl, 4-nitrobenzyl, 4-hydroxybenzyl, 4-aminobenzyl, 4-acetylbenzyl, 4-acetylaminobenzyl, 4-tetrazolylbenzyl, 2,6-difluorobenzyl, 2,6-dichlorobenzyl, 2,6-dibromobenzyl, 2,6-dimethylbenzyl, 2,6-dimethoxybenzyl, 2,6-ditrifluoromethylbenzyl, 2,6-dinitrobenzyl, 2,6-dihydroxybenzyl and the like.

An "unsubstituted phenethyl or phenethyl substituted with at least one substituent of substituent group E" represented by R^{b}, R^{c} and R^{d} represents phenethyl, 2-fluorophenethyl, 2-chlorophenethyl, 2-bromophenethyl, 2-methylphenethyl, 2-methoxyphenethyl, 2-trifluoromethylphenethyl, 2-methylthiophenethyl, 2-cyanophenethyl, 2-nitrophenethyl, 2-hydroxyphenethyl, 2-aminophenethyl, 2-acetylphenethyl, 2-acetylaminophenethyl, 2-tetrazolylphenethyl, 3-fluorophenethyl, 3-chlorophenethyl, 3-bromophenethyl, 3-methylphenethyl, 3-methoxyphenethyl, 3-trifluoromethylphenethyl, 3-methylthiophenethyl, 3-cyanophenethyl, 3-nitrophenethyl, 3-hydroxyphenethyl, 3-aminophenethyl, 3-acetylphenethyl, 3-acetylaminophenethyl, 3-tetrazolylphenethyl, 4-fluorophenethyl, 4-chlorophenethyl, 4-bromophenethyl, 4-methylphenethyl, 4-methoxyphenethyl, 4-trifluoromethylphenethyl, 4-methylthiophenethyl, 4-cyanophenethyl, 4-nitrophenethyl, 4-hydroxyphenethyl, 4-aminophenethyl, 4-acetylphenethyl, 4-acetylaminophenethyl, 4-tetrazolylphenethyl, 2,6-difluorophenethyl, 2,6-dichlorophenethyl, 2,6-dibromophenethyl, 2,6-dimethylphenethyl, 2,6-dimethoxyphenethyl, 2,6-ditrifluoromethylphenethyl, 2,6-dinitrophenethyl, 2,6-dihydroxyphenethyl and the like.

An "unsubstituted styryl or styryl substituted with at least one substituent of substituent group E" represented by R^{b}, R^{c} and R^{d} represents styryl, 2-fluorostyryl, 2-chlorostyryl, 2-bromostyryl, 2-methylstyryl, 2-methoxystyryl, 2-trifluoromethylstyryl, 2-methylthiostyryl, 2-cyanostyryl, 2-nitrostyryl, 2-hydroxystyryl, 2-aminostyryl, 2-acetylstyryl, 2-acetylaminostyryl, 2-tetrazolylstyryl, 3-fluorostyryl, 3-chlorostyryl, 3-bromostyryl, 3-methylstyryl, 3-methoxystyryl, 3-trifluoromethylstyryl, 3-methylthiostyryl, 3-cyanostyryl, 3-nitrostyryl, 3-hydroxystyryl, 3-aminostyryl, 3-acetylstyryl, 3-acetylaminostyryl, 3-tetrazolylstyryl, 4-fluorostyryl, 4-chlorostyryl, 4-bromostyryl, 4-methylstyryl, 4-methoxystyryl, 4-trifluoromethylstyryl, 4-methylthiostyryl, 4-cyanostyryl, 4-nitrostyryl, 4-hydroxystyryl, 4-aminostyryl, 4-acetylstyryl, 4-acetylaminostyryl, 4-tetrazolylstyryl, 2,6-difluorostyryl, 2,6-dichlorostyryl, 2,6-dibromostyryl, 2,6-dimethylstyryl, 2,6-dimethoxystyryl, 2,6-ditrifluoromethylstyryl, 2,6-dinitrostyryl, 2,6-dihydroxystyryl and the like.

An "unsubstituted naphthyl or naphthyl substituted with at least one substituent of substituent group E" represented by R^{b}, R^{c} and R^{d} represents 1-naphthyl, 2-naphthyl, 2-fluoronaphthyl, 2-chloronaphthyl, 2-bromonaphthyl, 2-methylnaphthyl, 2-methoxynaphthyl, Z-triffuoromethylnaphthyl, 2-cyanonaphthyl, 2-nitronaphthyl, 2-hydroxynaphthyl, 2-aminonaphthyl, 2,8-dichloronaphthyl, 2,8-dimethylnaphthyl, 2,8-dimethoxynaphthyl, 2,8-dinitronaphthyl, 2,8-dihydroxynaphthyl and the like.

An "unsubstituted naphthylmethyl or naphthylmethyl substituted with at least one substituent of substituent group E" represented by R^{b}, R^{c} and R^{d} represents napthylmethyl, (2-cyanonaphthyl)methyl, (2-hydroxynaphthyl)methyl, (2-chloronaphthyl)methyl, (2-nitronaphthyl)methyl, (2-aminonaphthyl)methyl, (2-bromonaphthyl)methyl, (2-fluoronaphthyl)methyl, (2-tetrazolylnaphthyl)methyl, (2,8-dihydroxynaphthyl)methyl, (2,8-dimethoxynaphthyl)methyl, (2,8-dichloronaphthyl)methyl, (2,8-dinitronaphthyl)methyl, (2,8-dimethylnaphthyl)methyl and the like.

While any integer is preferable as l, more preferable is 0 or 1, and most preferable is 0.

While any integer is preferable as m, more preferable is 1 or 2, and most preferable is 1.

While any integer is preferable as n, more preferable is 1.

While any group is preferable as A, more preferable is -C(O)-.

While any atom is preferable as B, more preferable is -NH-.

While any group is preferable as C' and D, more preferable is where C' and D represent together =O.

While any group is preferable as X₁ and Y₁, more preferable is hydrogen, halogen, methyl, trifluoromethyl, methoxy, cyano, nitro or hydroxyl and even more preferable is hydrogen.

While any group is preferable as R₁, more preferable is hydrogen or C₁₋₆ linear alkyl.

While any group is preferable as R₂, more preferable is hydrogen.

Preferable as R₃ is hydrogen, C₁₋₆ linear alkyl, C₃₋₈ branched alkyl, C₆₋₁₀ cycloalkylalkyl or benzyl and more preferable is methyl, ethyl, propyl, 2-methylpropyl or benzyl.

R₄ is preferably a group selected from the following (i) through (vii).
(i) C₃₋₈ cycloalkyl that may be substituted with 1 to 4 substituents of R₆,
(ii) 3- to 8-membered monocyclic or bicyclic heterocycle that includes from 1 to 4 nitrogen atoms or oxygen atoms independently selected, which heterocycle may be substituted with 1 to 4 substituents of R₆ (with the proviso that the hetero atoms do not bond directly with A),
(iii) tetrahydrothiophene or tetrahydrothiopyran that may be substituted with 1 to 4 substituents of R₆,
(iv) phenyl that may be substituted with from 3 to 5 substituents of R₇,
(v) naphthyl that may be substituted with 1 to 4 substituents of R₇, or 5- to 8-membered monocyclic or bicyclic heteroaryl that includes 1 to 4 nitrogen atoms, oxygen atoms or sulfur atoms independently selected, which heteroaryl may be substituted with 1 to 4 substituents of R₇,
(vi) C₃₋₈ cycloalkyl-C₁₋₈ alkyl,
(vii) 3- to 8-membered monocyclic heterocycle-C₁₋₈ alkyl, the monocyclic heterocycle including 1 to 4 nitrogen atoms or oxygen atoms independently selected, and may be substituted with 1 to 4 substituents of R₆

In the above cases, while any group is preferable as the C₃₋₈ cycloalkyl, more preferable is cyclopropyl, cyclopentyl or cyclohexyl.

A 3- to 8-membered monocyclic or bicyclic heterocycle that includes 1 to 4 nitrogen atoms or oxygen atoms independently selected is preferably a 3- to 8-membered monocyclic heterocycle that includes 1 to 2 nitrogen atoms or oxygen atoms (oxirane, oxetane, dihydrofuran, tetrahydrofuran, dihydropyran, tetrahydropyran, oxepane, oxocane, dioxolane, dioxane, aziridine, azetidine, dihydropyrrole, pyrrolidine, piperidine, azepan, azocane, imidazolidine, dihydroimidazolidine, tetrahydropyrimidine, hexahydropyrimidine, piperidine, oxazolidine, dihydrooxazole, oxathiolanedihydrooxazine or the like) and more preferably a 3-to 8-membered monocyclic heterocycle that includes I nitrogen atom or oxygen atom (oxirane, oxetane, tetrahydrofuran, tetrahydropyran, oxepane, oxocane, aziridine, azetidine, pyrrolidine, piperidine, azepan or azocane).

The 5- to 8-membered monocyclic or bicyclic heteroaryl that includes 1 to 4 nitrogen atoms, oxygen atoms or sulfur atoms independently selected may be preferably any group, more preferably furan, benzofuran, thiophene, benzothiophene, pyrrole, pyridine, indole, quinoline, isoquinoline, imidazole, pyrazole, indazole, benzimidazole, cinnoline, quinazoline, quinoxaline, oxazole, isoxazole, thiazole or isothiazole.

The C₃₋₈ cycloalkyl-C₁₋₈ alkyl is preferably cyclopropylmethyl, cyclobutylmethyl, cyclopentylmethyl, cyclohexylmethyl, cyclopropylethyl, cyclobutylethyl, cyclopentylethyl or cyclohexylmethyl.

The 3- to 8-membered monocyclic heterocycle-C₁₋₈ alkyl, the heterocycle including 1 to 4 nitrogen atoms or oxygen atoms independently selected, is preferably pyrrolidinylmethyl, piperidinylmethyl, imidazolinylmethyl, piperidinylmethyl, tetrahydrofuranylmethyl, tetrahydropyranylmethyl, oxazolinylmethyl, pyrrolidinylethyl, piperidinylethyl, imidazolinylethyl, piperidinylethyl, tetrahydrofuranylethyl, tetrahydropyranylethyl, oxazolinylethyl, pyrrolidinylpropyl, piperidinylpropyl, imidazolinylpropyl, piperidinylpropyl, tetrahydrofuranylpropyl, tetrahydropyranylpropyl or oxazolinylpropyl, more preferable is pyrrolidinylmethyl, piperidinylmethyl, tetrahydrofuranylmethyl, tetrahydropyranylmethyl, pyrrolidinylethyl, piperidinylethyl, tetrahydrofuranylethyl or tetrahydropyranylethyl.

Among R₄, the substituent R₆ for C₃₋₈ alkyl is preferably selected from the following substituent groups.

That is, halogen, trifluoromethyl, -OR^{a}, C₁₋₈ alkyl, =O, -C(O)R^{b}, -C(O)NR^{c}R^{d}, -SO₂R^{b}, -SO₂NR^{c}R^{d}, -NR^{c}C(O)R^{b}, -NR^{c}SO₂R^{b}, phenyl or benzyl.

Among R₄, the substituent R₆ for 3- to 8-membered monocyclic or bicyclic heterocycle comprising from 1 to 4 nitrogen atoms or oxygen atoms independently selected is preferably selected from the following substituent groups.

That is, C₁₋₈ alkyl, C₃₋₈ cycloalkyl-C₁₋₈ alkyl, =O, -C(O)R^{b}, -C(O)OR^{b}, -C(O)NR^{c}R^{d}, -SO₂R^{b}, -SO₂NR^{c}R^{d}, unsubstituted phenyl or phenyl substituted with at least one substituent of substituent group E, or unsubstituted benzyl or benzyl substituted with at least one substituent of substituent group E.

Among R₄, the substituent R₆ for tetrahydrothiophene or tetrahydrothiopyran is preferably -OR^{a}, -SR^{a}, C₁₋₈ alkyl, =O, phenyl or benzyl.

Among R₄, the substituent R₇ for phenyl and naphthyl is preferably halogen, trifluoromethyl, -OR^{a}, C₁₋₈ alkyl, cyano, nitro or phenyl, and more preferably halogen, trifluoromethyl, hydroxyl, methoxy, trifluoromethoxy, methyl, cyano or nitro.

Among R₄, the substituent R₇ for the 5- to 8-membered monocyclic or bicyclic heteroaryl that includes 1 to 4 nitrogen atoms, oxygen atoms or sulfur atoms independently selected, is preferably selected from the following substituent group.

That is, halogen, trifluoromethyl, -OR^{a}, C₁₋₈ alkyl, C₃₋₈ cycloalkyl-C₁₋₈ alkyl, -C(O)R^{b}, -SO₂R^{b}, or unsubstituted benzyl or benzyl substituted with at least one substituent of substituent group E.

Among R₄, the substituent R₇ for the 3- to 8-membered monocyclic heterocycle-C₁₋₈ alkyl, the heterocycle including 1 to 4 nitrogen atoms or oxygen atoms independently selected, is preferably selected from the following substituent group.

That is, halogen, trifluoromethyl, -OR^{a}, C₁₋₈ alkyl, C₃₋₈ cycloalkyl-C₁₋₈ alkyl, -C(O)R^{b}, -SO₂R^{b}, or unsubstituted benzyl or benzyl substituted with at least one substituent of substituent group E.

While for R₆, R₇, R^{a}, R^{b} and R^{c} any of the substituent group E, that is the substituents for phenyl, benzyl, phenethyl, styryl, naphthyl and naphthylmethyl, is preferable, more preferable is halogen, methyl, methoxy, trifluoromethyl, trifluoromethoxy, cyano, nitro and hydroxyl.

Specific examples of the compound according to the present invention include the compounds illustrated in the following Tables I to 46, pharmaceutically acceptable salt thereof and the compounds illustrated in the Examples. However, the present invention is not to be restricted to these compounds. Further, the present invention may comprise isomers formed from the presence of asymmetric centers, that is, comprises opitical isomers and mixtures thereof.

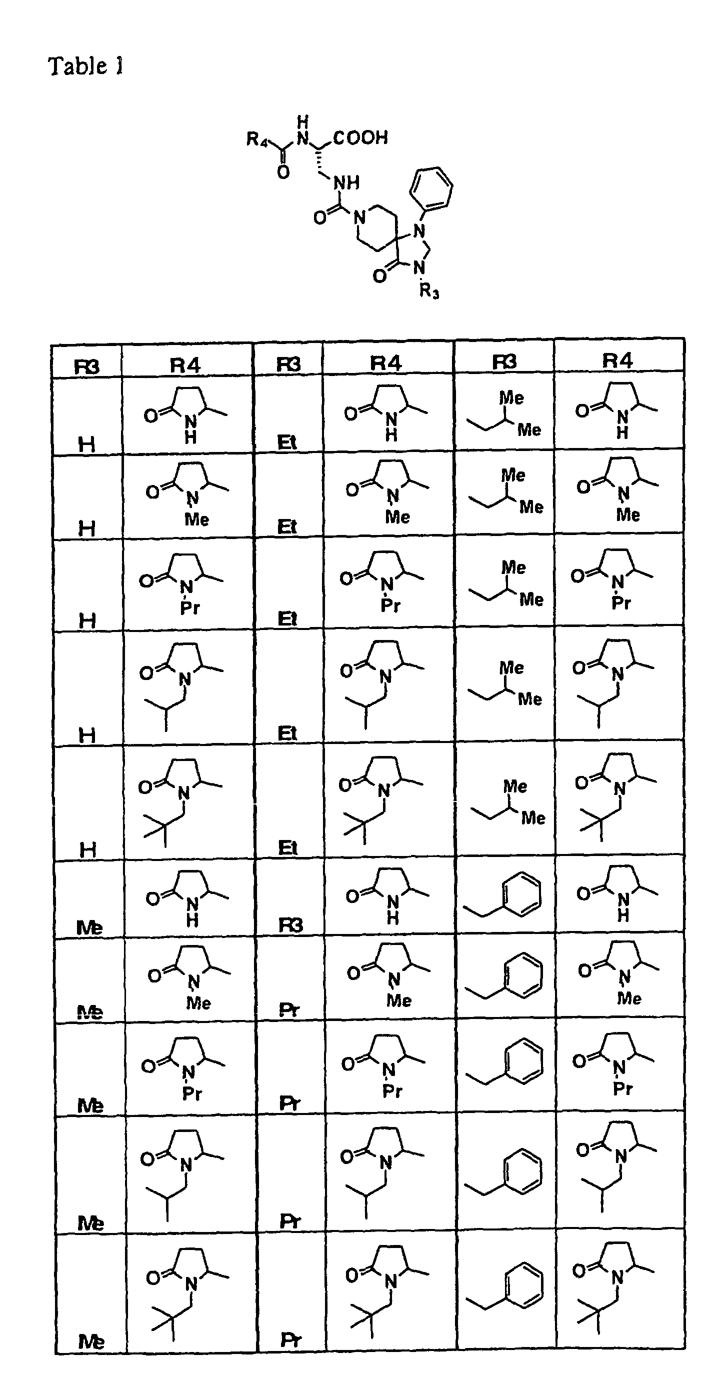

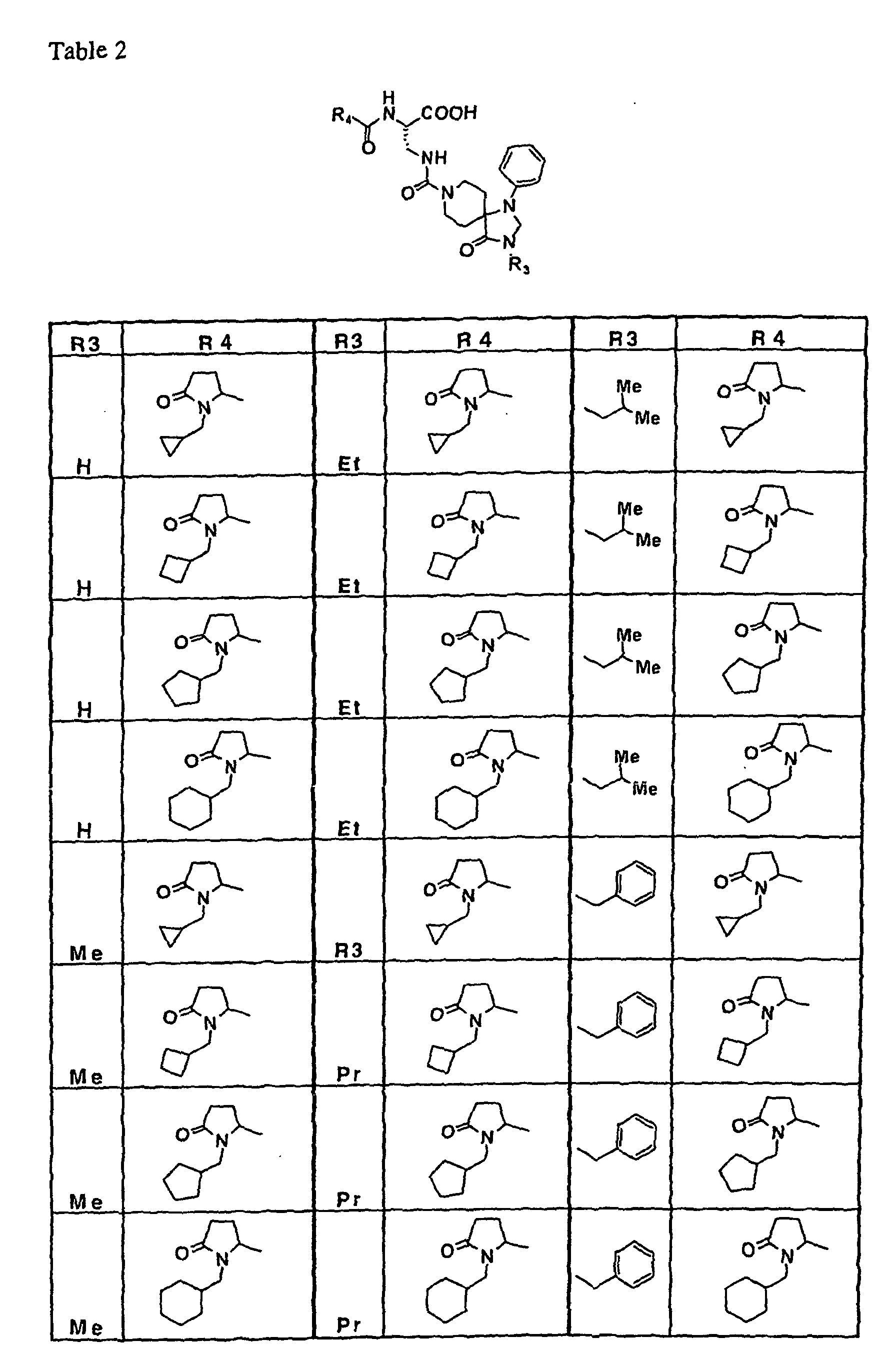

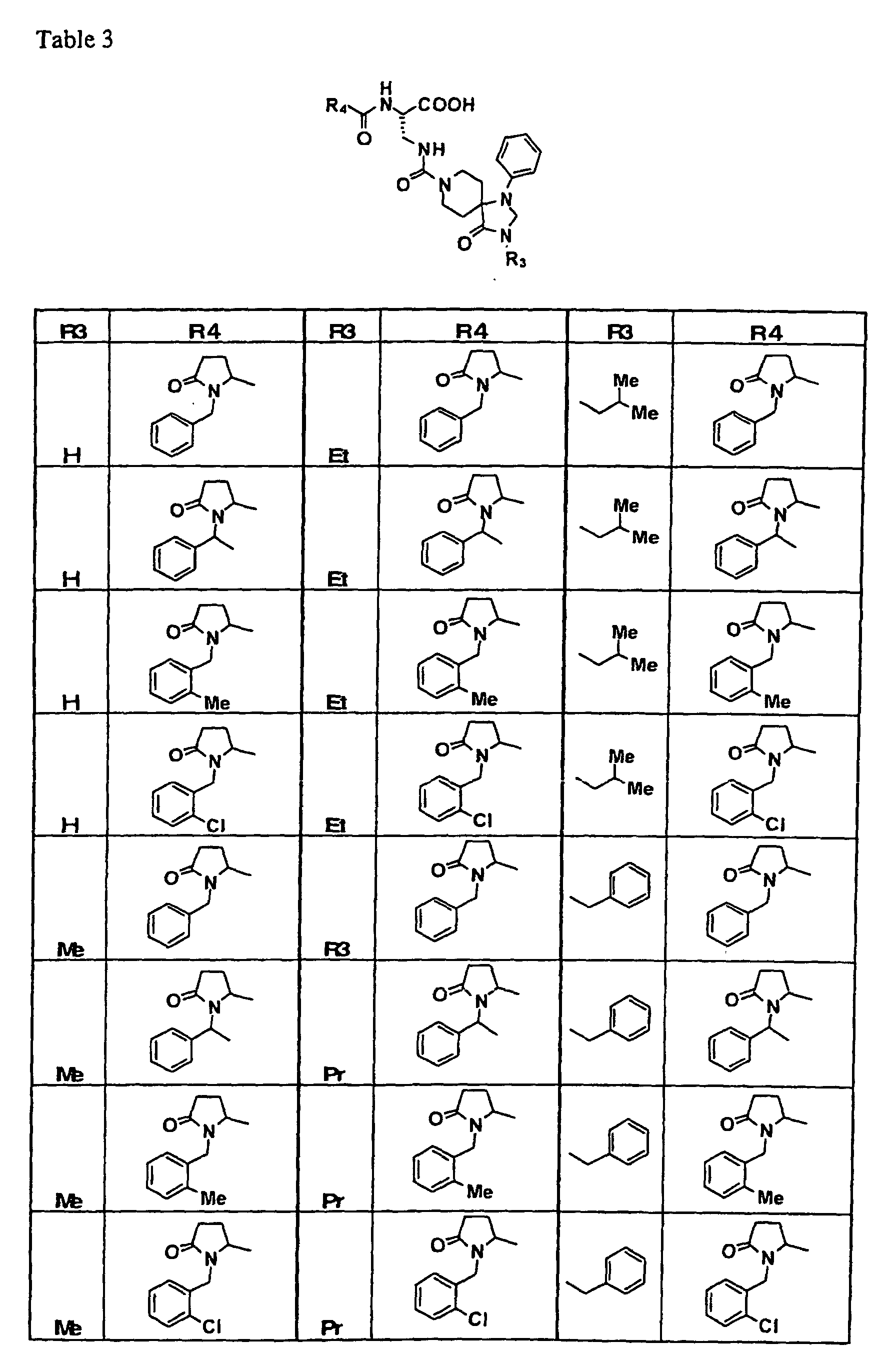

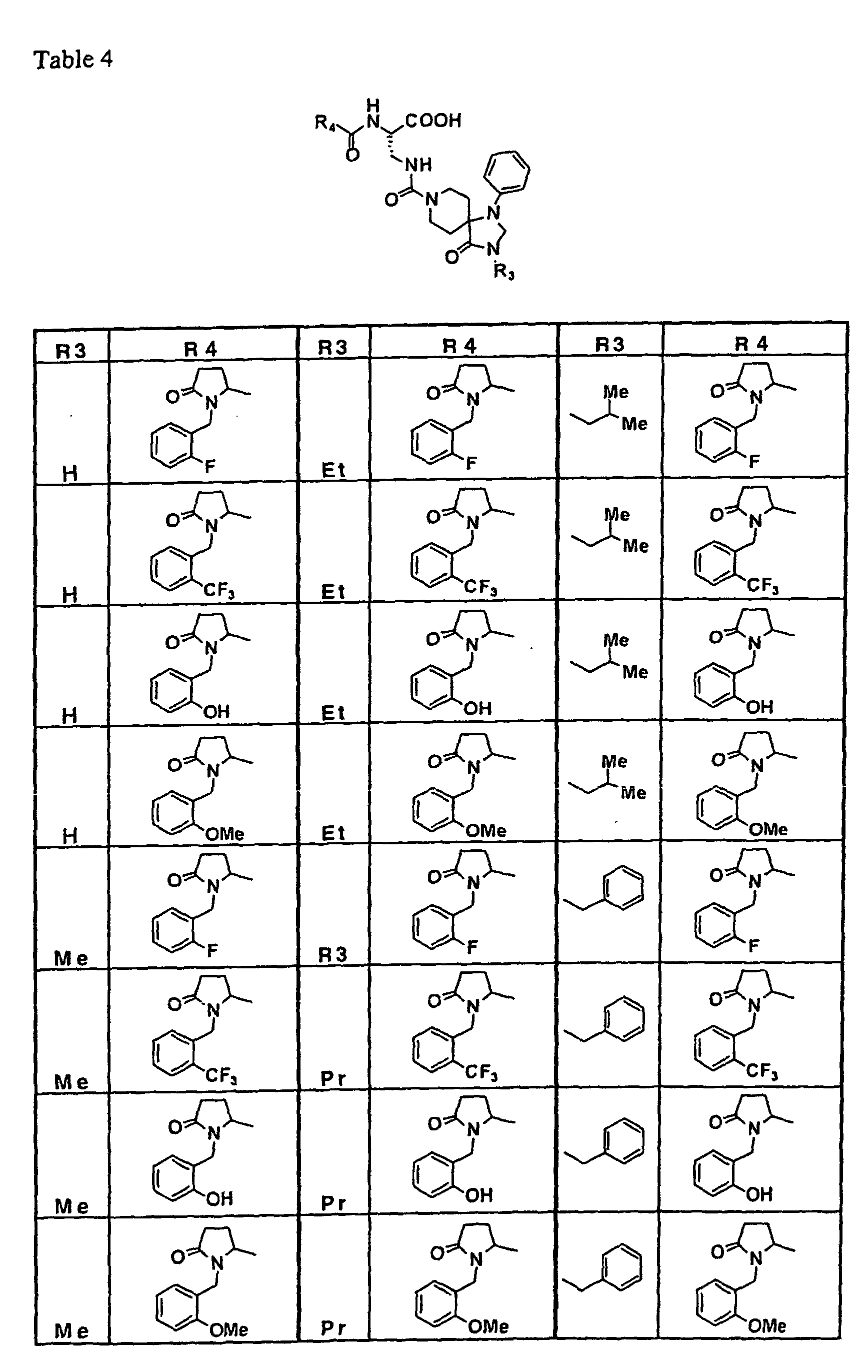

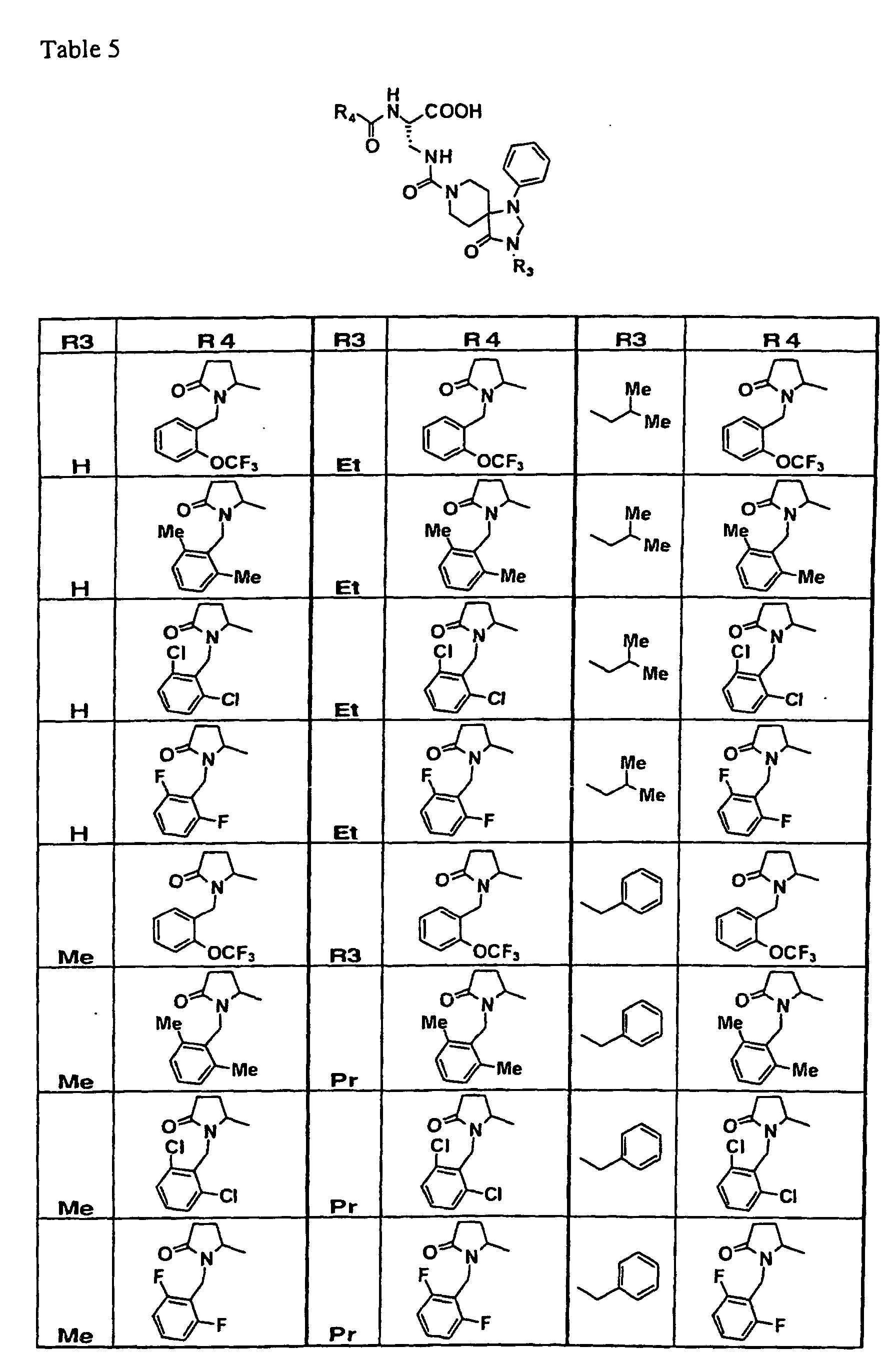

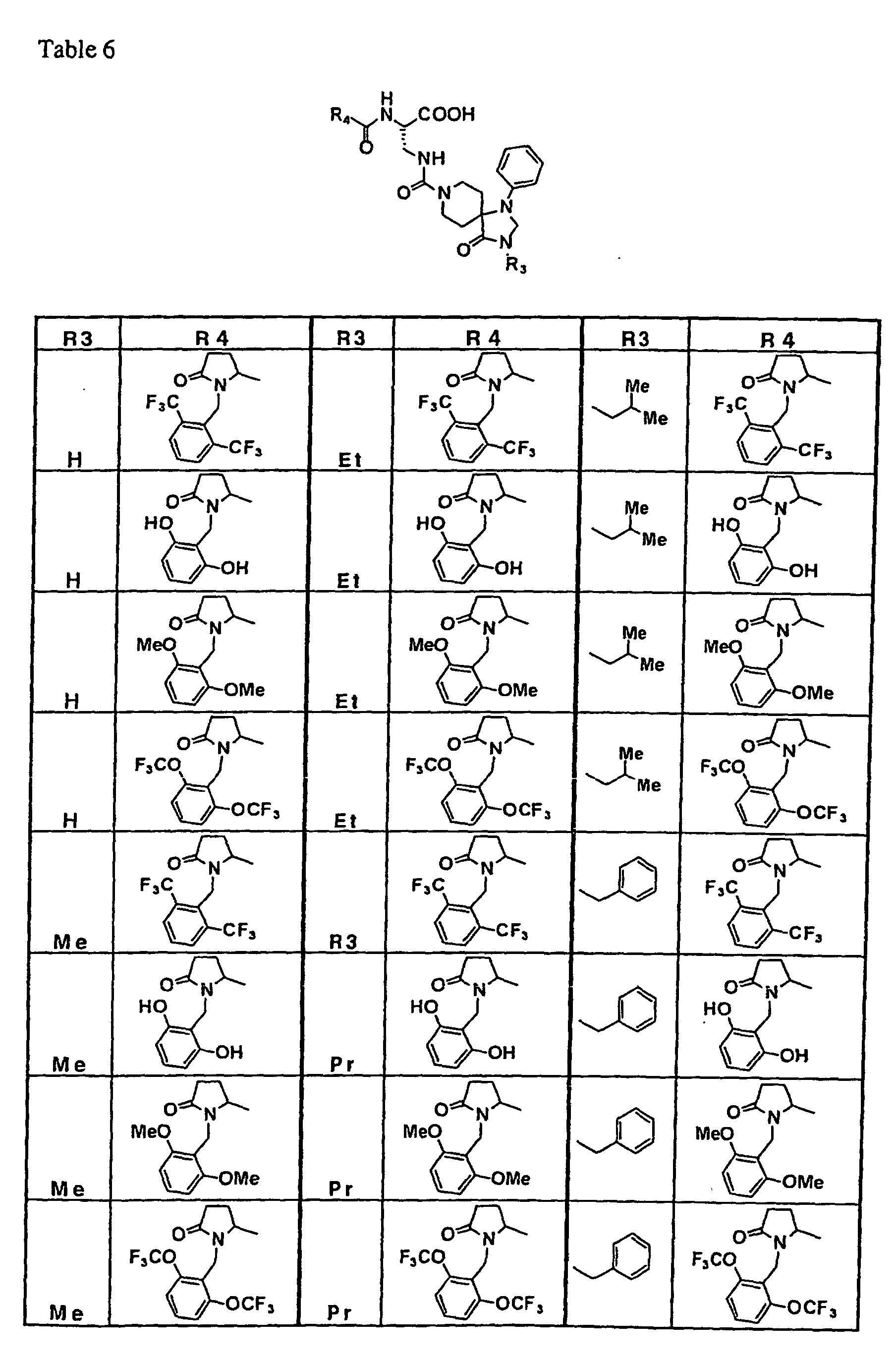

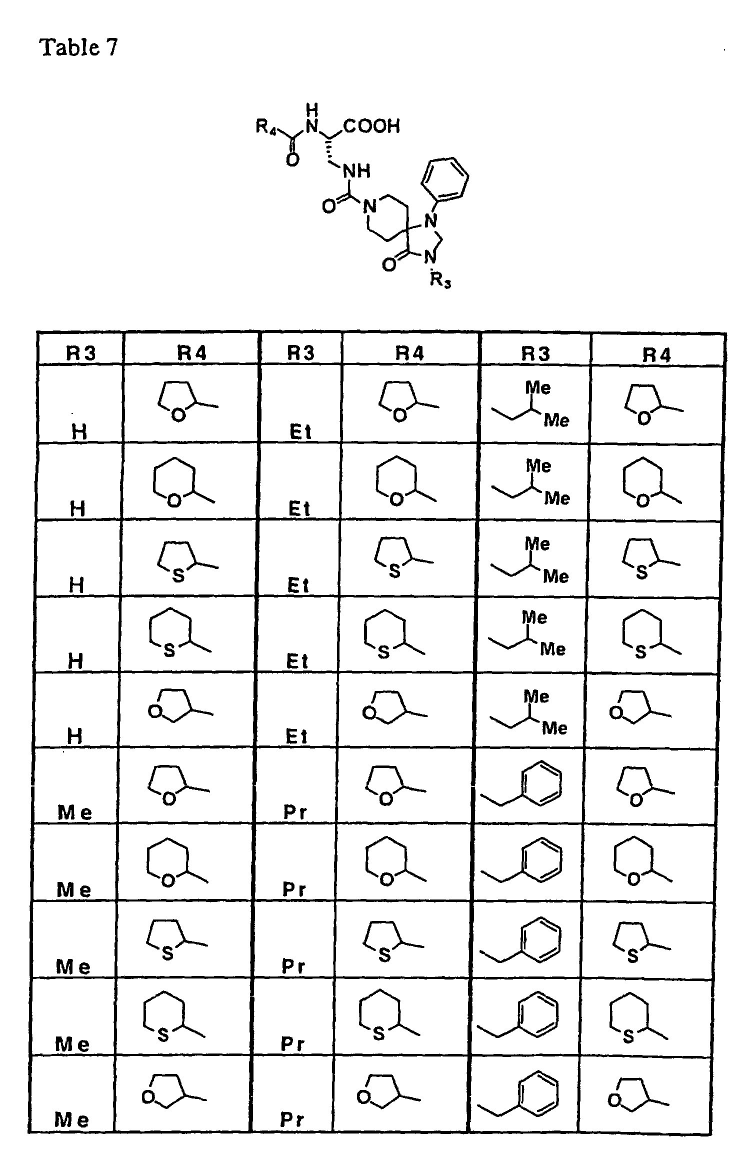

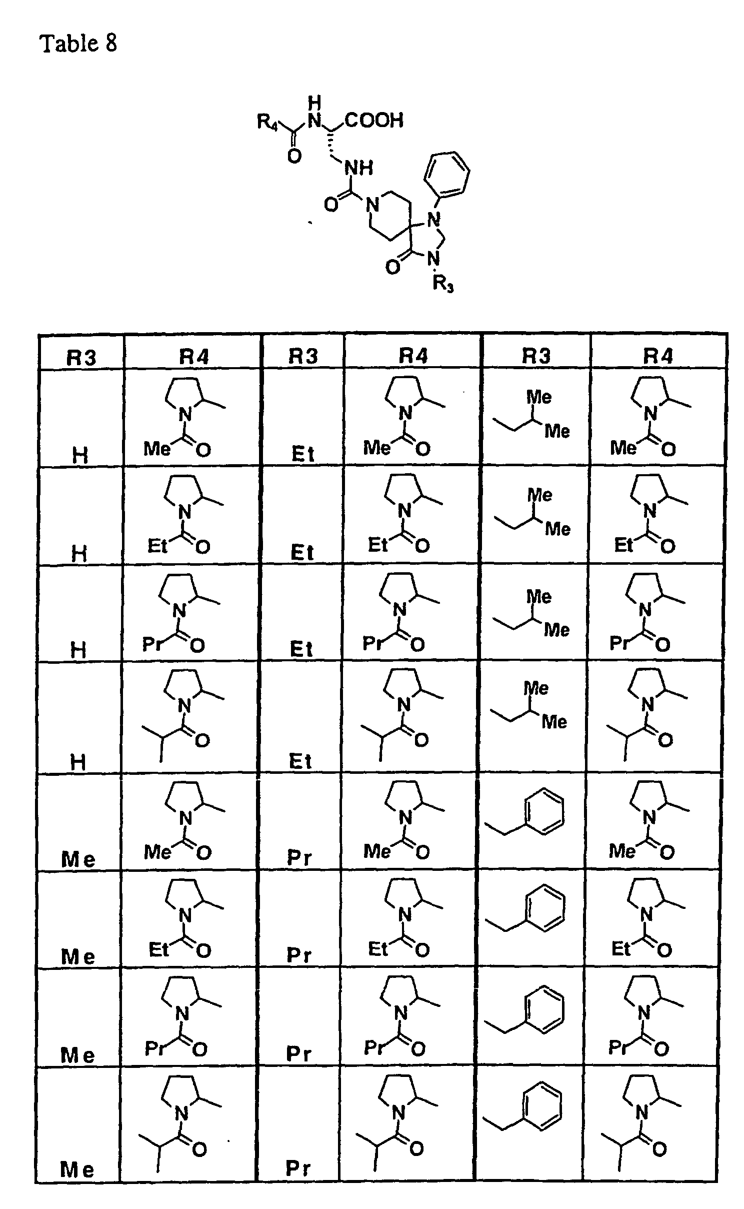

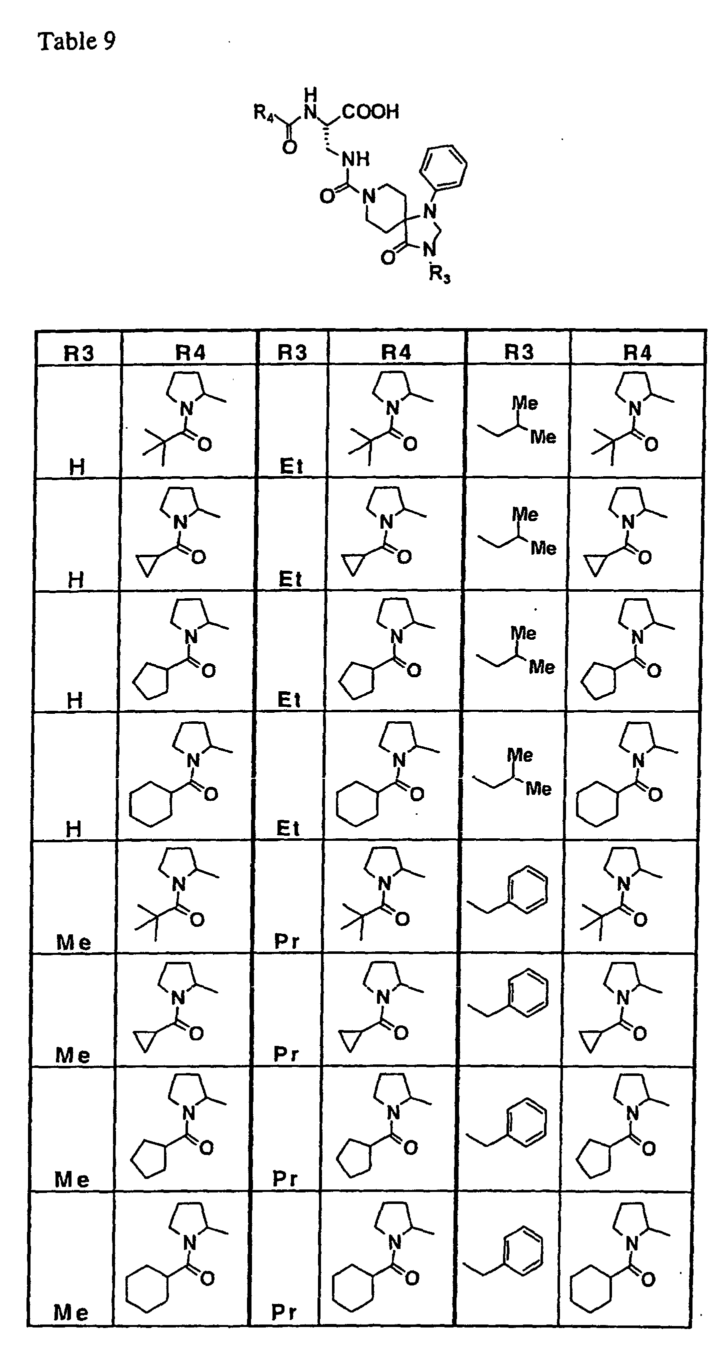

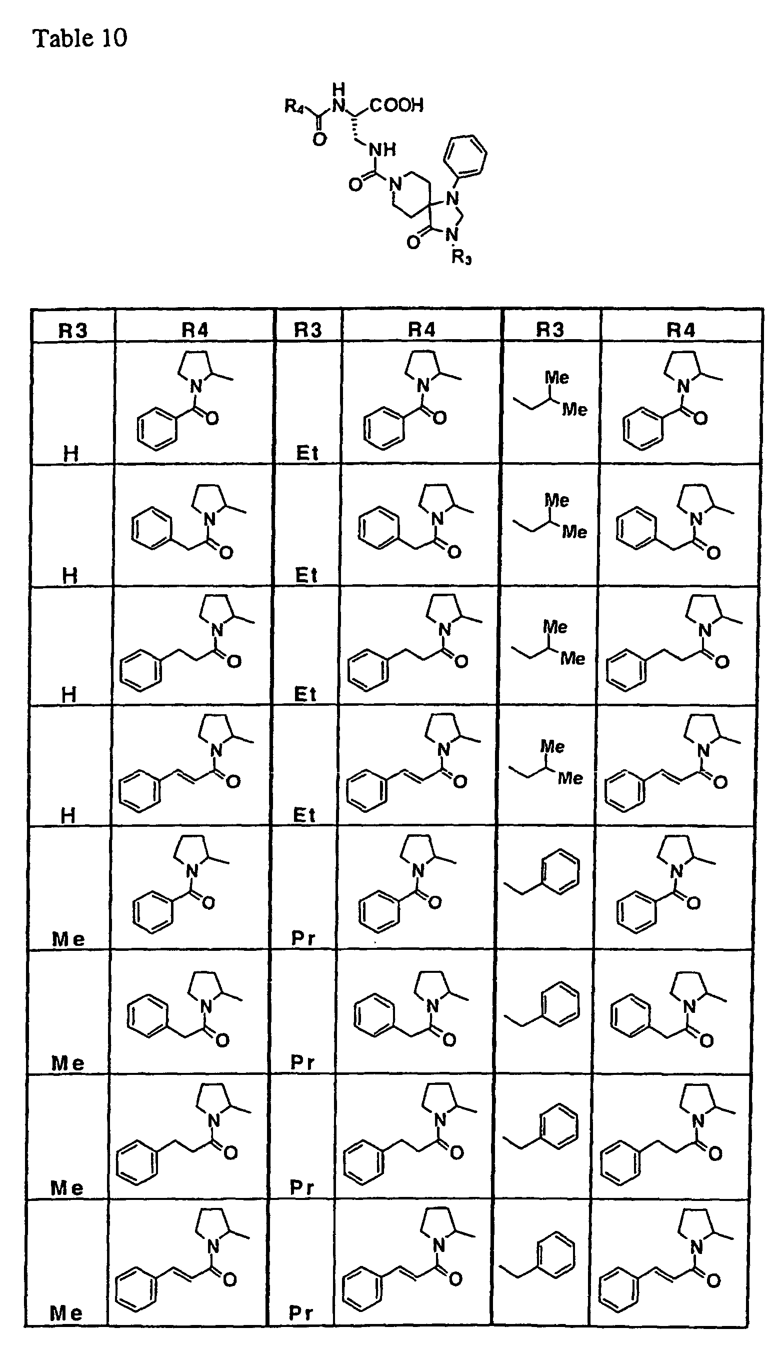

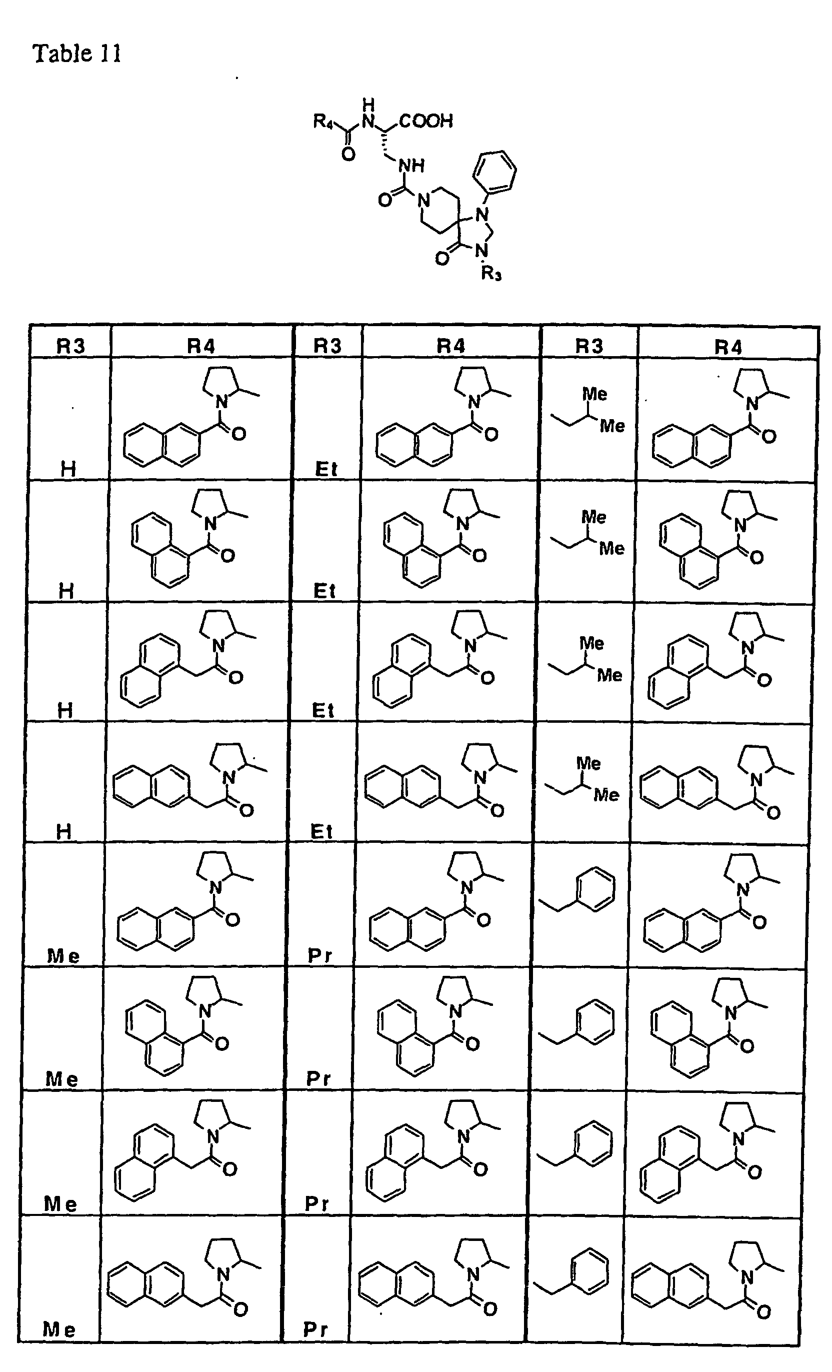

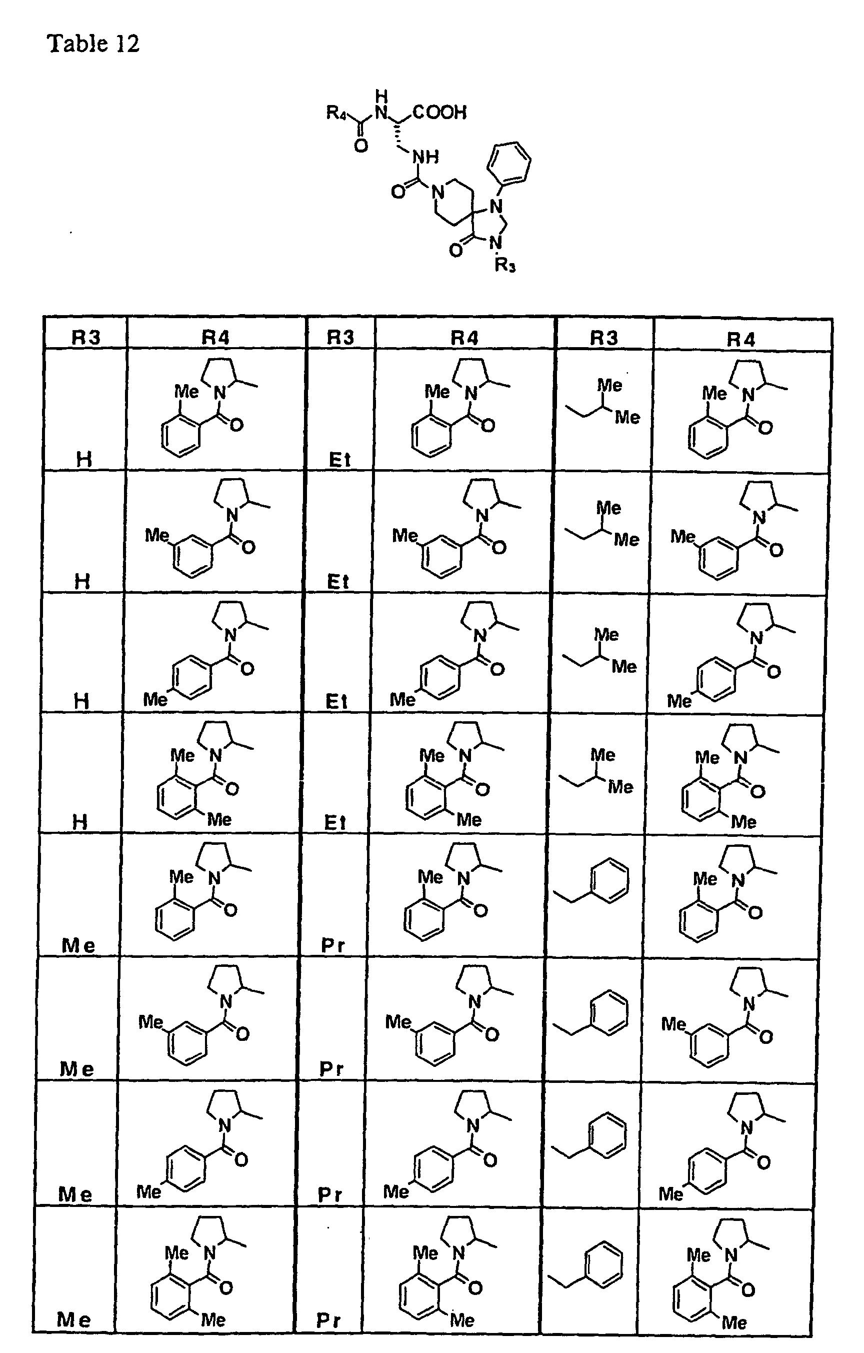

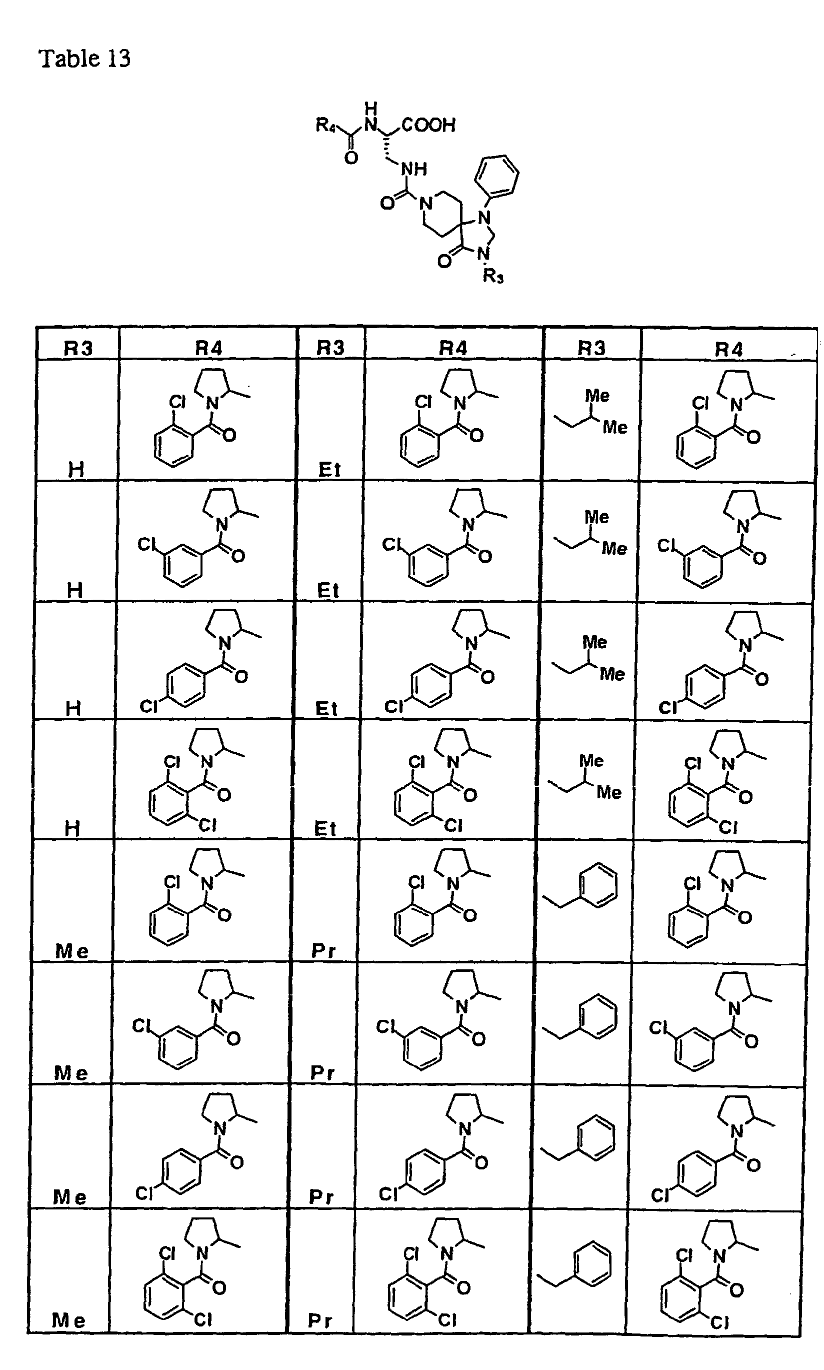

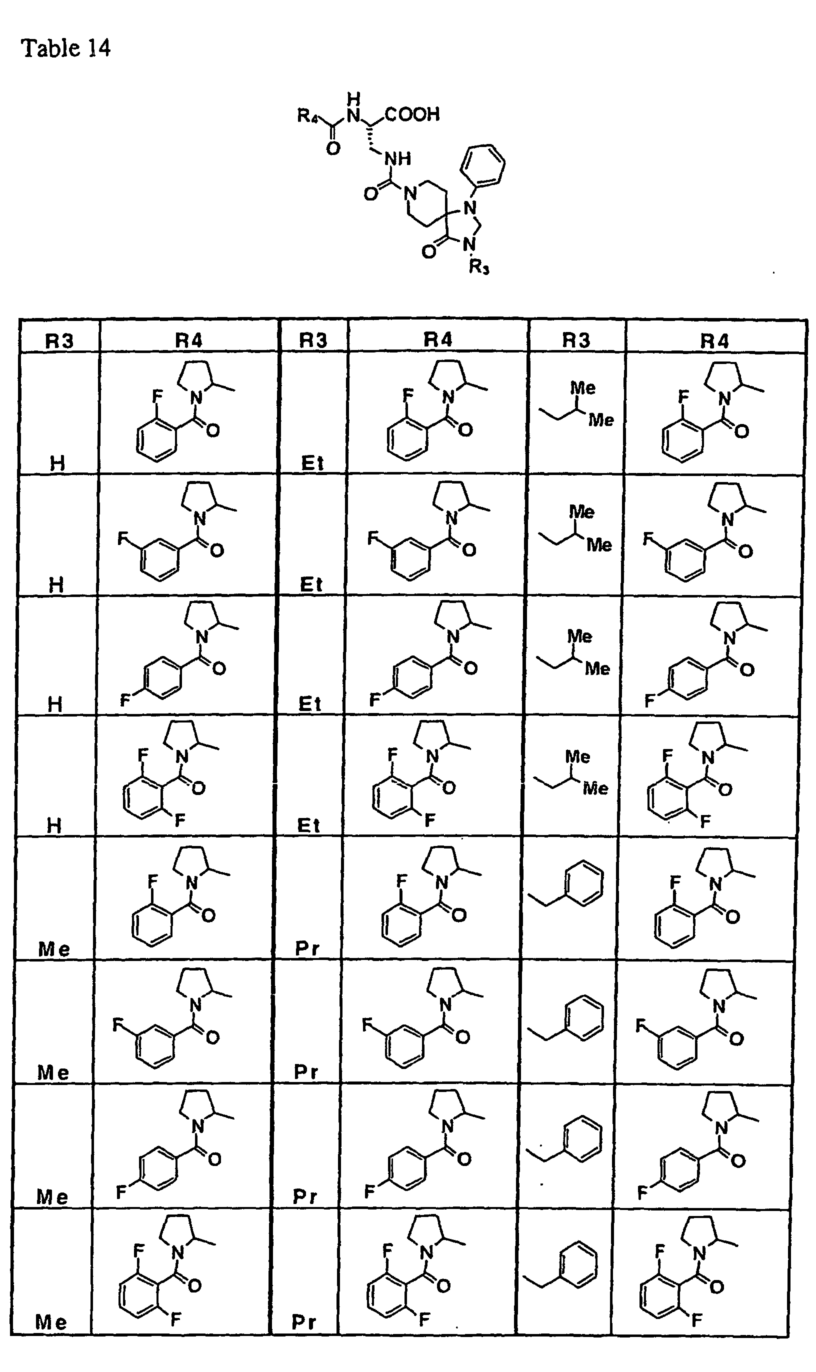

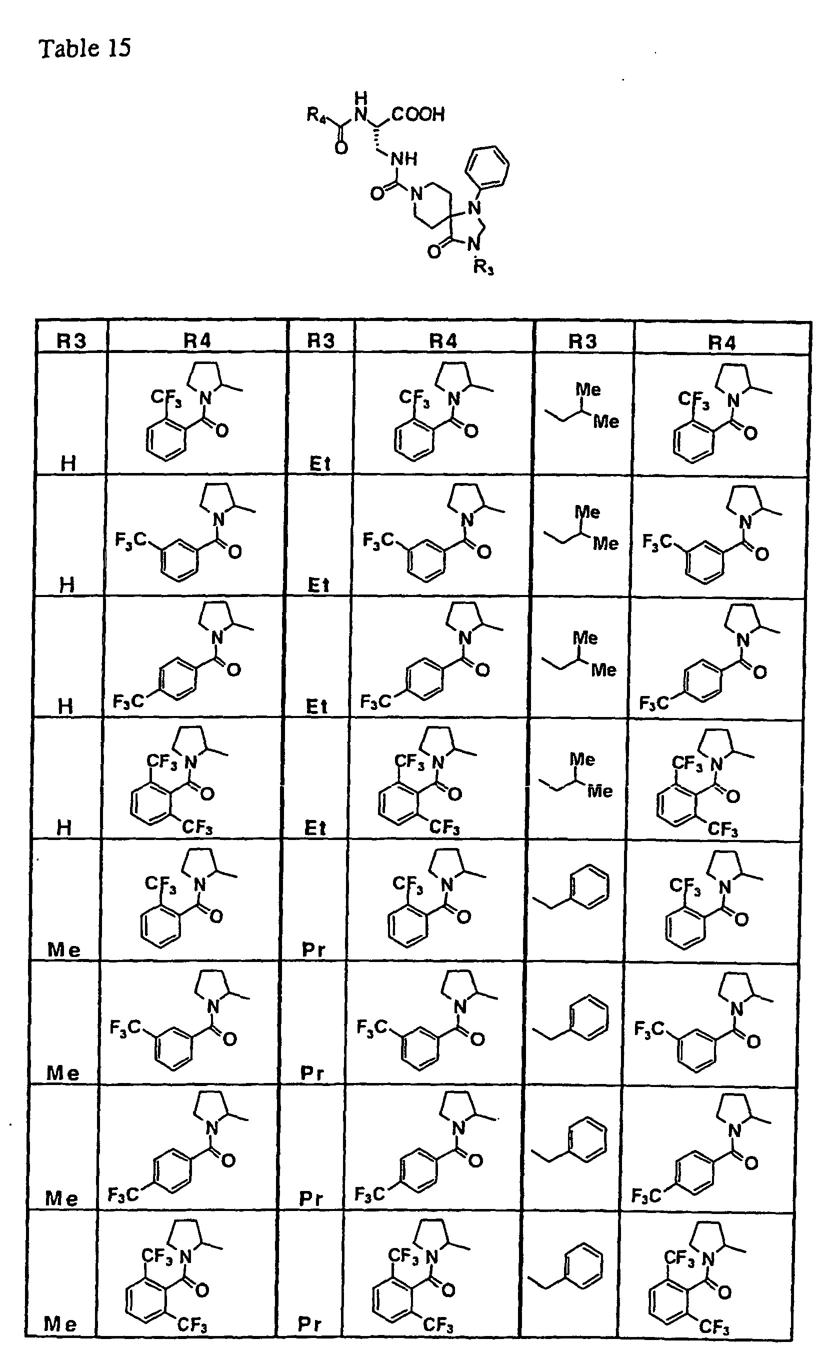

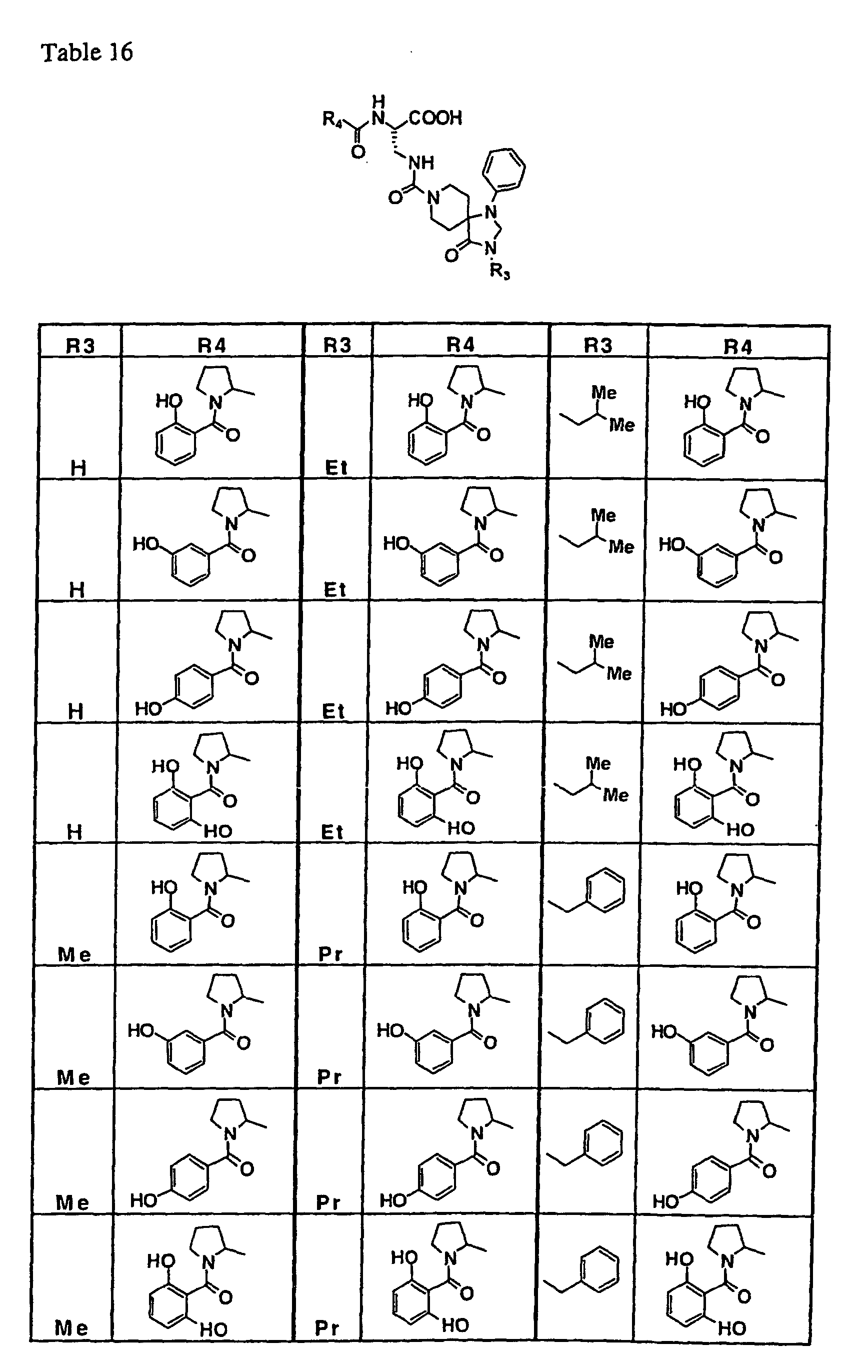

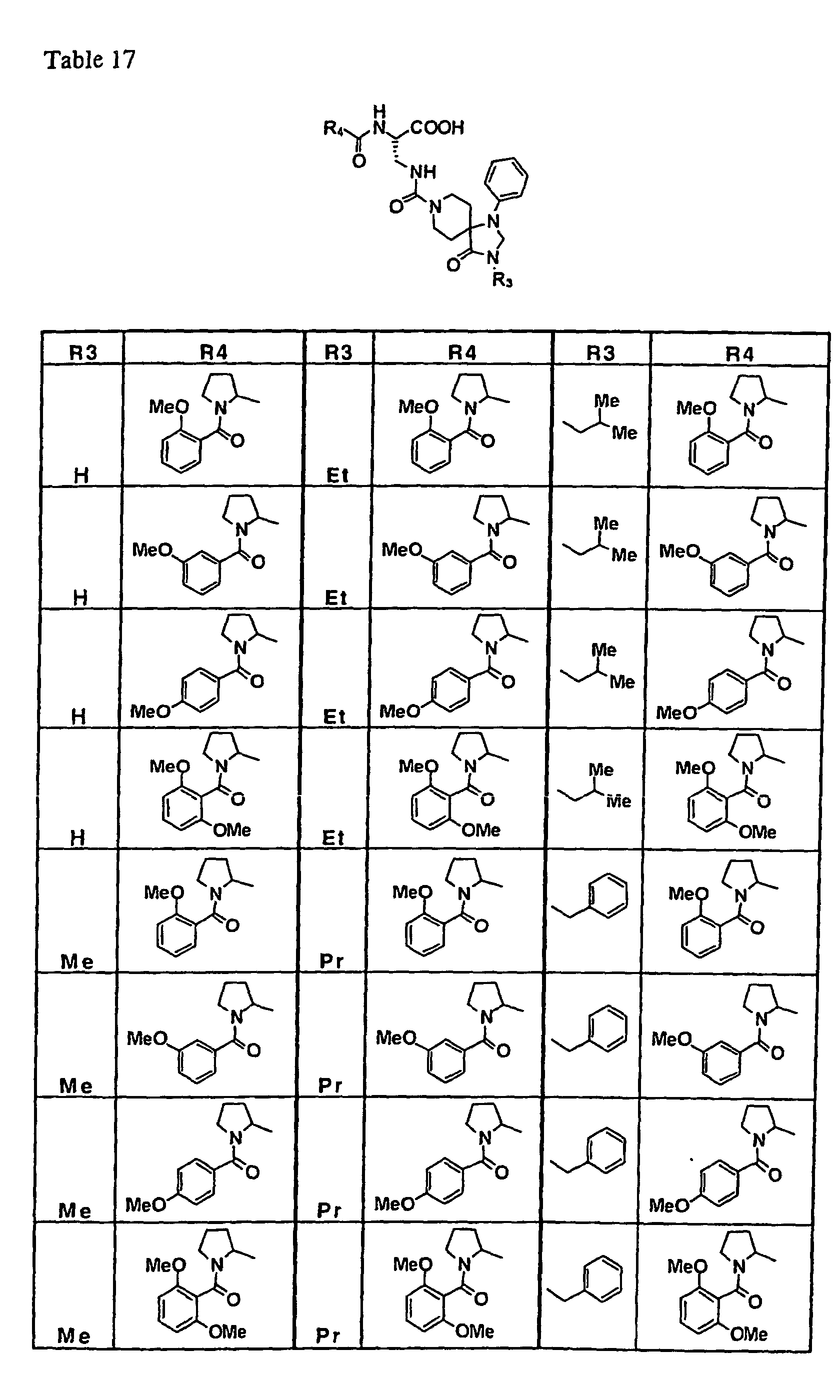

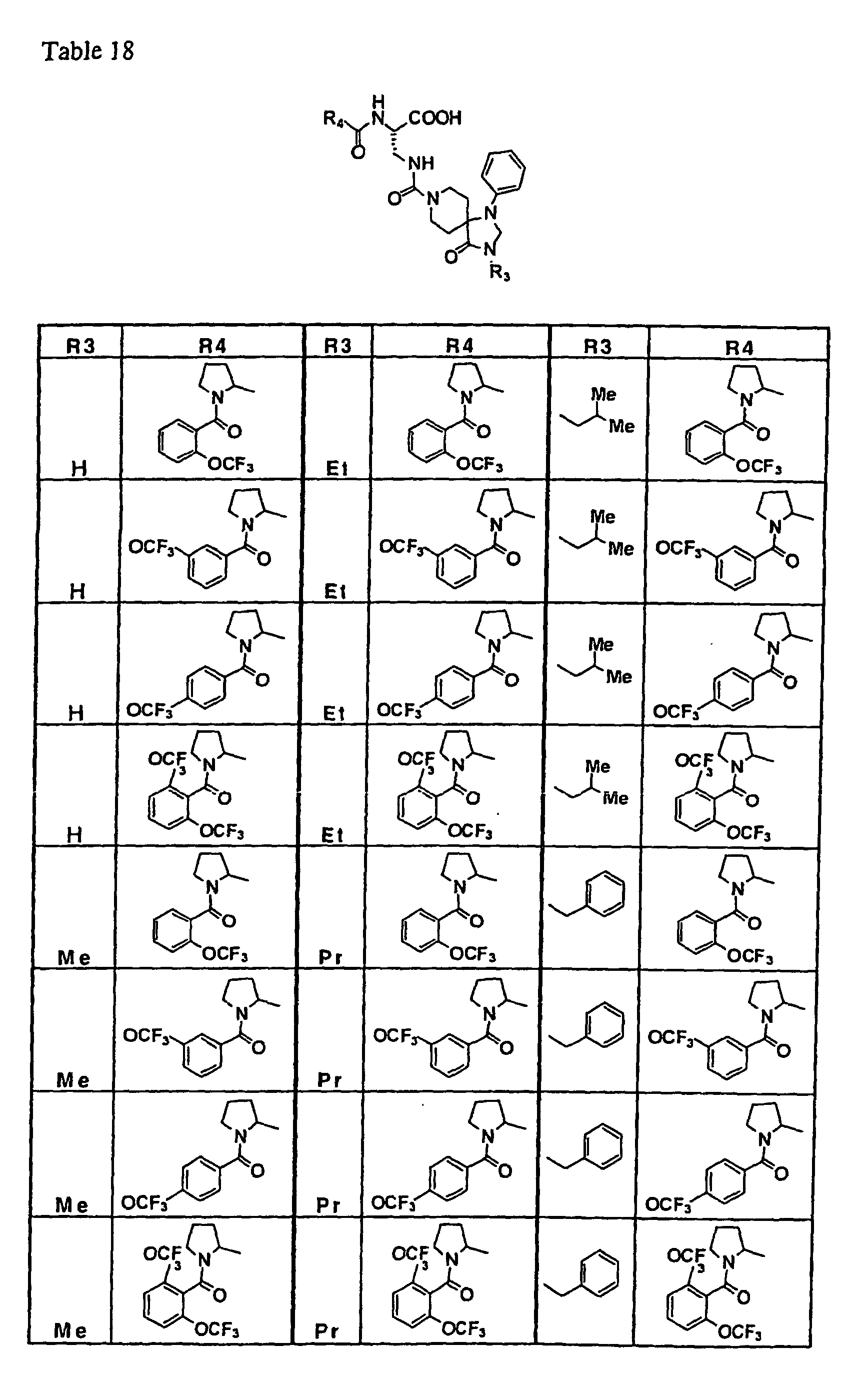

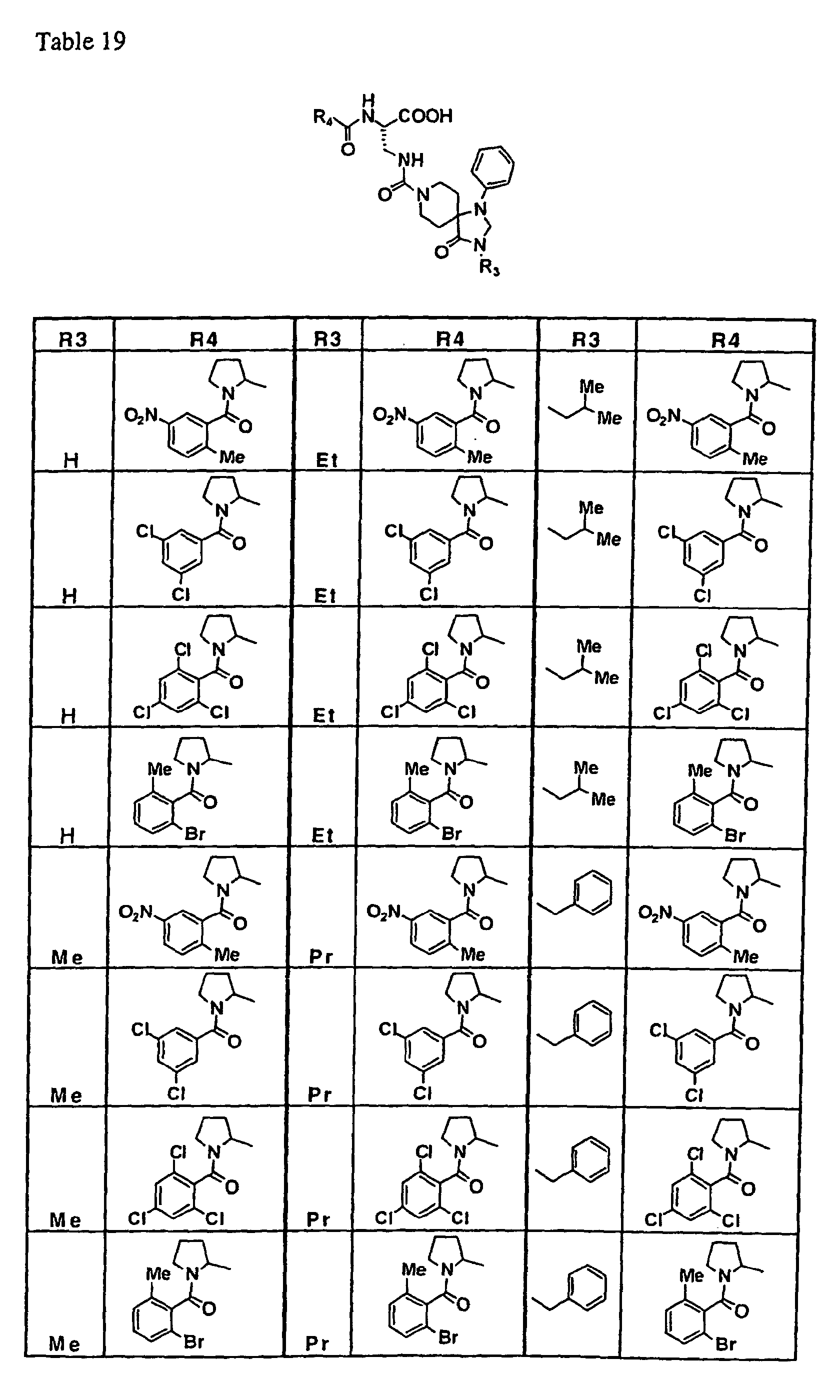

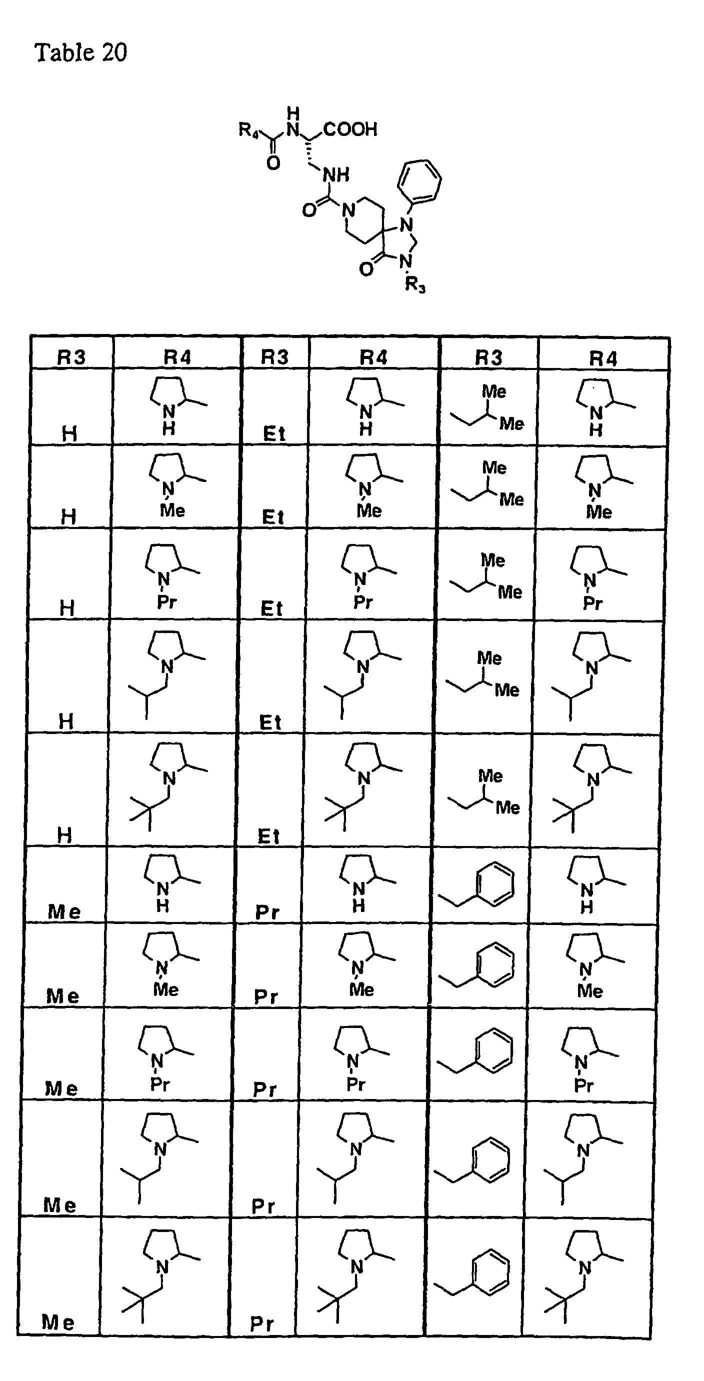

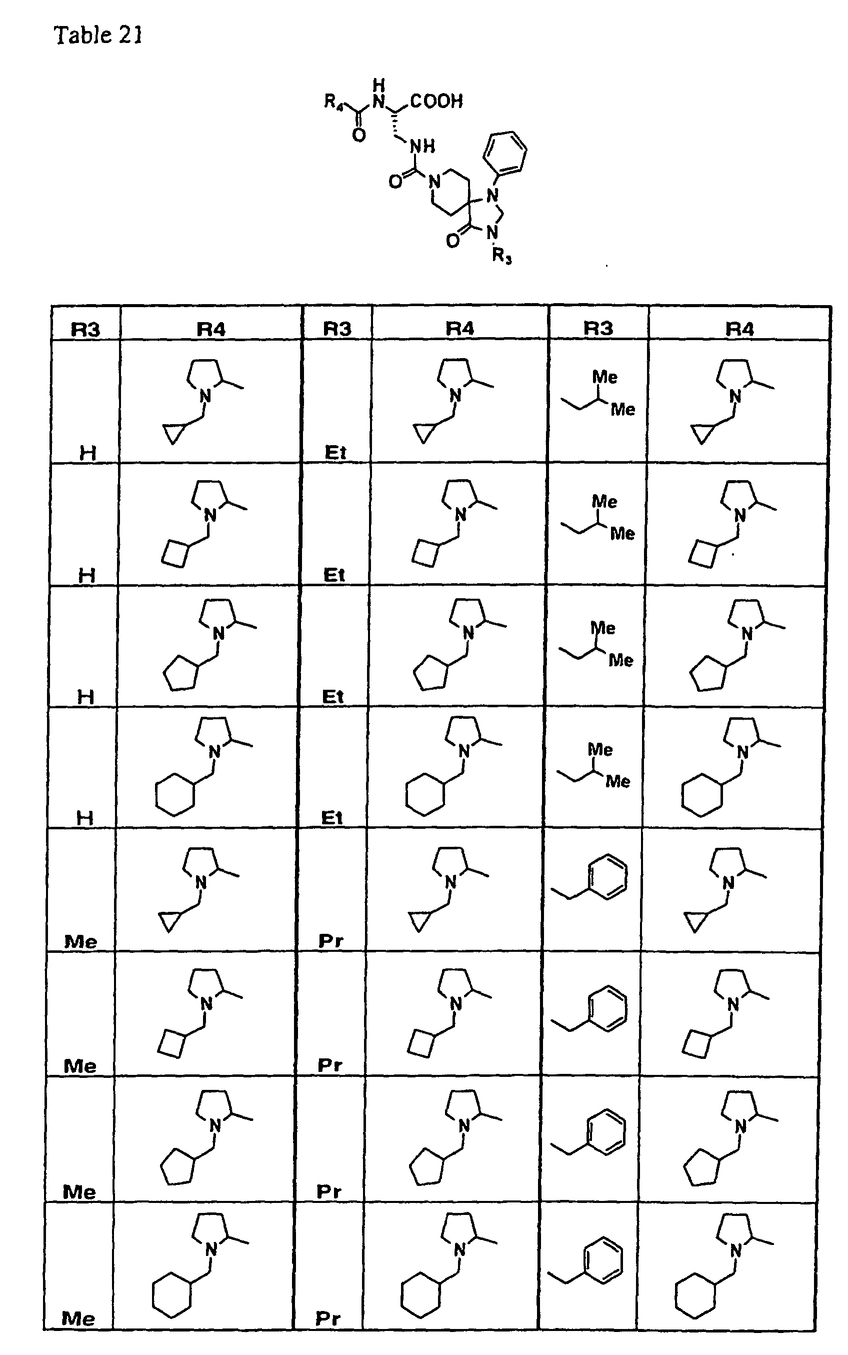

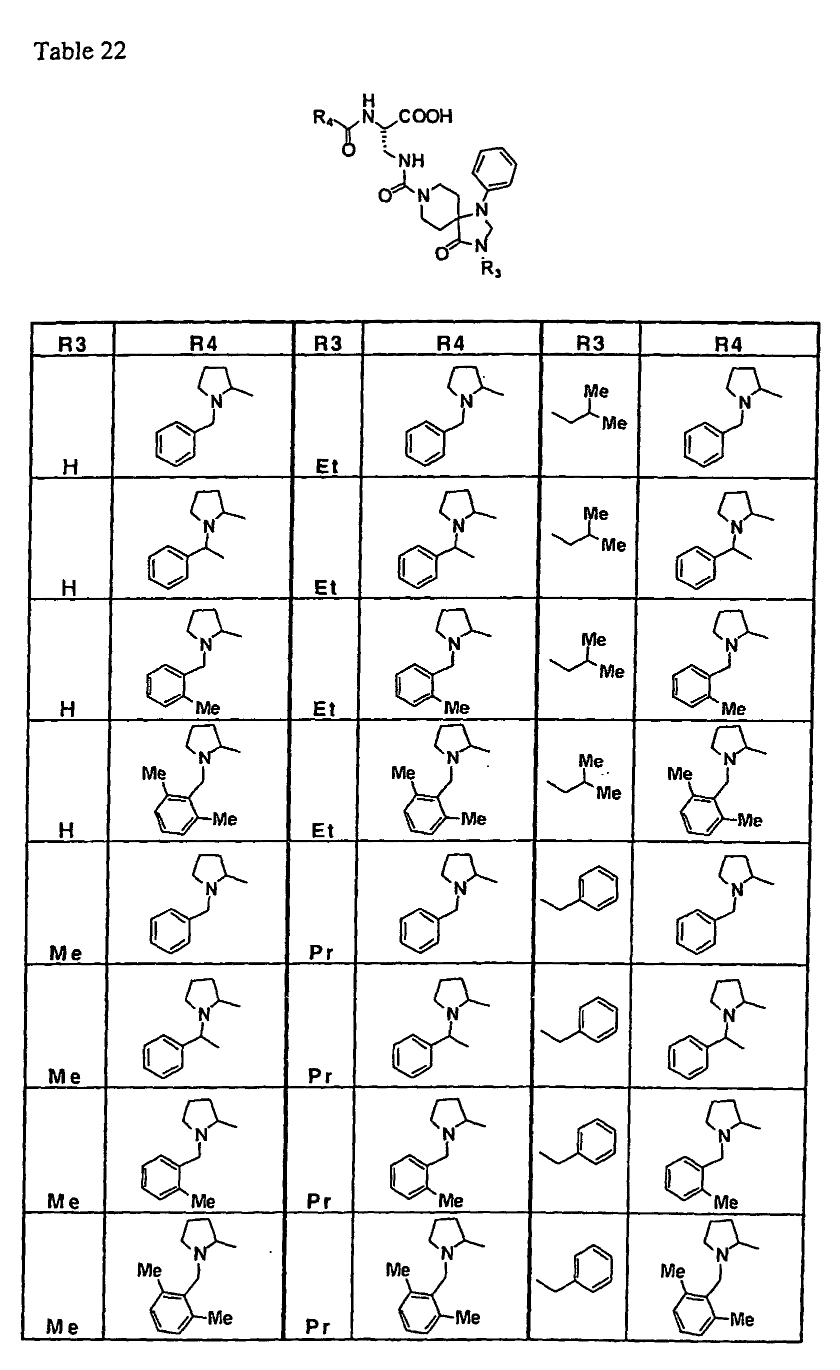

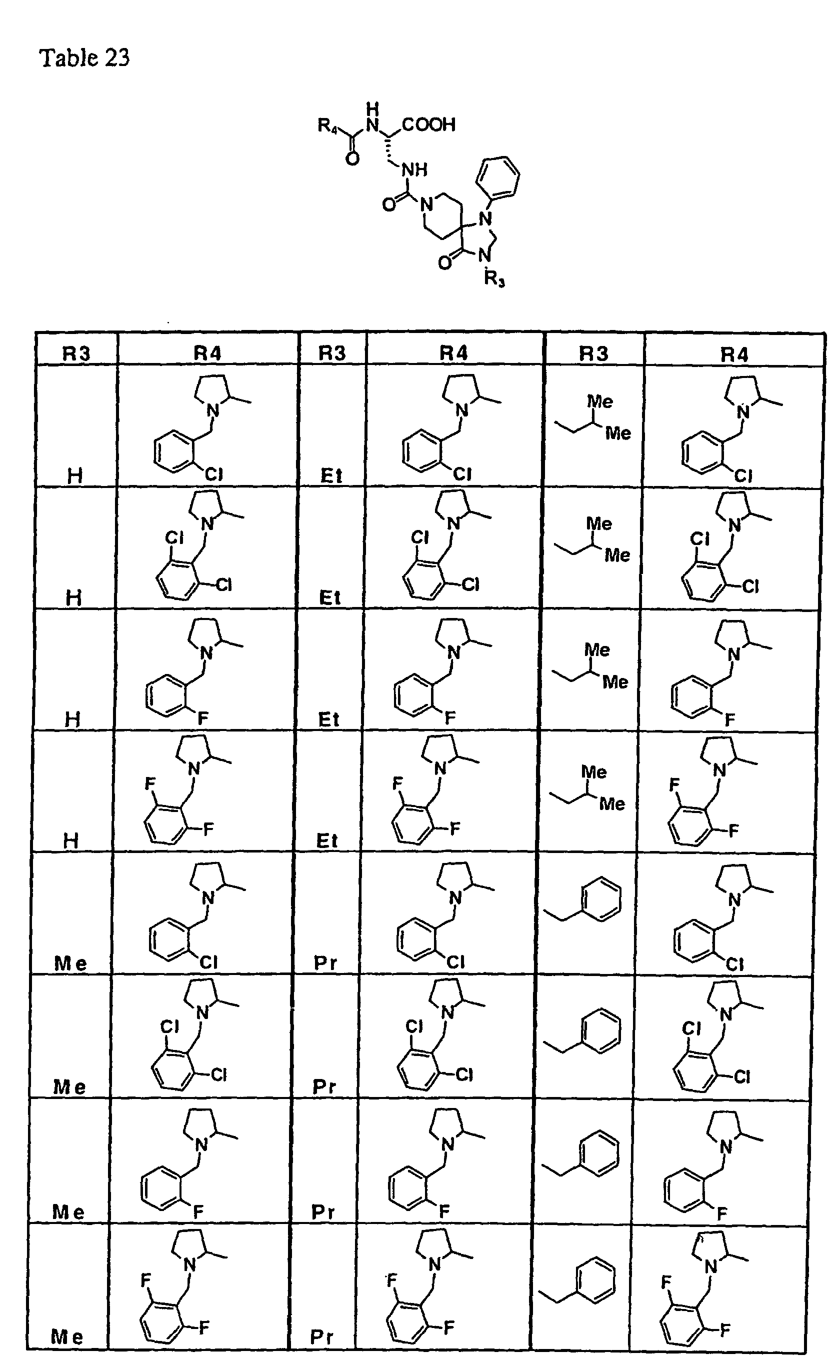

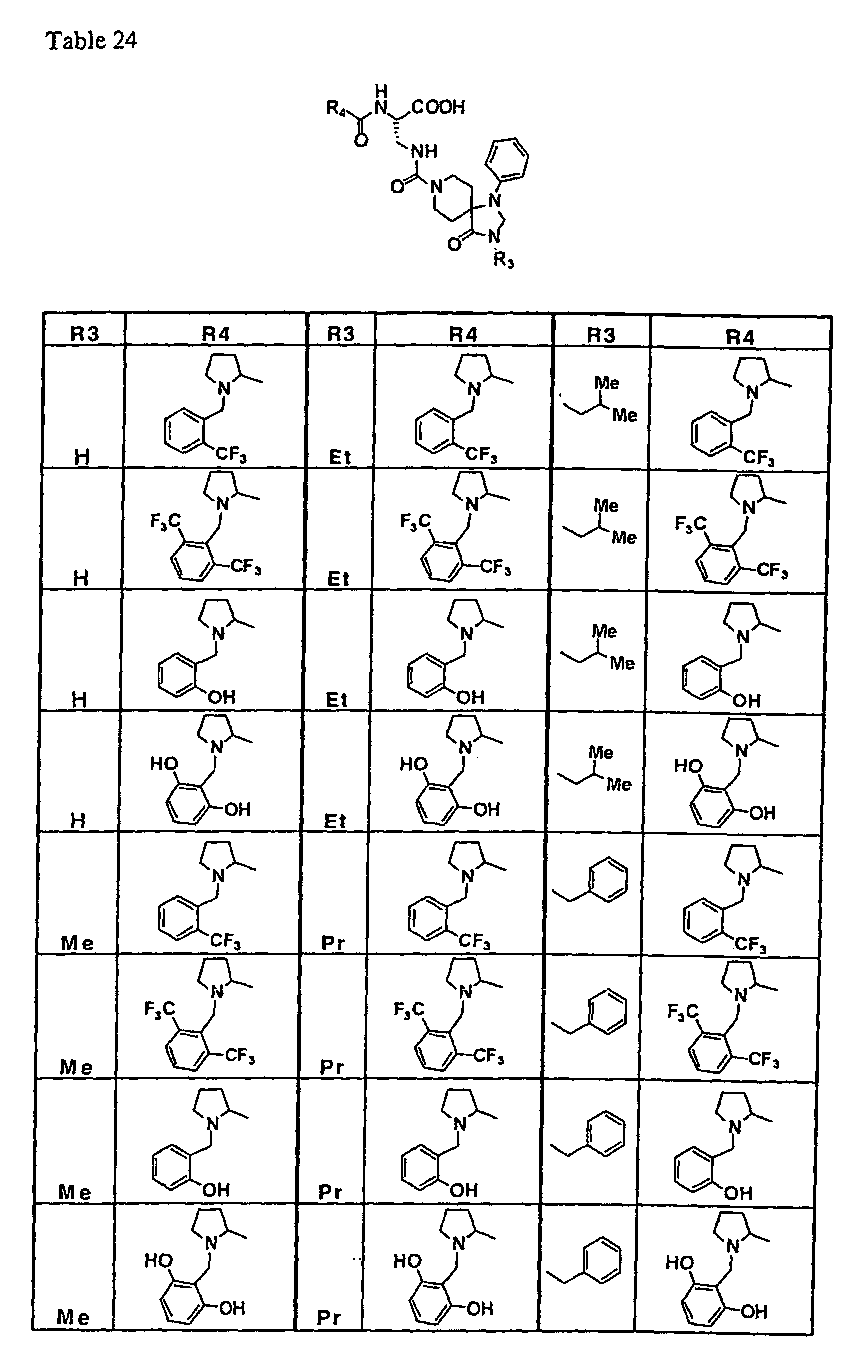

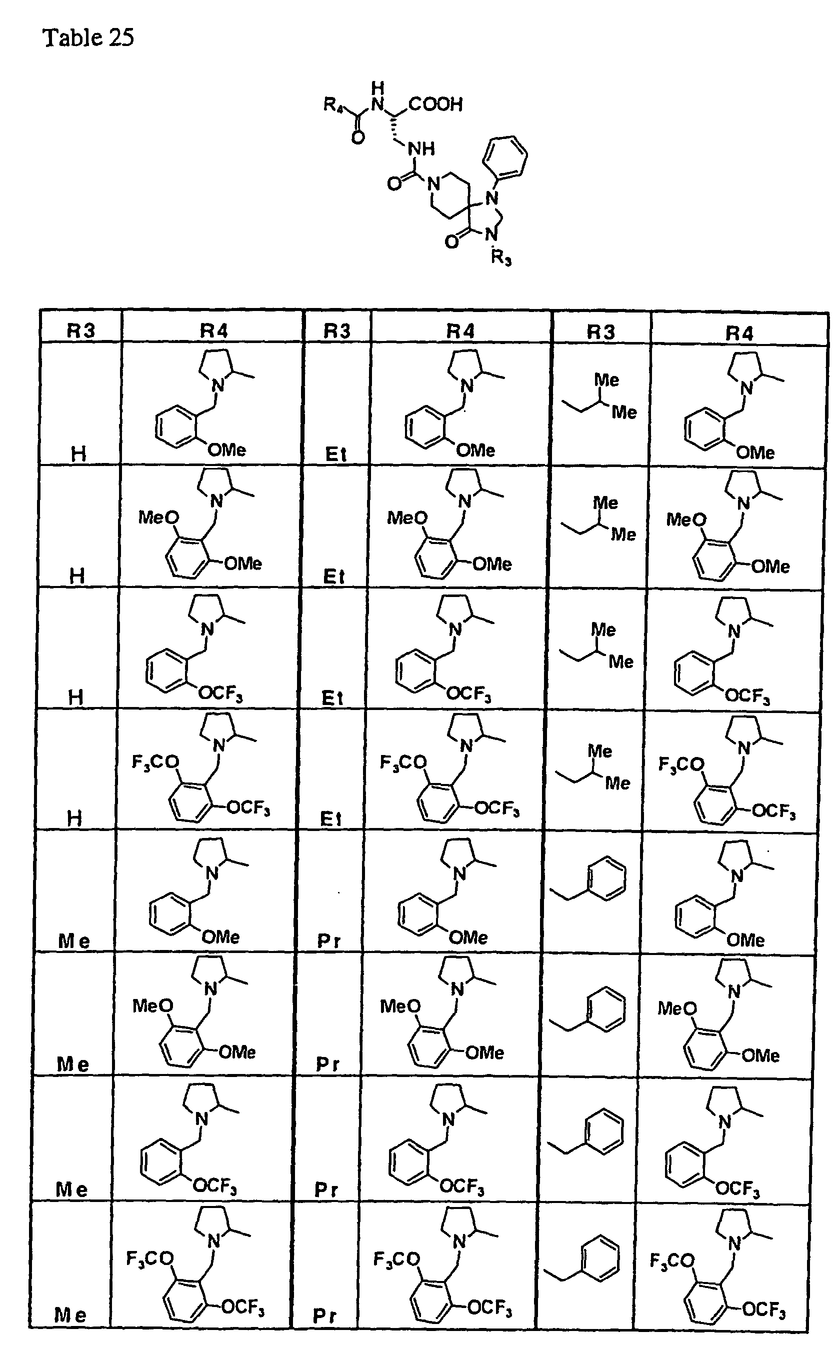

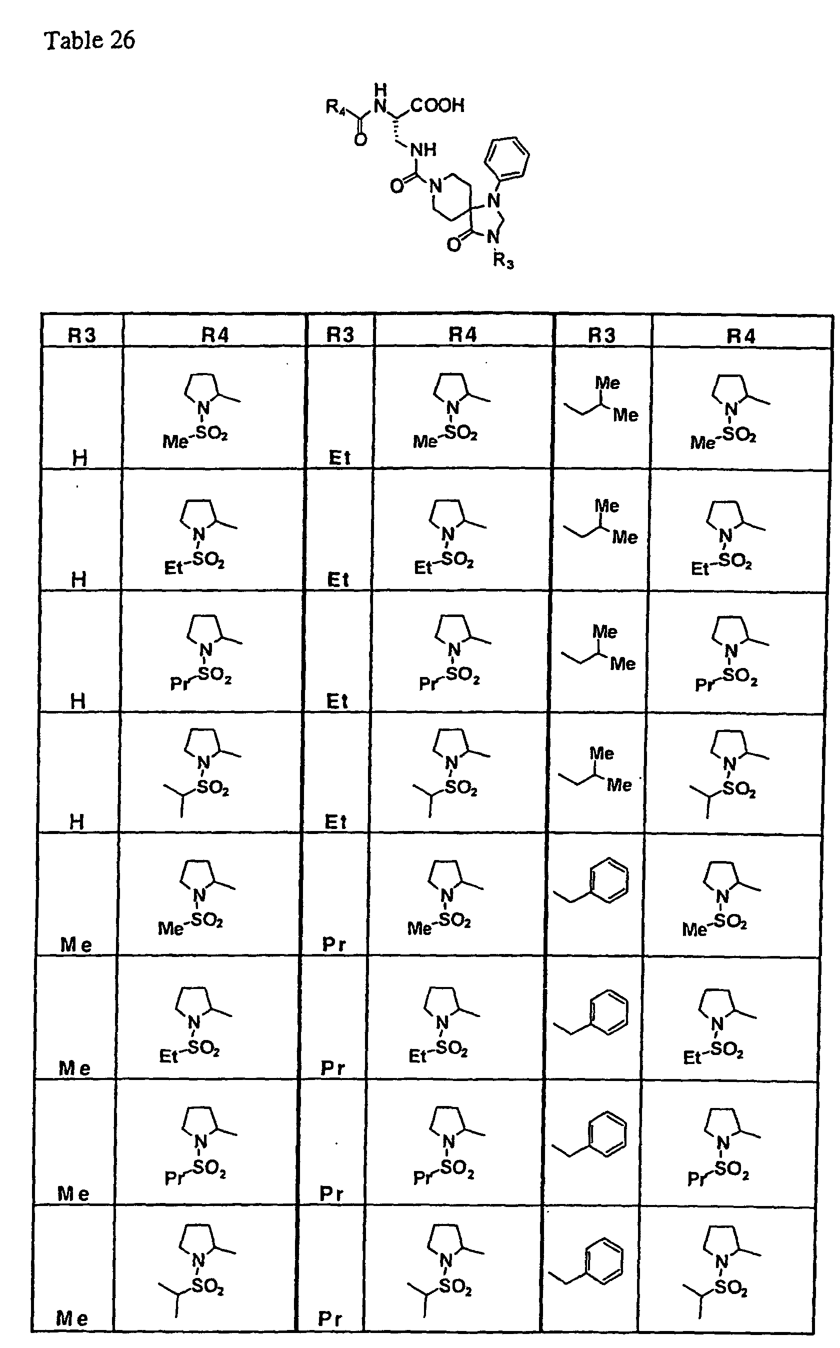

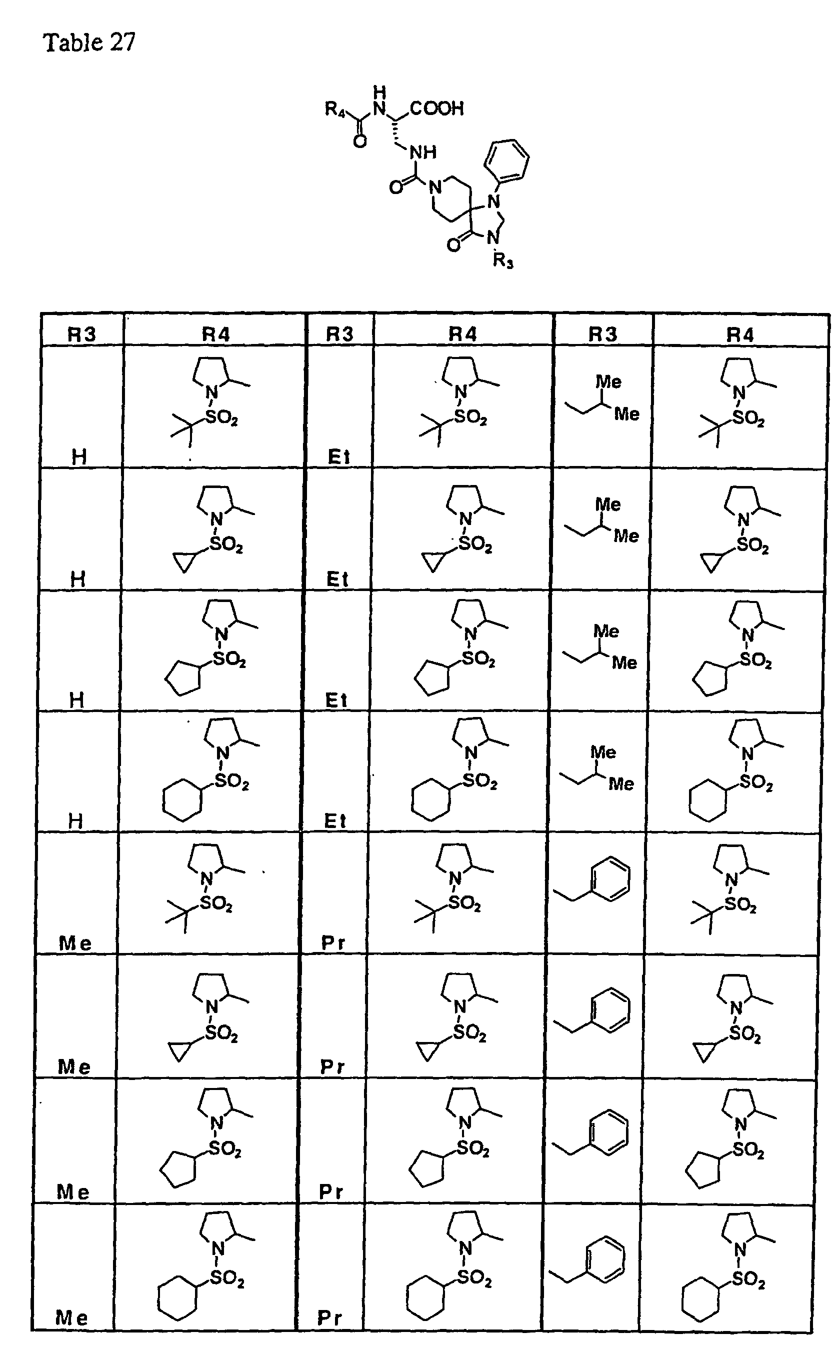

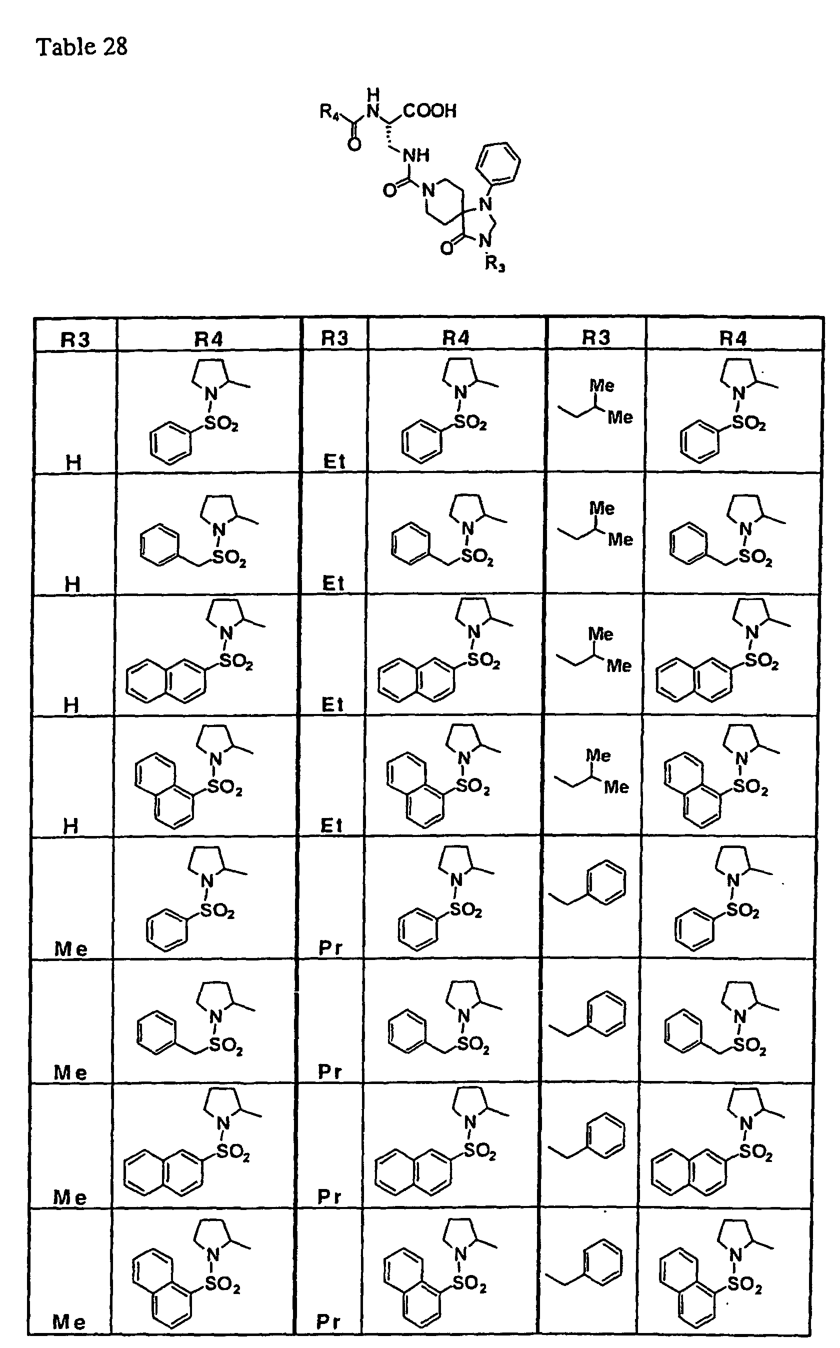

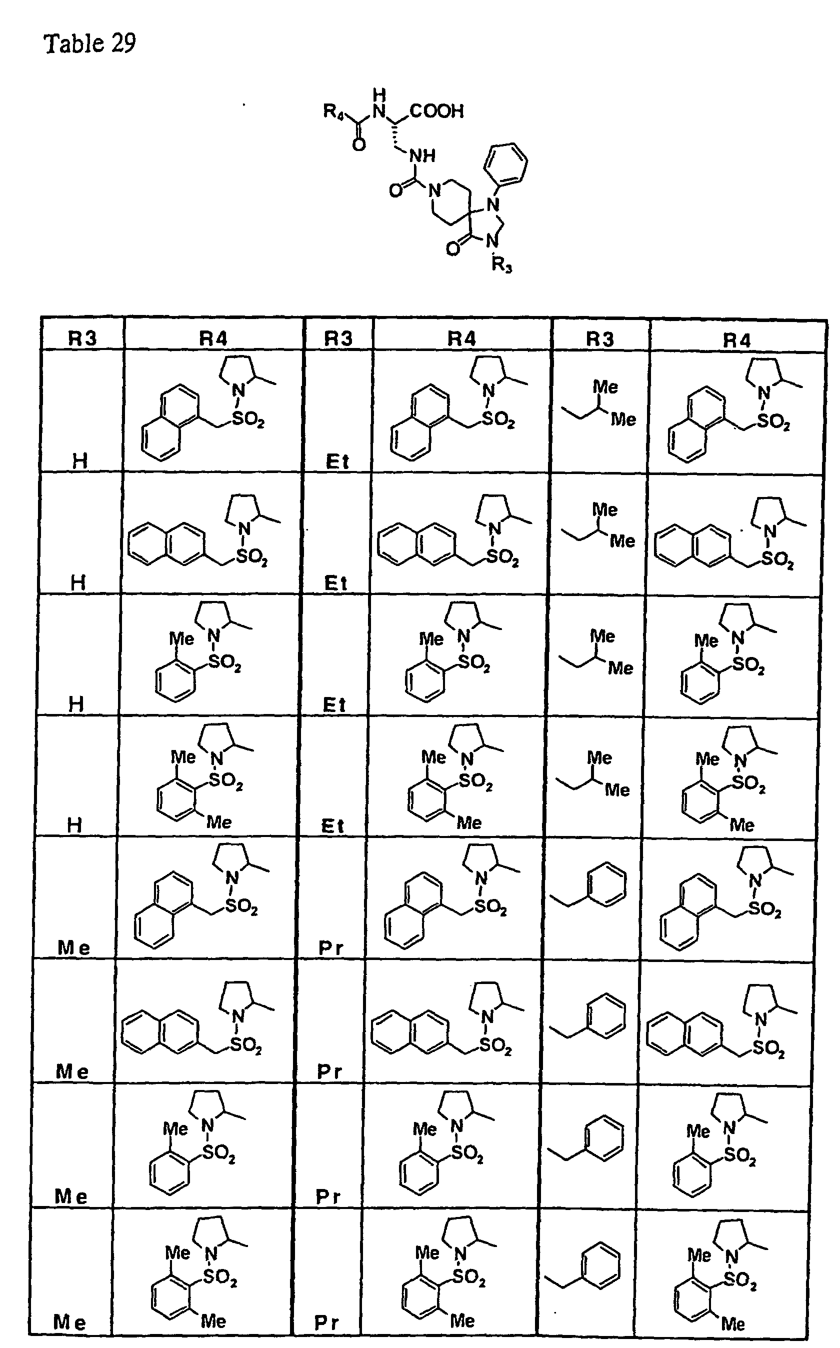

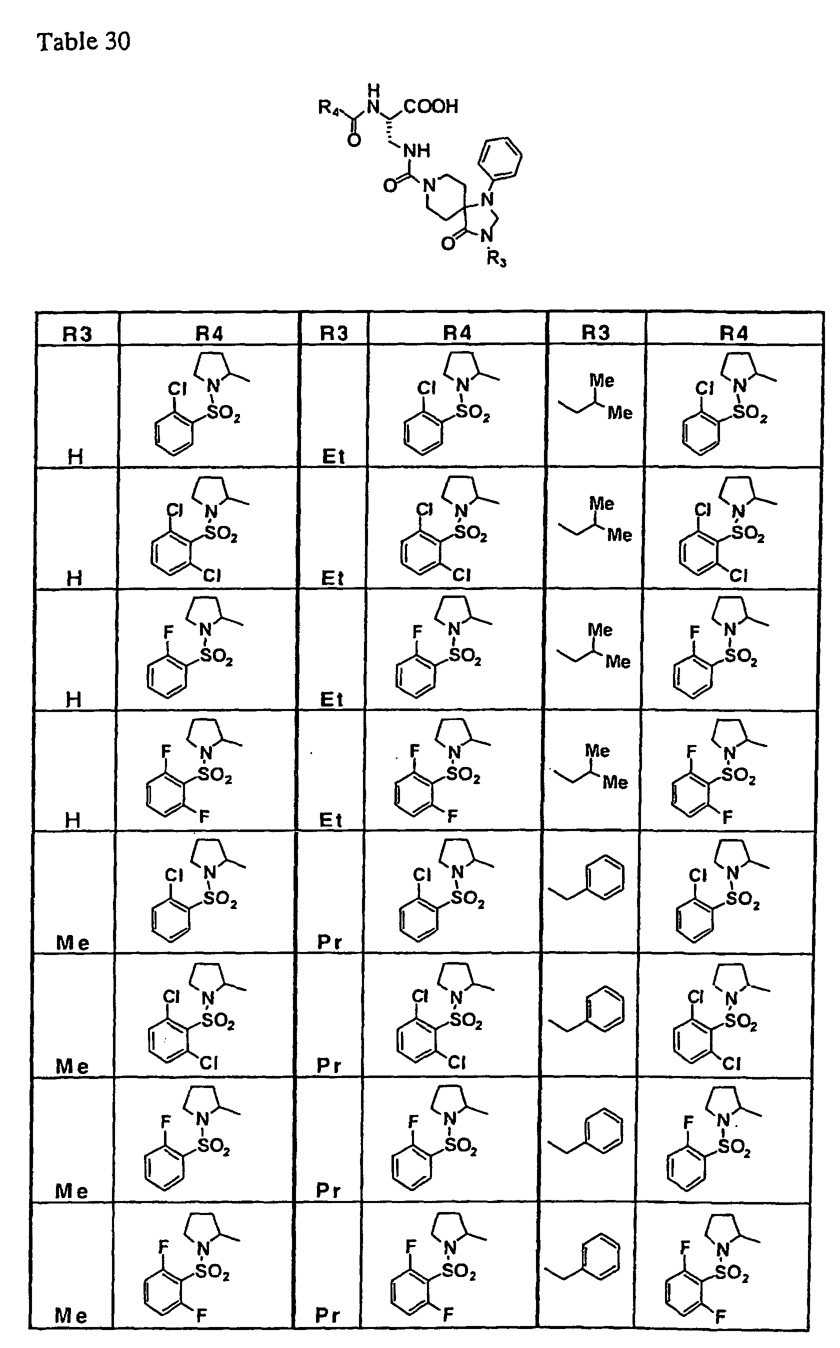

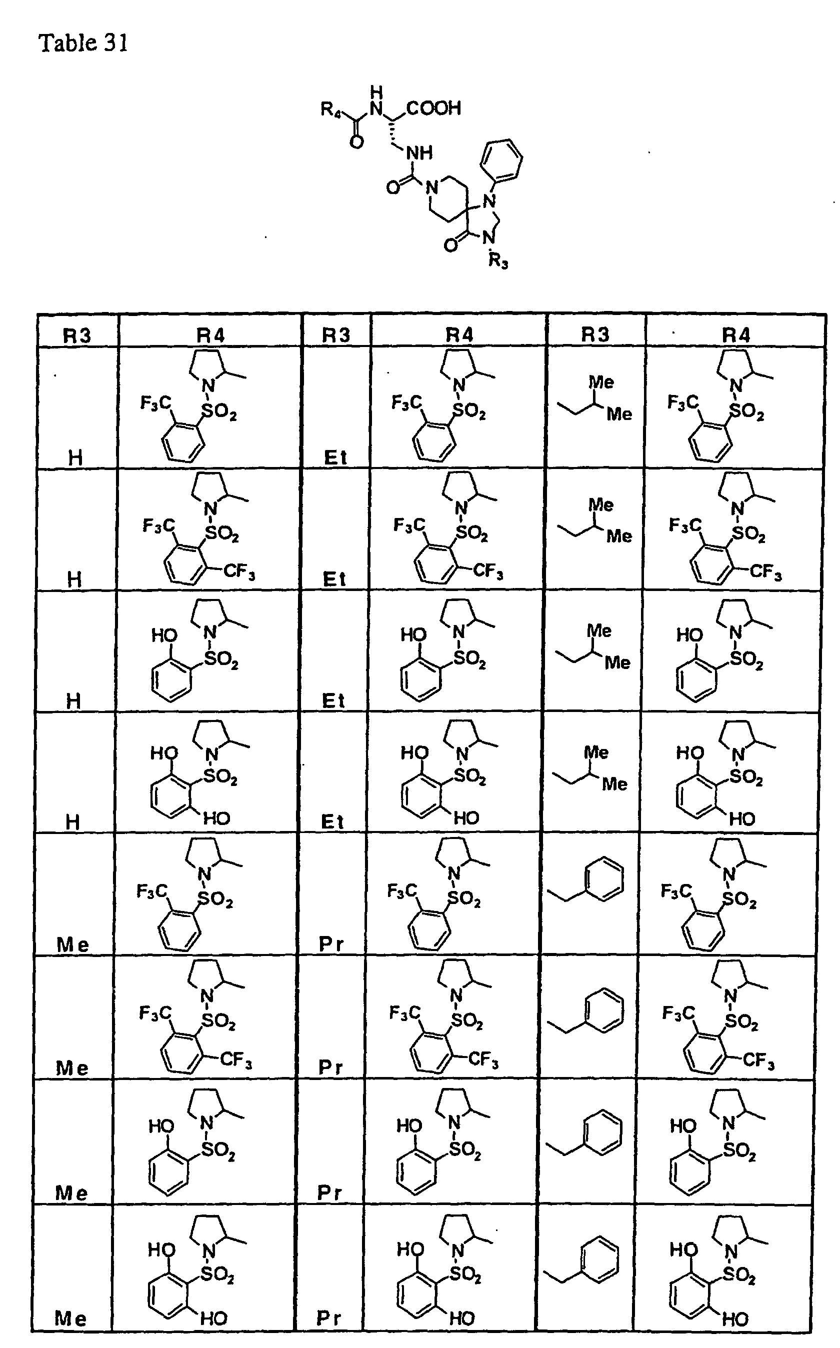

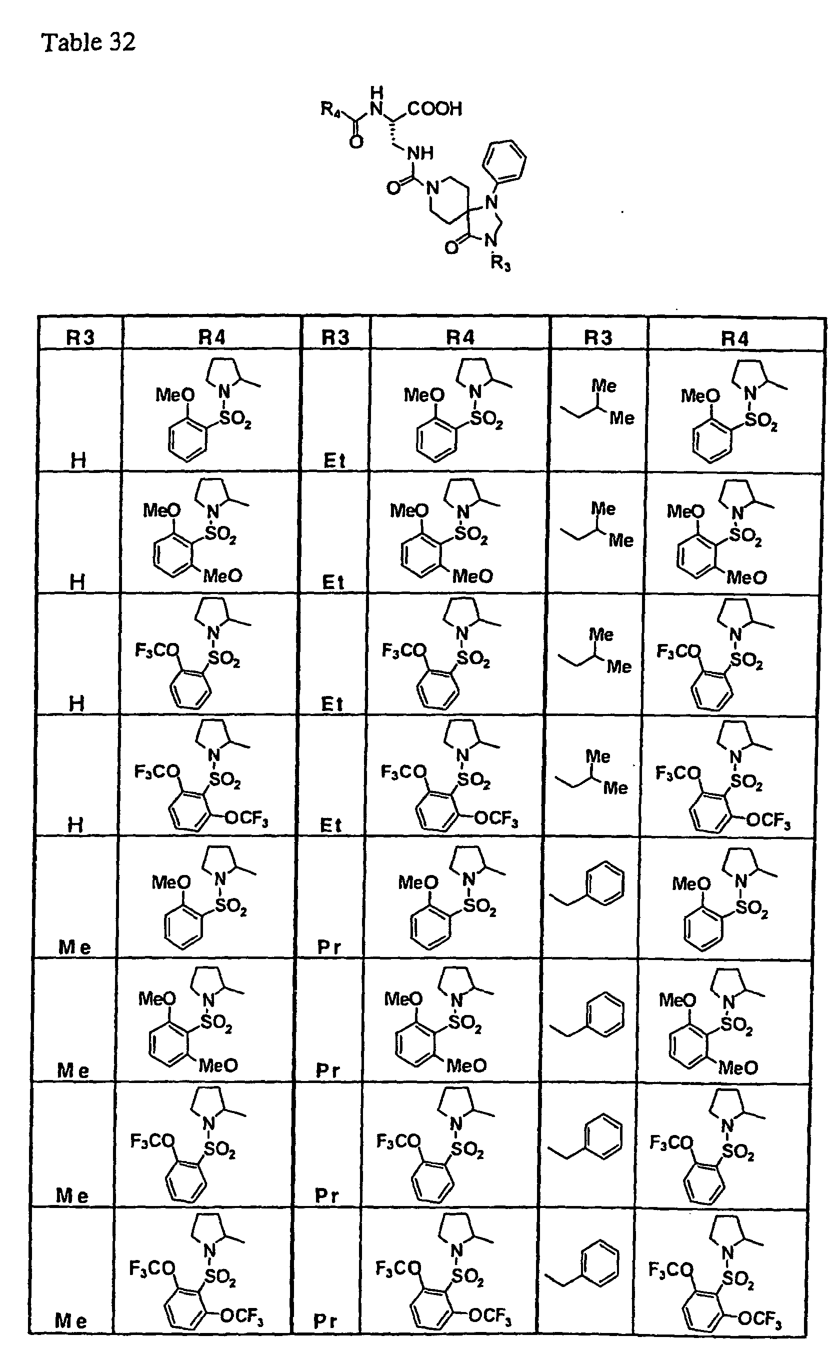

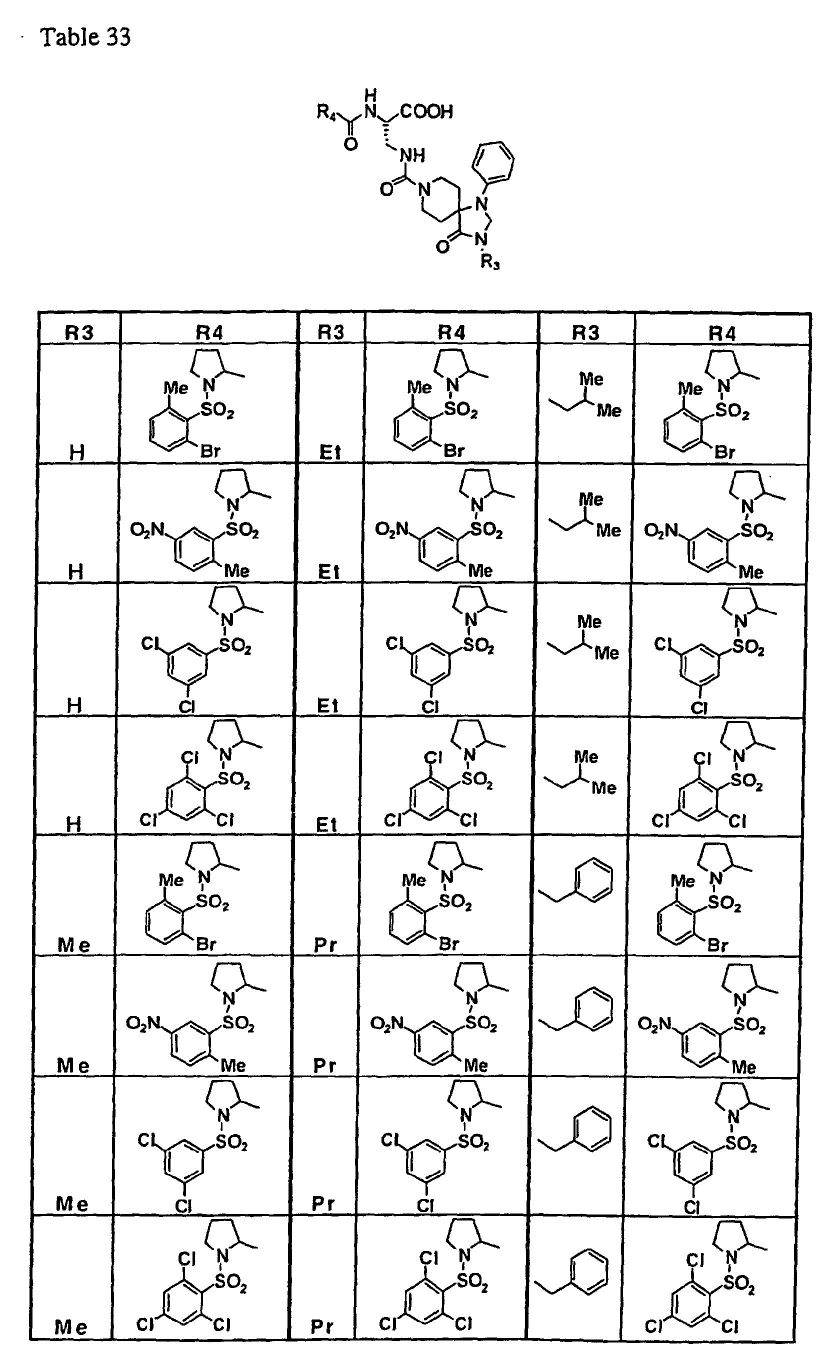

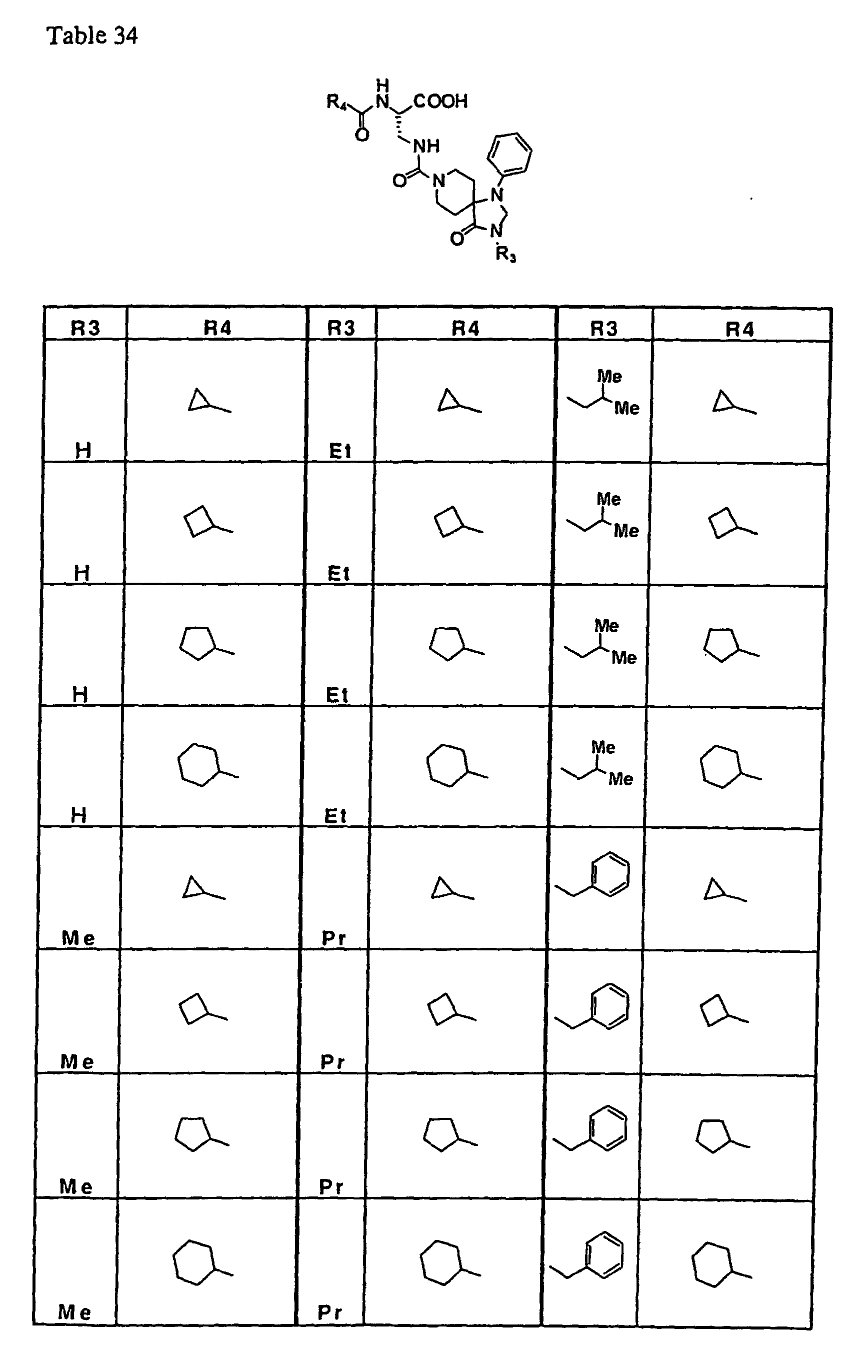

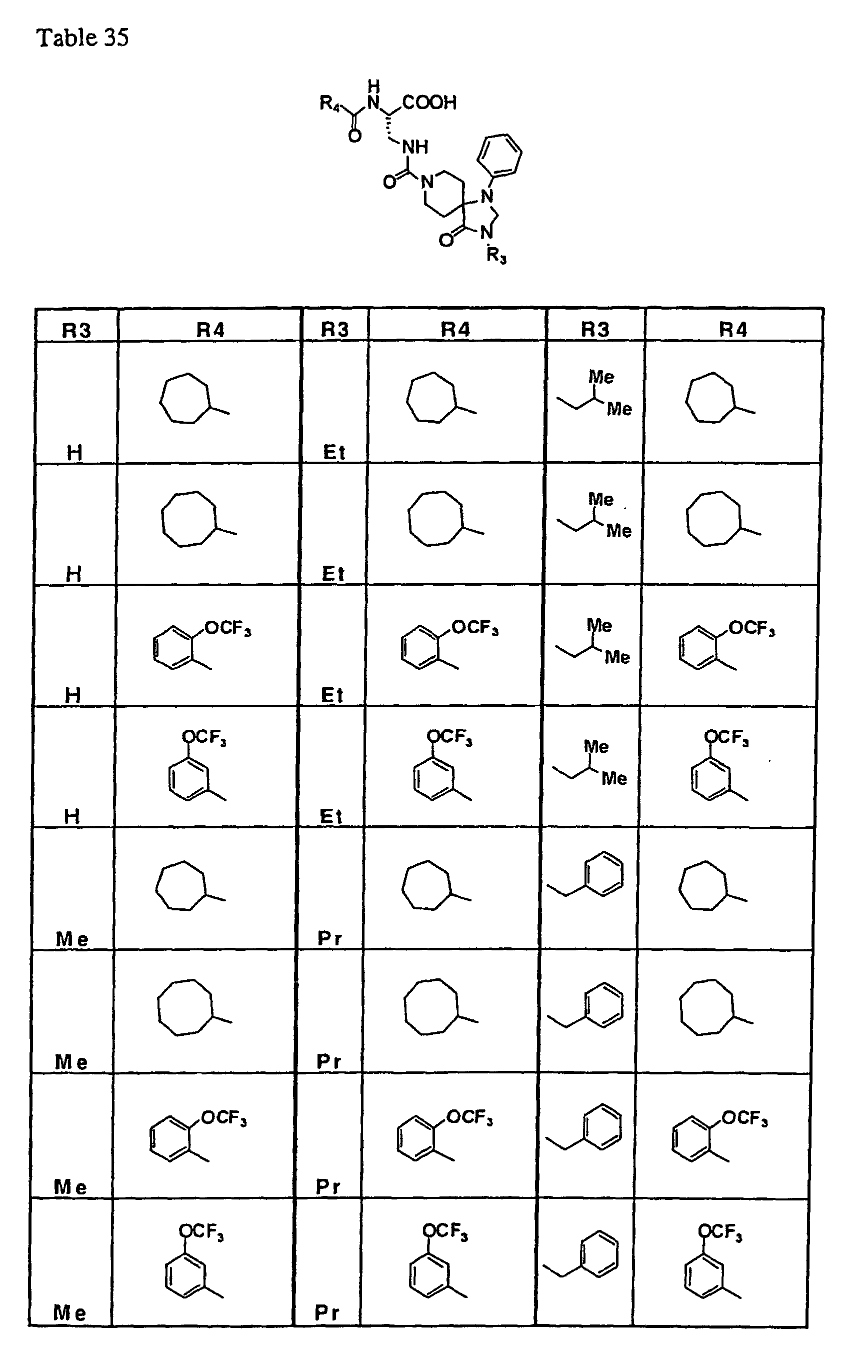

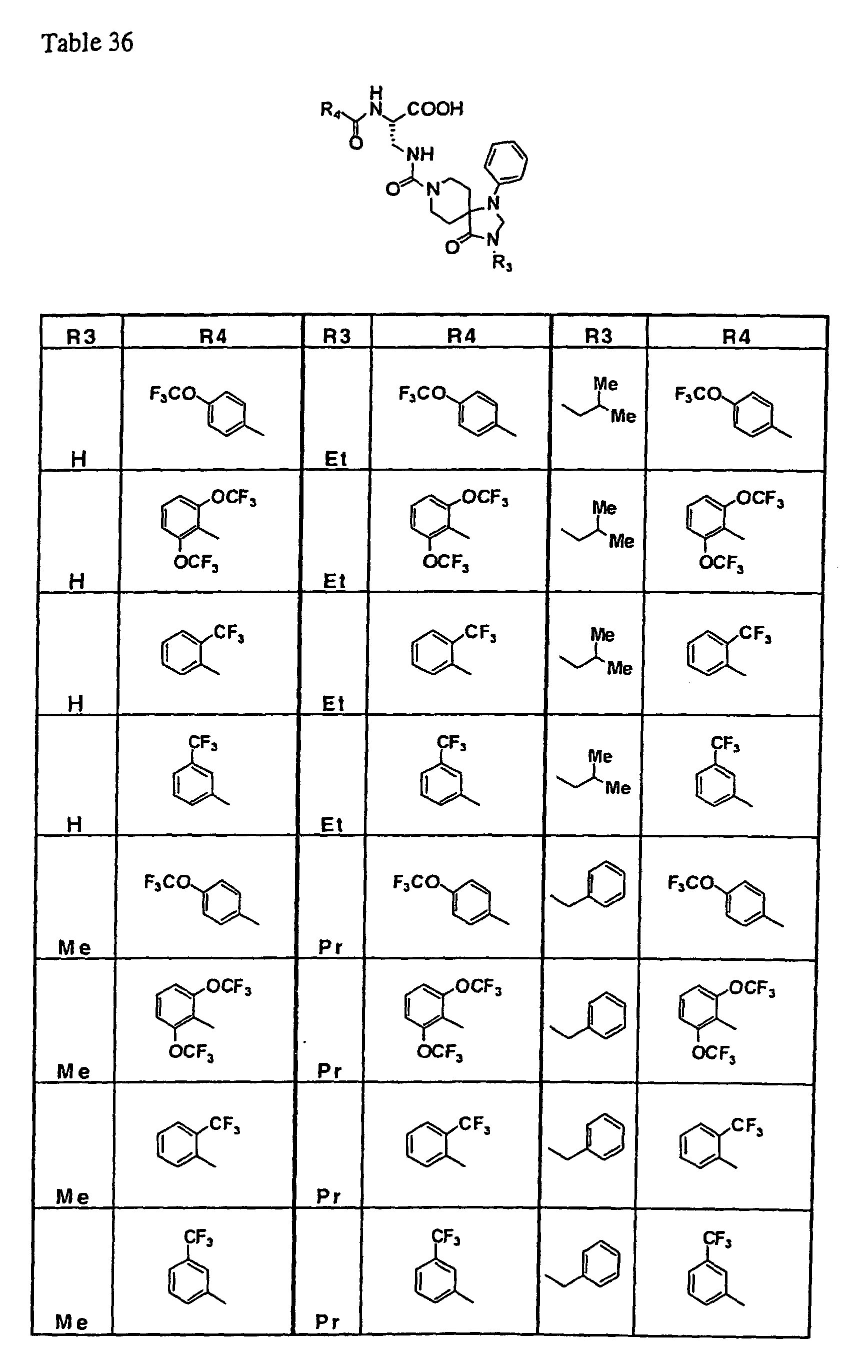

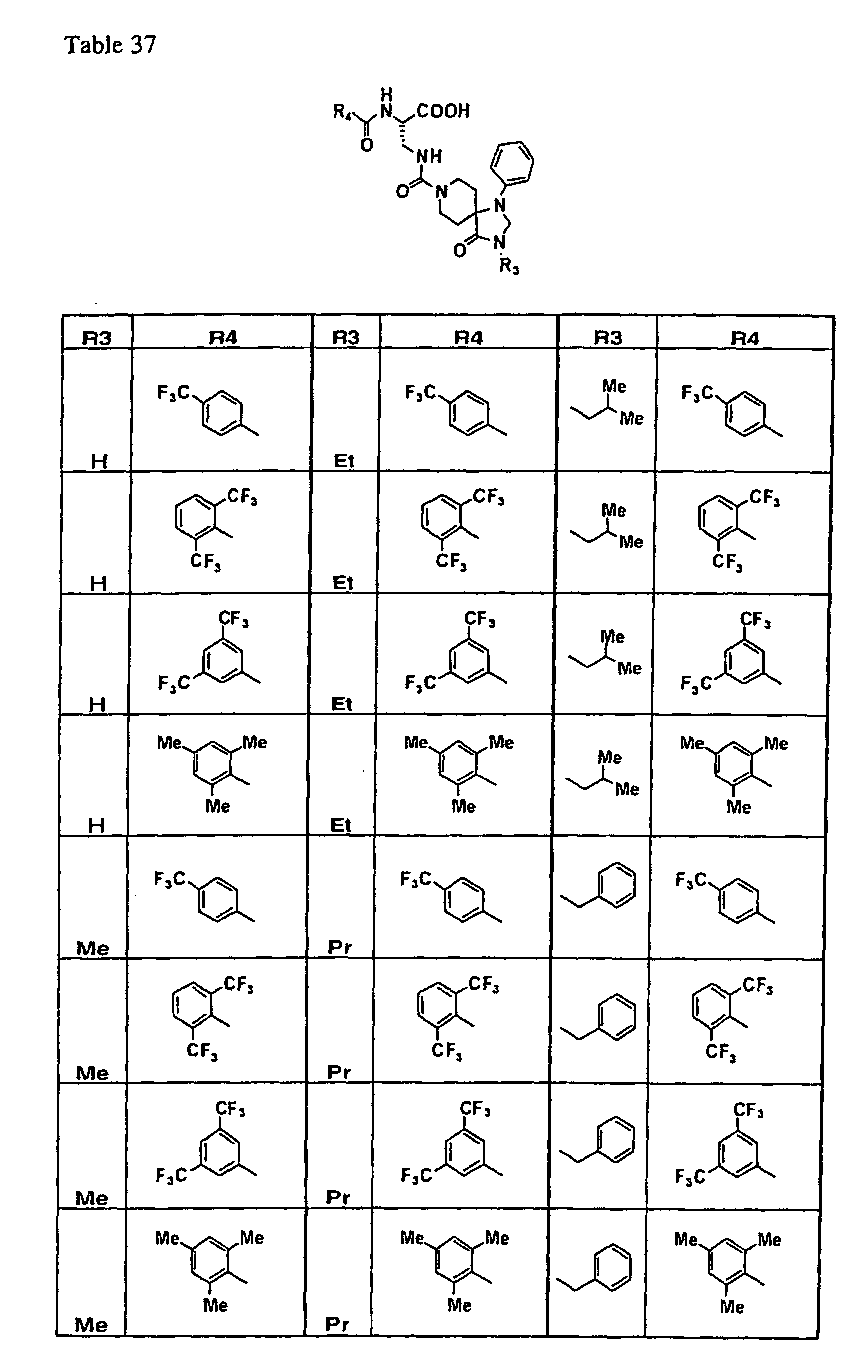

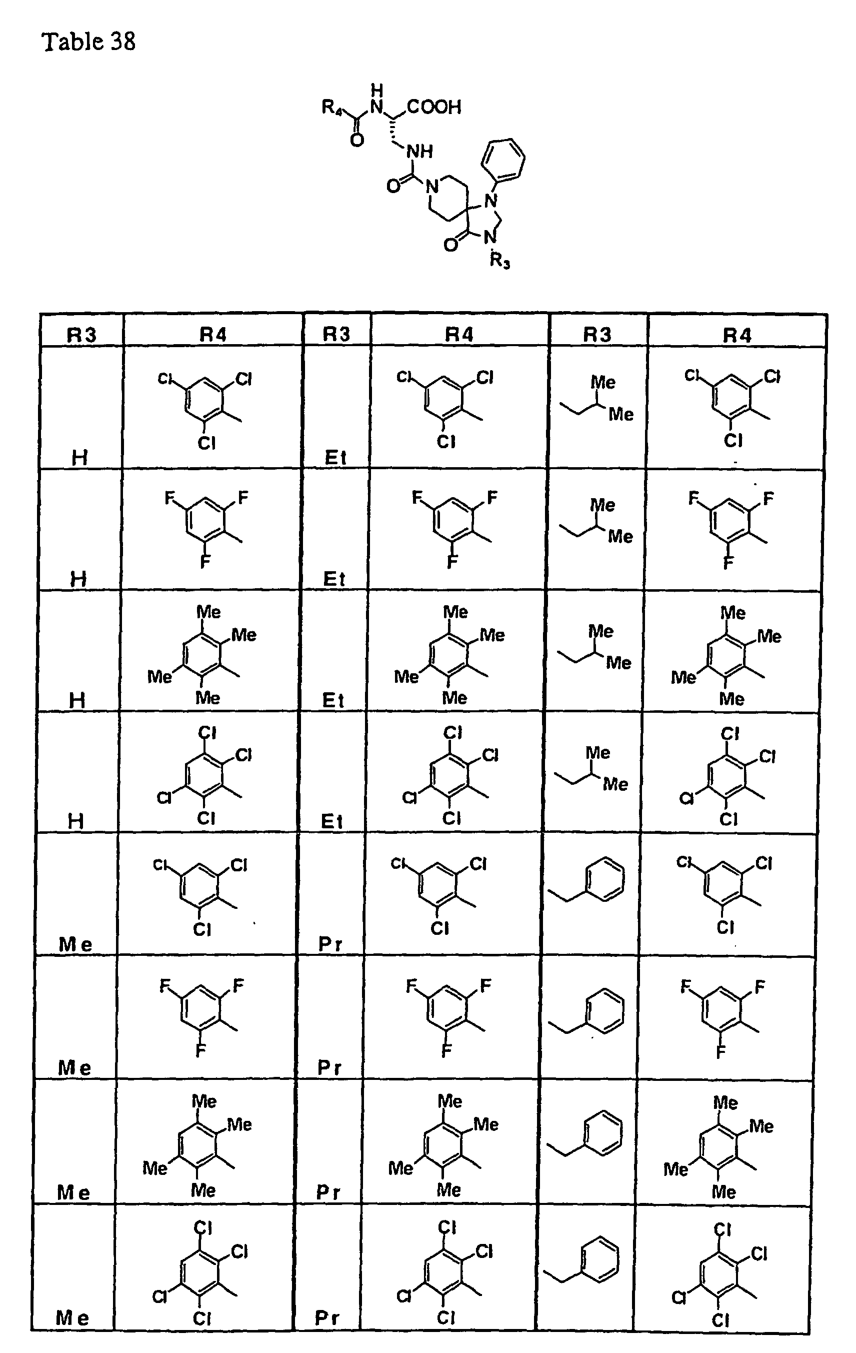

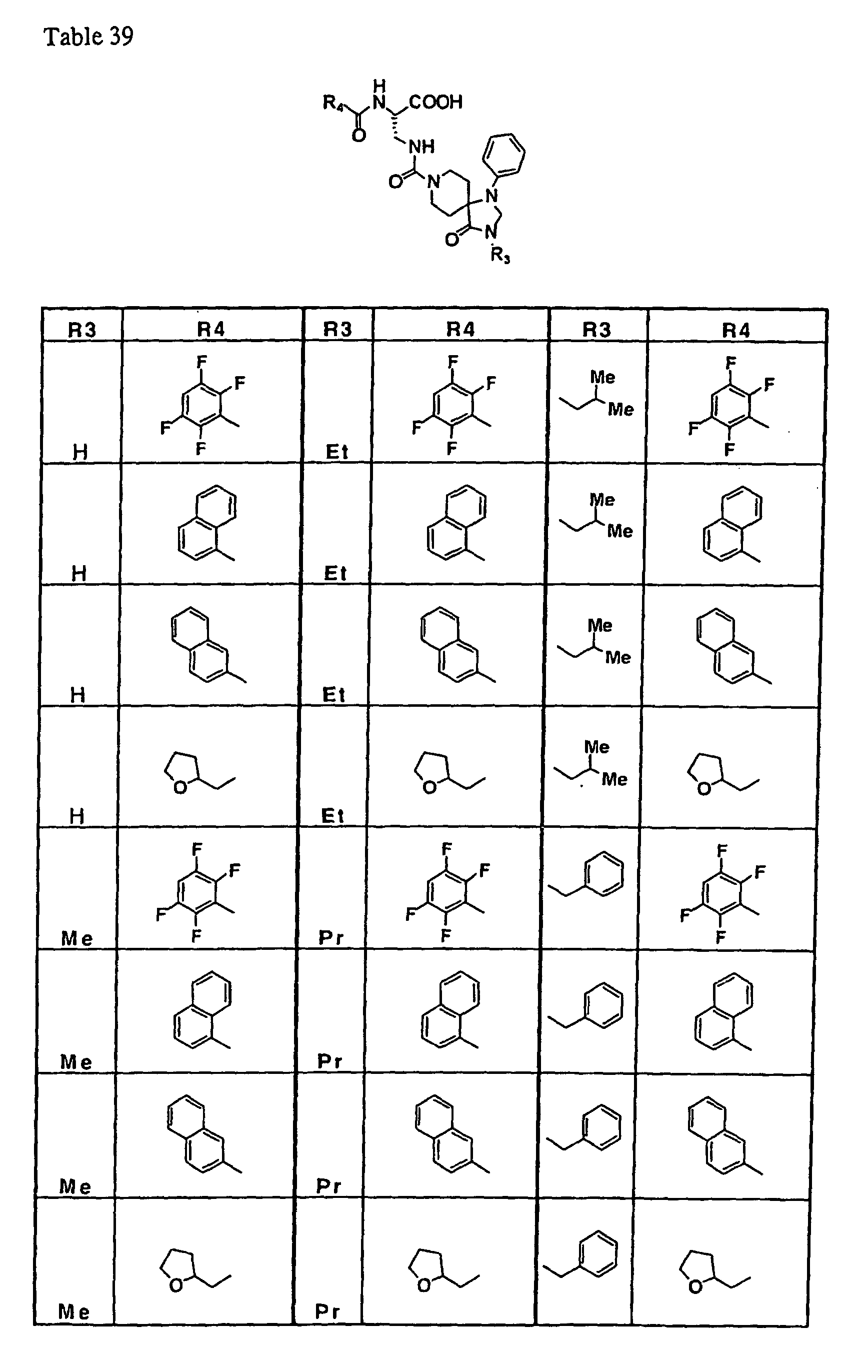

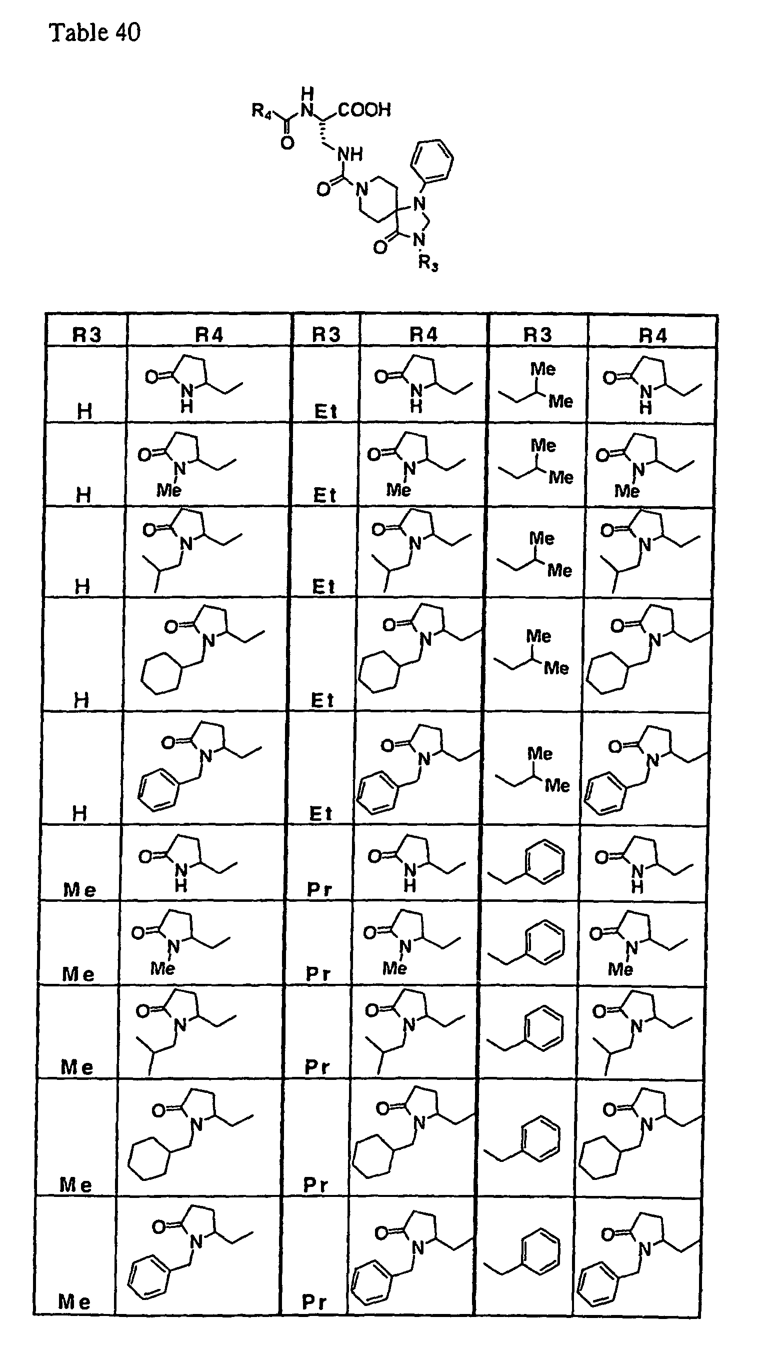

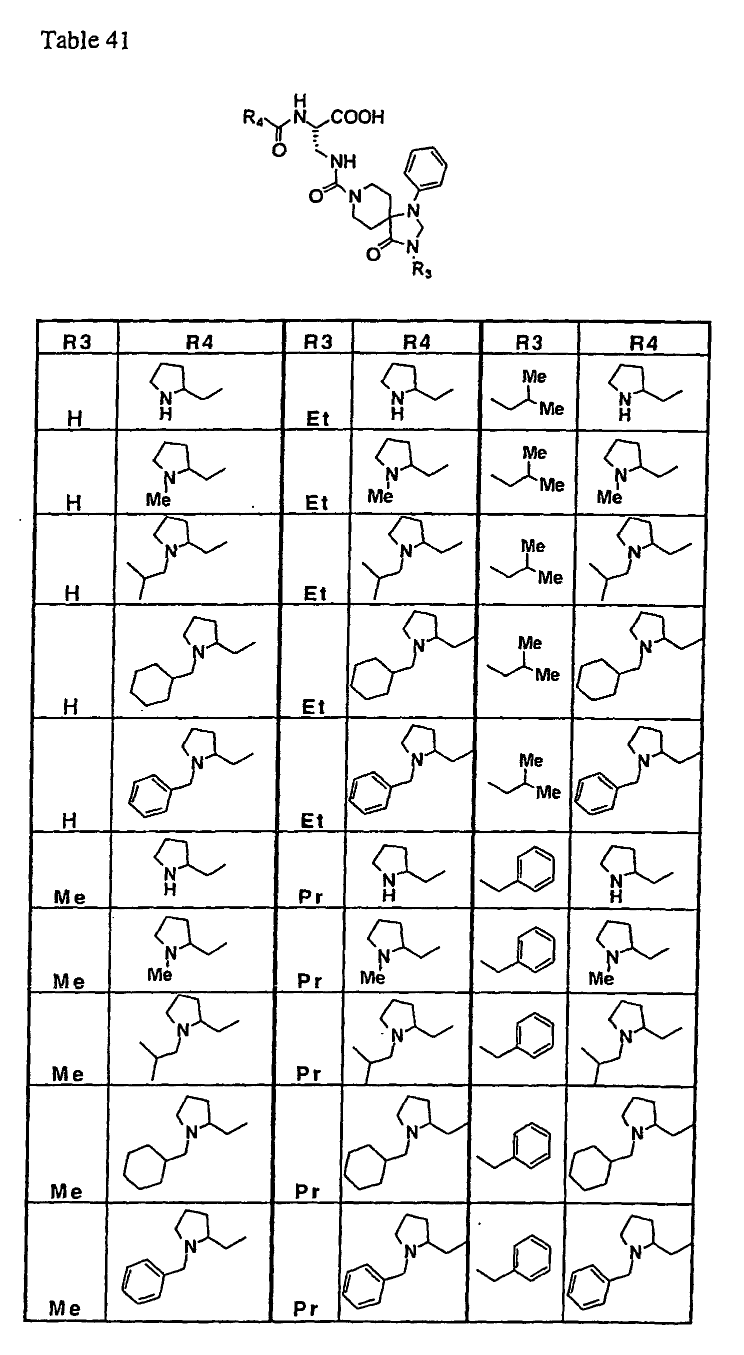

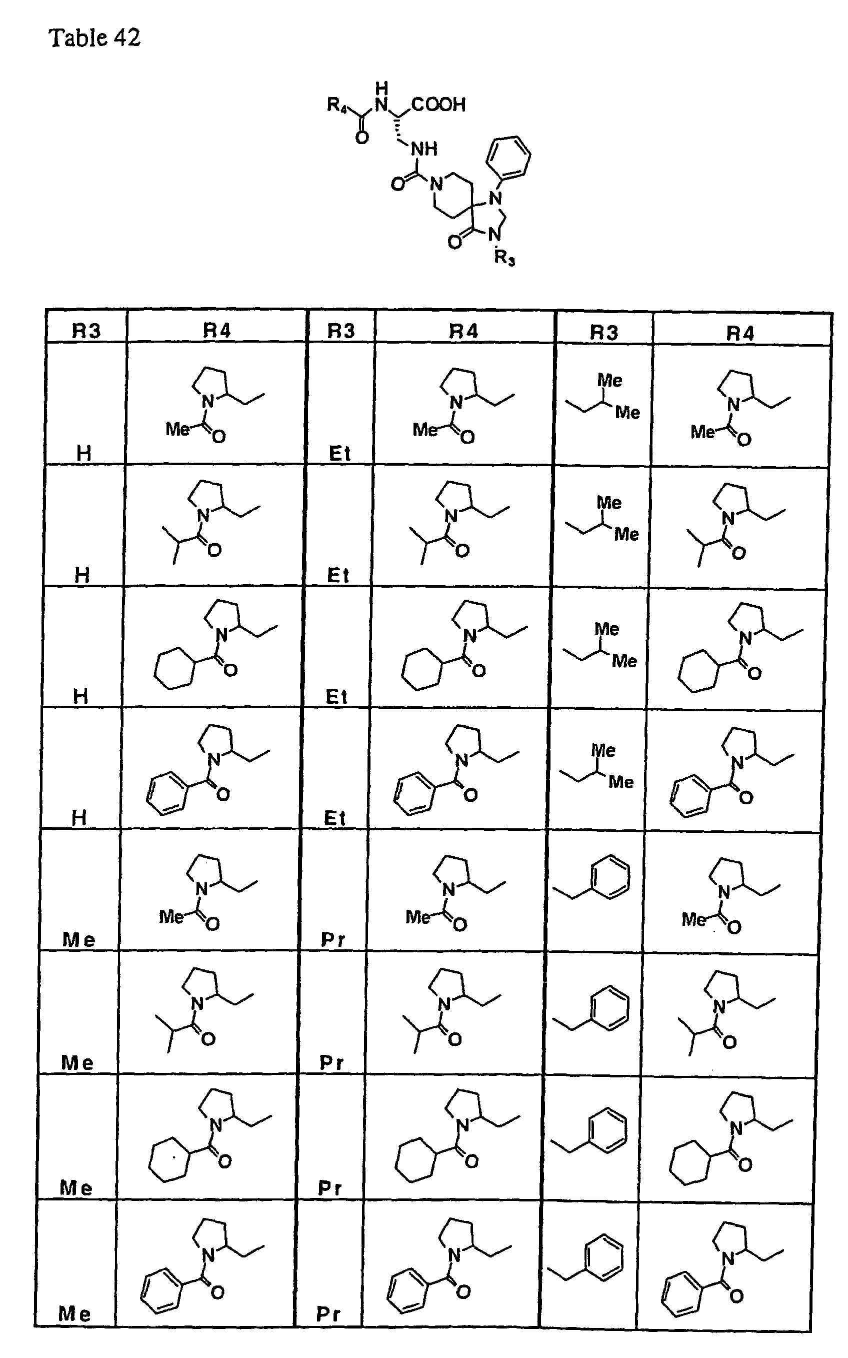

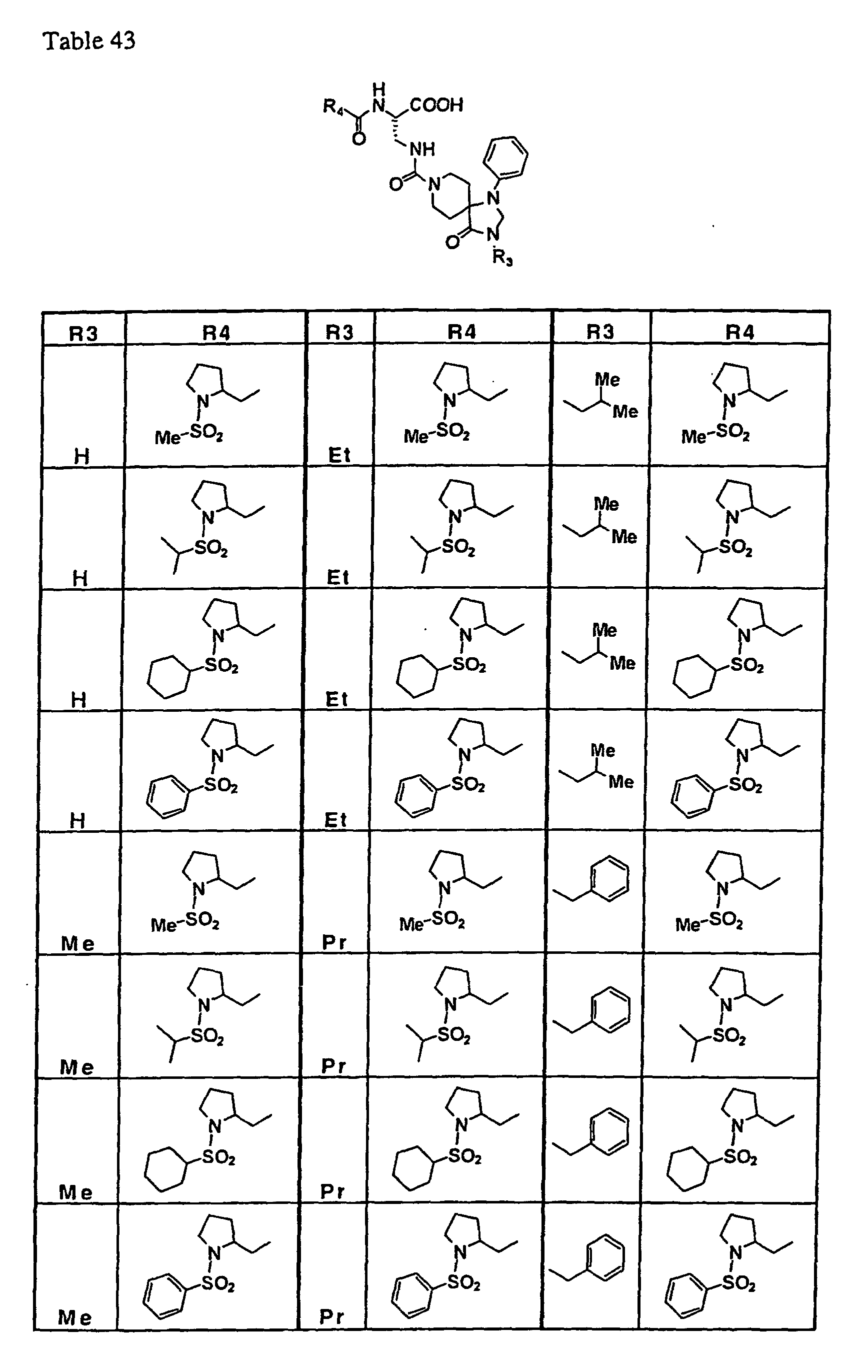

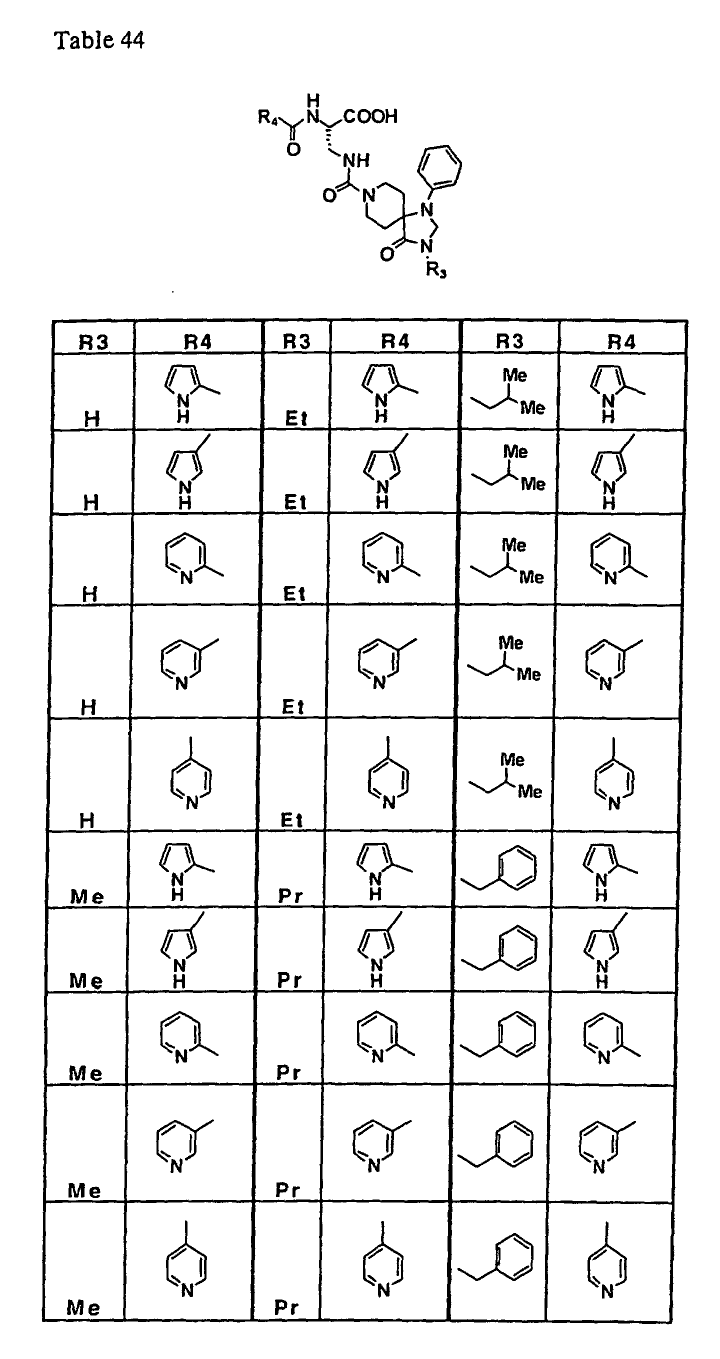

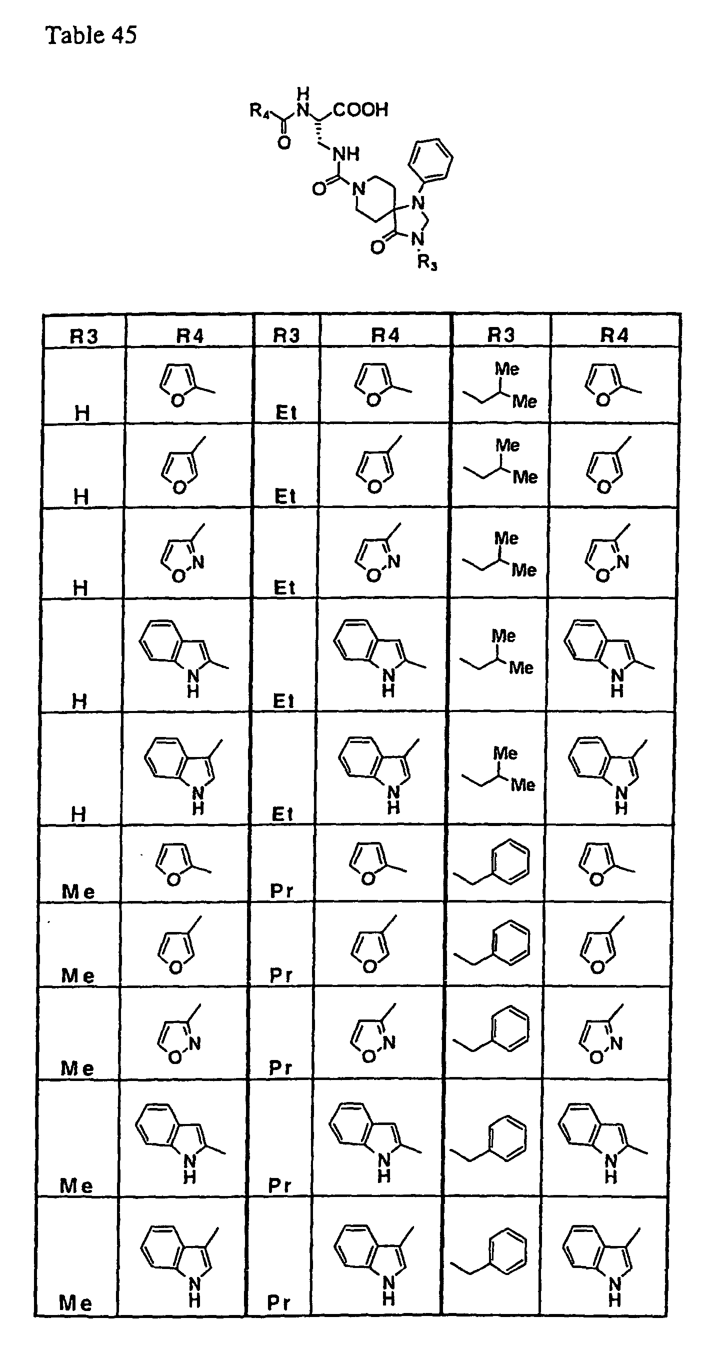

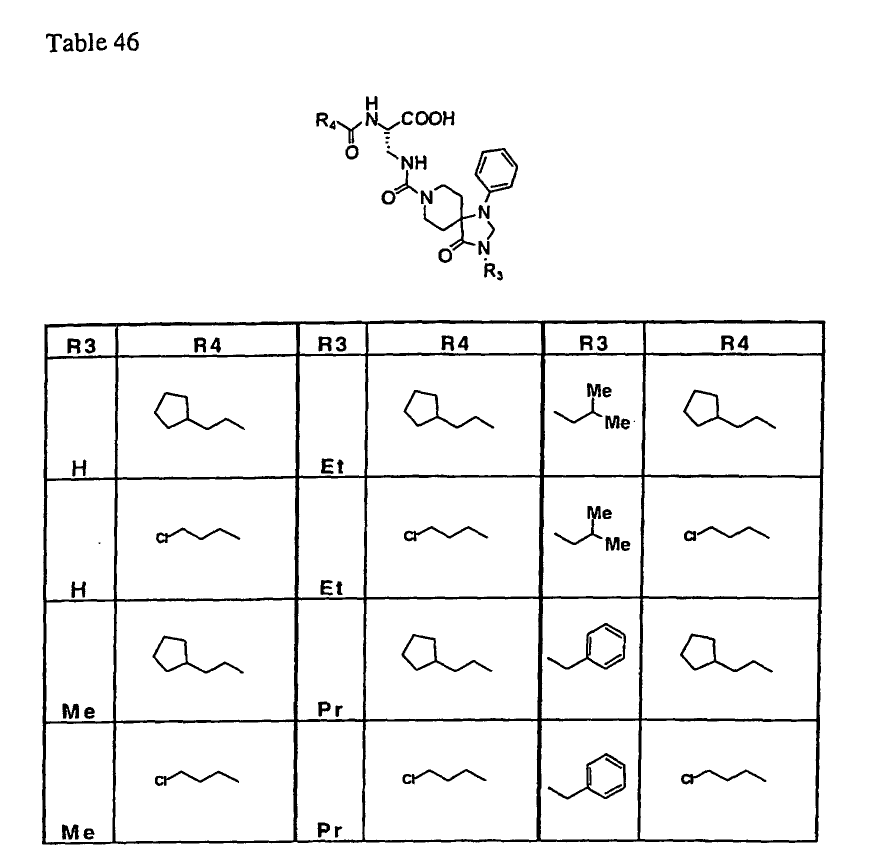

The processes for producing the compounds represented by Formula I (hereinafter, for example, "the compounds represented by Formula I" may also be indicated simply as "Formula I") will now be described. However, the process for producing each of the compounds is not restricted to that described herein. In the various production processes, the reaction conditions may be appropriately selected from those described below.

Among the compounds represented by Formula I, Formula VII, that is, Formula I, wherein l=0, m=1, n=1, A is -C(O)-, B is -NH-, C' and D form together =O and R₁ and R₂ are hydrogen atoms 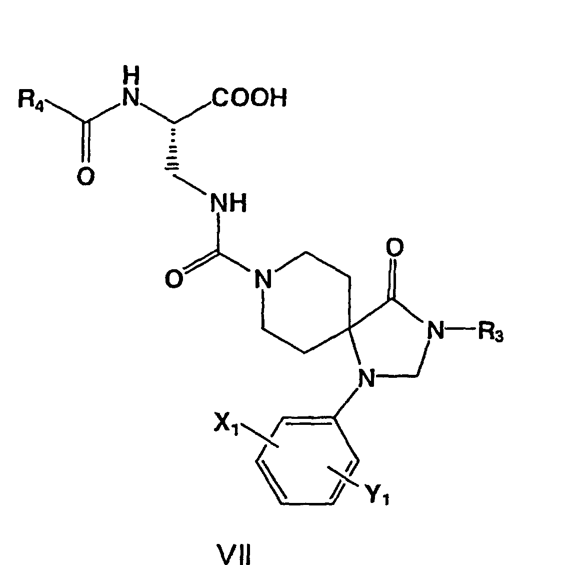 (wherein X₁, Y₁, R₃ and R₄ have the same definition as that described above) can be produced by hydrolysis of Formula VIII 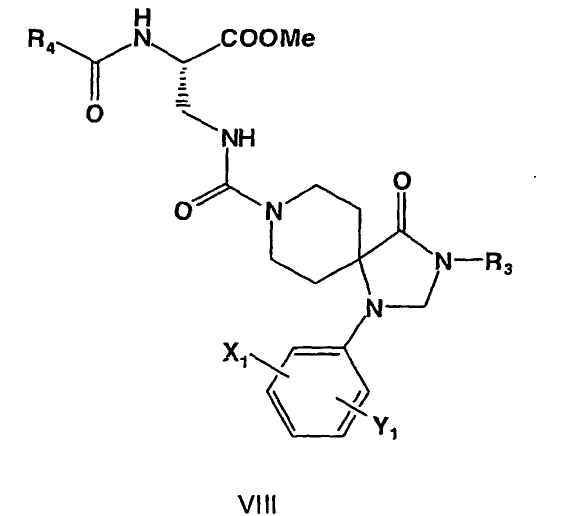 (wherein X₁, Y₁, R₃ and R₄ have the same definition as that described above) using a base such as aqueous sodium hydroxide solution, aqueous lithium hydroxide solution or aqueous barium hydroxide solution in a solvent such as an alcoholic solvent, such as methanol, or tetrahydrofuran, dimethoxyethane or 1,4-dioxane. While the hydrolysis using a base, such as aqueous sodium hydroxide solution, aqueous lithium hydroxide solution or aqueous barium hydroxide solution, is not particularly restricted, it is usually carried out by reacting at a temperature of from 0°C to about room temperature for approximately 1 to 48 hours. The added amount of the base is usually about 1 to 4 equivalents with respect to Formula VIII.

Formula VIII can be produced from Formula IX 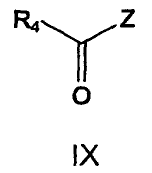 (wherein R₄ has the same definition as that described above, Z represents chloro, bromo or hydroxyl)
and Formula X 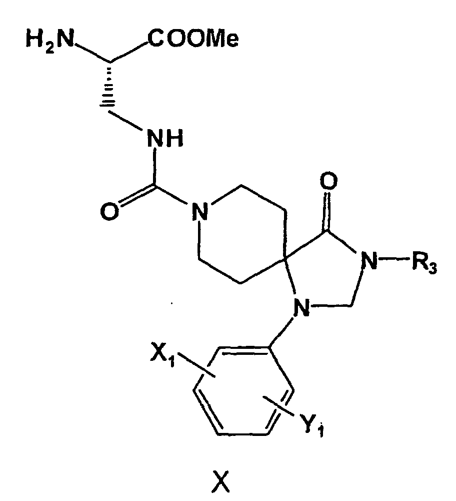 (wherein X₁, Y₁ and R₃ have the same definition as that described above).

In Formula IX, when Z is chloro or bromo, Formula VIII can be produced by reacting Formula IX and Formula X in a solvent such as tetrahydrofuran, dimethylformamide, chloroform, dichloromethane or 1,4-dioxane in the presence of a tertiary amine, such as triethylamine or diisopropylethylamine, pyridine or 4-(N,N-dimethylamino)pyridine. While the reaction of Formula IX and Formula X is not particularly restricted, it can usually be carried out by reacting at a temperature of from 0°C to about room temperature for approximately 1 to 24 hours. While the mixing ratio (molar ratio, unless otherwise specified, the term "mixing ratio" means molar ratio hereinafter) of Formula IX and Formula X is not restricted, it may usually be about 1:1 to 2:1, and the amount of the tertiary amine to be added is, although not restricted, usually about 1 to 4 equivalents with respect to Formula IX.

In Formula IX, when Z is hydroxyl, usually, a condensing agent such as dicyclohexylcarbodiimide (DCC), benzotriazol-1-yloxytris(dicyclopentylamino)phosphonium hexafluorophosphate salt (PyBOP), benzotriazol-1-yloxytris(dimethylamino)phosphonium hexafluorophosphate salt (BOP), diphenylphosphoryl azide (DPPA) or 1-ethyl-3-[3-(dimethylamino))propyl]carbodiimide (WSC) is used in a solvent such as tetrahydrofuran, dimethylformamide, chloroform or dichloromethane in the presence of a tertiary amine such as triethylamine, diisopropylethylamine or N-methylmorpholine. The amount of such a condensing agent to be added is not restricted, and is usually about 1 to 3 equivalents with respect to Formula IX. Addition of an additive such as 1-hydroxybenzotriazole (HOBT) may be advantageous in the proceeding of the reaction in some cases.

Formula VIII can also be produced by treating Formula XI, 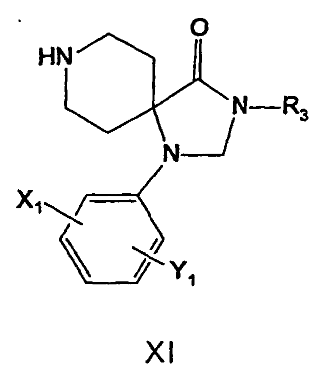 (wherein X₁, Y₁ and R₃ have the same definition as that described above)
with usually 0.5 to 2 equivalents of diphosgene, triphosgene, 1,1-carbonyldiimidazole or the like in a solvent such as dichloromethane or chloroform, in the presence of a tertiary amine such as triethylamine or diisopropylethylamine, then reacting the obtained product with Formula XII, 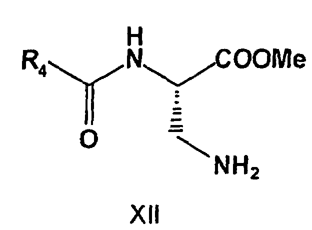 (wherein R₄ has the same definition as that described above).
The mixing ratio of Formula XI to Formula XII may usually be, although not restricted, about 1:1 to 2:1, and the reaction may usually be carried out at about 0°C to room temperature for about 1 to 24 hours. The amount of the tertiary amine to be added is not restricted, and is usually about 1 to 4 equivalents with respect to diphosgene, triphosgene or 1,1-carbonyldiimidazole.

Alternatively, Formula VIII may be achieved by reacting Formula XII with Formula XIII, 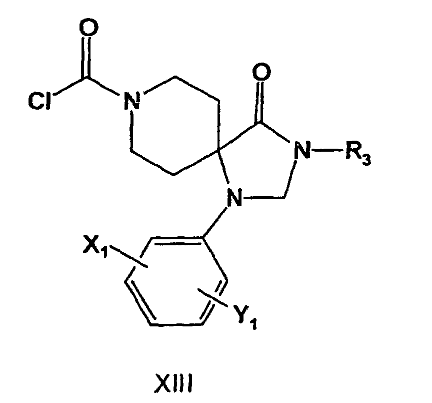 (wherein X₁, Y₁ and R₃ have the same definition as that described above)
in a solvent such as dimethylformamide, tetrahydrofuran or dimethoxyethane at about 0°C to room temperature for about 1 to 24 hours. The mixing ratio of Formula XII to Formula XIII is not restricted, and is usually about 1:1 to 1: 3.

Another method is to react Formula XII with p-nitrophenyl chloroformate or phenyl chloroformate in a solvent such as acetonitrile, dichloromethane or dimethoxyethane, in the presence of a base such as sodium hydrogen carbonate or tribenzylamine (first step); and then react the obtained product with Formula XI in a solvent such as acetonitrile, dichloromethane or dimethoxyethane, in the presence of a tertiary amine such as triethylamine or diisopropylethylamine (second step). In this method, the amount of the base such as sodium hydrogen carbonate or tribenzylamine used in the reaction is not restricted, and is usually about 1 to 4 equivalents with respect to p-nitrophenyl chloroformate or phenyl chloroformate. The amount of the tertiary amine to be added is not restricted, and is usually about 1 to 6 equivalents with respect to Formula XI. In the first step, the temperature of the reaction between Formula XII and p-nitrophenyl chloroformate or phenyl chloroformate, while not restricted, is usually about 0°C to room temperature. The reaction temperature in the second step may usually be about 0°C to 50°C when p-nitrophenyl chloroformate is used, and may usually be about room temperature to refluxing temperature when phenyl chloroformate is used.

Formula X may be produced by the following steps (in the present specification the Japanese word for "step" is rendered using the English term "step" in the chemical reactions): 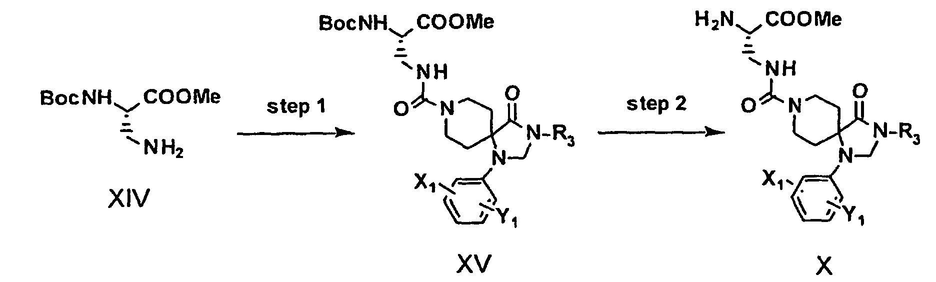 (wherein X₁, Y₁ and R₃ have the same definition as that described above)

Step 1 may be carried out in the same manner as in the reaction between Formula XI and Formula XII.

Step 2 is a step for removing t-butoxycarbonyl group (referred to as "Boc" for short) on the nitrogen atom. Usually, this step may be carried out by using trifluoroacetic acid, hydrochloric acid, hydrobromic acid and the like in a halogen-containing solvent such as chloroform or dichloromethane as the reaction solvent. Alternatively, this step may be carried out by using trifluoroacetic acid alone. The reaction temperature is not restricted, and usually a temperature between 0°C and room temperature is selected. The reaction time may be appropriately selected depending on the reaction temperature and the like, and usually, is about 1 to 24 hours.

Formula XII may be produced by the following steps using commercially available asparagine XVI as a starting material. 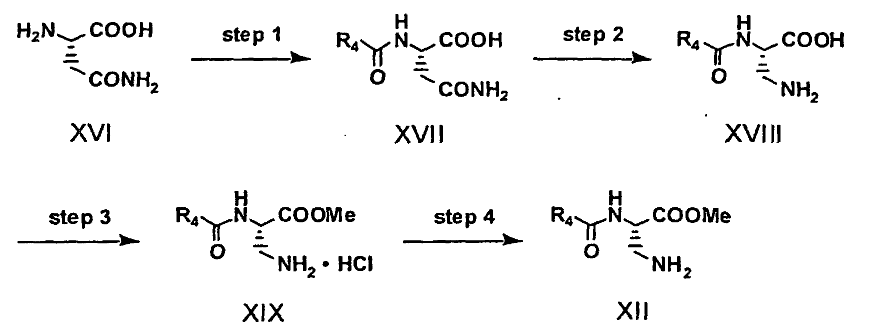 (wherein R₄ has the same definition as that described above).

Step 1 may be carried out in the same manner as in the reaction between Formula IX and X using Formula XVI and IX. When Z in Formula IX is chloro or bromo, aqueous sodium hydroxide solution, aqueous potassium hydroxide solution or the like may be used as the base.

Step 2 may be carried out by reacting Formula XVII and bromine in a basic solvent such as aqueous sodium hydroxide solution, aqueous potassium hydroxide solution or the like, for about 1 to 8 hours, although this is not restricted. The reaction temperature is not restricted, and is usually about room temperature to 100°C. Bromine is usually used in excess to Formula XVII. This step may also be carried out by the method described in J. Org. Chem., 62, 6918 (1997) or J. Org. Chem., 49, 4272 (1984).

Step 3 may be carried out by using thionyl chloride in a solvent such as methanol at about 0°C to room temperature. The reaction time is not restricted, and is usually about 1 to 8 hours. The mixing ratio of Formula XVIII to thionyl chloride is not restricted, and is usually about 1:1 to 1:10. The reaction may also be carried out by treating Formula XVIII with, although this is not restricted, an excess amount of diazomethane or trimethylsilyldiazomethane in a solvent such as methanol at about 0°C to room temperature.

Step 4 may be carried out by using a base such as aqueous sodium hydroxide solution, aqueous potassium hydroxide solution, aqueous potassium carbonate solution or triethylamine, in an excess amount with respect to Formula XIX, in a solvent such as chloroform or dichloromethane at about 0°C to room temperature.

Formula XIV may be produced by the following steps using commercially available Formula XX.

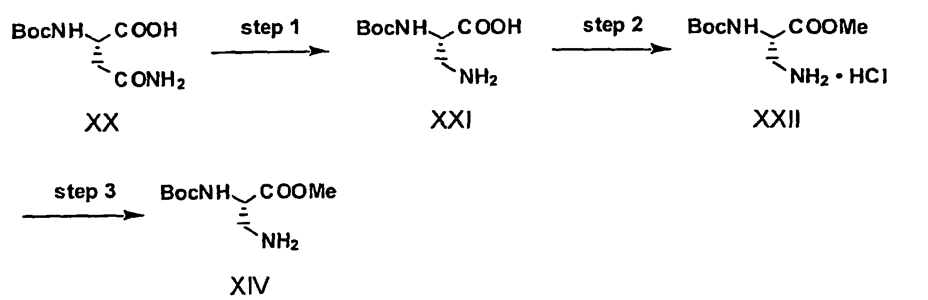

Step 1 may be carried out in the same manner as in step 2 in the process of producing Formula XII. Step 2 may be carried out in the same manner as in step 3 in the process of producing Formula XII. Step 3 may be carried out in the same manner as in step 4 in the process of producing Formula XII.

Among the compounds represented by Formula I, Formula XXIII, that is, Formula I, wherein l=0, m=1, n=1, A is -S(O)₂-, B is -NH-, C' and D cooperatively form =O and R₁ and R₂ are hydrogen atoms 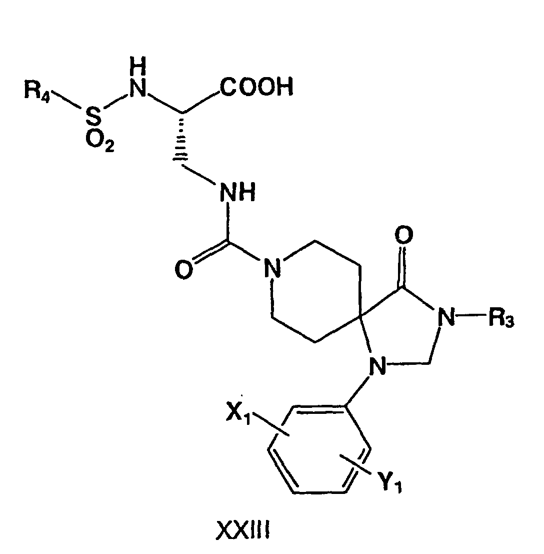 (wherein X₁, Y₁, R₃ and R₄ have the same definition as that described above)
can be produced by hydrolysis of Formula XXIV, 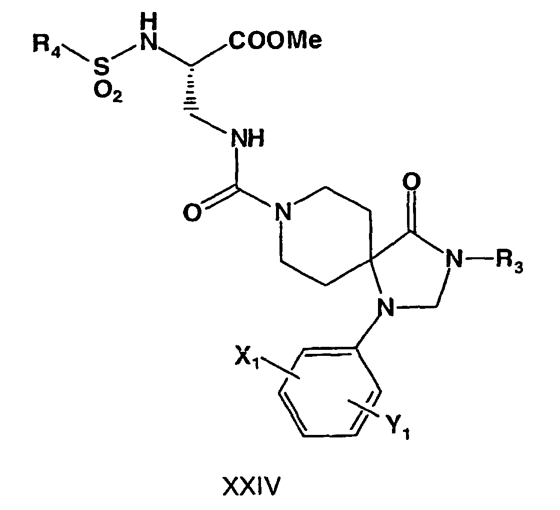 (wherein X₁, Y₁, R₃ and R₄ have the same definition as that described above)
using a base such as aqueous sodium hydroxide solution, aqueous lithium hydroxide solution or aqueous barium hydroxide solution in an solvent such as an alcoholic solvent, for example, methanol, or tetrahydrofuran, dimethoxyethane or 1,4-dioxane. While the hydrolysis using a base such as aqueous sodium hydroxide solution, aqueous lithium hydroxide solution or aqueous barium hydroxide solution is not particularly restricted, usually, it is carried out by reacting at a temperature of from 0°C to about room temperature for approximately 1 to 24 hours. The added amount of the base is usually about 1 to 4 equivalents with respect to Formula XXIV.

Formula XXIV can be produced by reacting Formula XXV 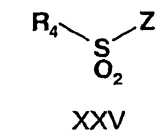 (wherein R₄ has the same definition as that described above, Z represents chloro or bromo)
and Formula X in a solvent such as tetrahydrofuran, dimethylformamide, chloroform, dichloromethane or 1,4-dioxane in the presence of a tertiary amine, such as triethylamine or diisopropylethylamine, pyridine or 4-(N,N-dimethylamino)pyridine. While the reaction of Formula X and Formula XXV is not particularly restricted, usually, it can be carried out by reacting at a temperature of from 0°C to about room temperature for approximately 1 to 24 hours. While the mixing ratio of Formula X and Formula XXV is not restricted, it may be about 1:1 to 1:2, and the amount of the tertiary amine to be added is, although not restricted, usually about 1 to 4 equivalents with respect to Formula XXV.

The compounds represented by Formula I can also be produced by solid-phase synthesis. The compounds can also be produced using the split and pool process employing solid-phase synthesis. In the split and pool process, IRORTs MicroKan system or Mimotopes' Lantern system can be used.

Among the compounds represented by Formula I, Formula VII, that is, Formula I, wherein l=0, m=1, n=1, A is -C(O)-, B is -NH-, C' and D cooperatively form =O and R₁ and R₂ are hydrogen atoms, can also be produced by a splitting reaction from Formula XXVI 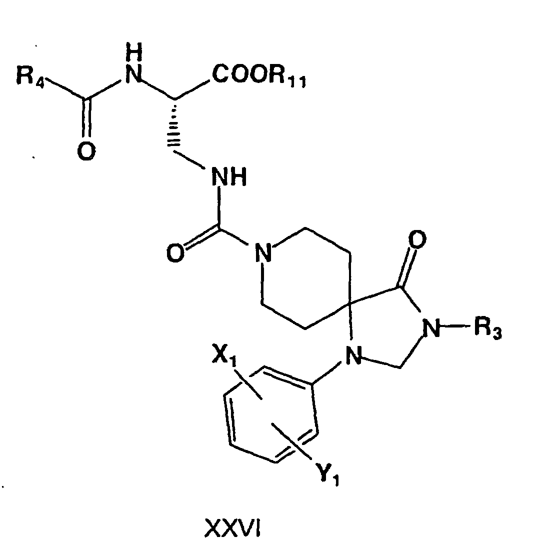 (wherein X₁, Y₁, R₃ and R₄ have the same definition as that described above and R₁₁ represents a resin used in ordinarily solid-phase synthesis, for example a Wang resin).

The splitting from Formula XXVI can be carried out by, for example, using an acid such as trifluoroacetic acid, acetic acid or hydrochloric acid in a solvent such as methylene chloride, tetrahydrofuran, water or methanol. Preferable acid conditions are a solution of 1 to 20% trifluoroacetic acid-methylene chloride. While the reaction temperature is not particularly restricted, usually, the temperature is from 0 to 100°C and preferably from 10 to 30°C. While the reaction time is not particularly restricted, usually, it is from 0.1 to 24 hours and preferably from 0.1 to 2 hours.

Formula XXVI can be produced by reacting Formula XI and Formula XXVII 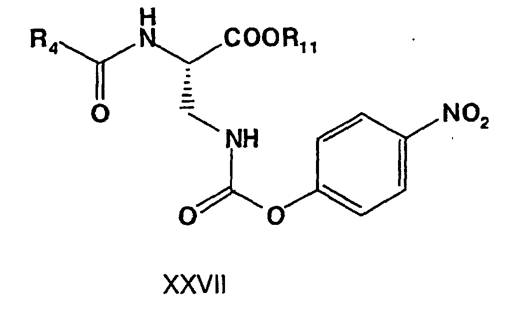 (wherein R₄ and R₁₁ have the same definition as that described above) in a solvent such as dimethylformamide, methylene chloride or tetrahydrofuran in the presence of a tertiary amine, such as triethylamine or diisopropylethylamine. While the mixing ratio of Formula XI and Formula XXVII is not particularly restricted, it is usually about 1:1 to 50:1, preferably 2:1 to 20:1. The amount of the tertiary amine to be added is, although not restricted, usually about I to 50 equivalents with respect to Formula XXVII, preferably 1 to 20 equivalents. The reaction temperature is not restricted, and is preferably between 0 and 50°C. The reaction time may be appropriately selected depending on the reaction temperature and the like, and usually, is about 0.1 to 2 hours.

Formula XXVII can be produced by reacting Formula XXVIII, 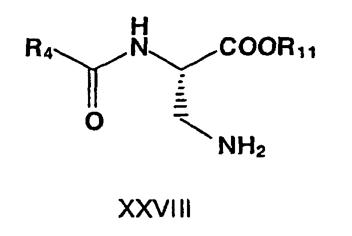 (wherein R₄ and R₁₁ have the same definition as that described above)
and p-nitrophenyl chloroformate in a solvent such as methylene chloride, tetrahydrofuran or a mixture of methylene chloride-tetrahydrofuran in the presence of a tertiary amine such as diisopropylethylamine or N-methylmorpholine. The amount of the p-nitrophenyl chloroformate to be used is, although not restricted, usually about 1 to 50 equivalents with respect to Formula XXVIII, preferably 1 to 20 equivalents. The amount of the tertiary amine to be added is, although not restricted, usually about 1 to 4 equivalents with respect to the p-nitrophenyl chloroformate. The reaction temperature is not restricted, and is preferably between 0 and 50°C. The reaction time may be appropriately selected depending on the reaction temperature and the like, and usually, is about 0.1 to 2 hours.

Formula XXVIII can be produced by reacting Formula XXIX 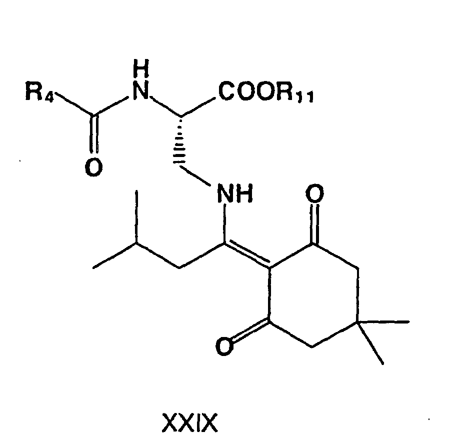 (wherein R₄ and R₁₁ have the same definition as that described above)
with 1 to 20% hydrazine hydrate in a solvent such as dimethylformamide or dimethylacetamide. The reaction temperature is not restricted, and is usually between 0 and 50°C. The reaction time may be appropriately selected depending on the reaction temperature and the like, and usually, is about 0.1 to 2 hours.

Formula XXIX can be produced from Formula IX and Formula XXX. 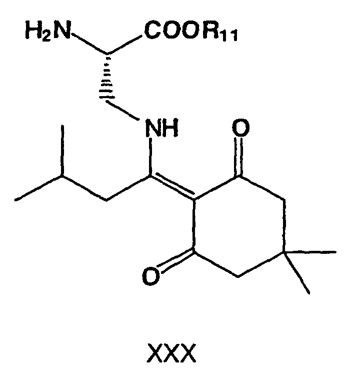 (wherein R₁₁ has the same definition as that described above)

In Formula IX, when Z is chloro or bromo, Formula XXIX can be produced by reacting Formula IX and Formula XXX in a solvent such as tetrahydrofuran, dimethylformamide or dichloromethane in the presence of an amine, such as triethylamine, diisopropylethylamine or pyridine. While the reaction of Formula IX and Formula X is not particularly restricted, and may be carried out at a temperature of, usually, between 0 and 50°C, for about 1 to 48 hours. While the mixing ratio of Formula IX and Formula XXX is not particularly restricted, it is usually about 1:1 to 50:1, preferably 1:1 to 20:1. The amount of the amine to be added is, although not restricted, usually about 1 to 4 equivalents with respect to Formula IX.

In Formula IX, when Z is hydroxyl, usually, a condensing agent such as dicyclohexylcarbodiimide (DCC), benzotriazol-1-yloxytris(dicyclopentylamino)phosphonium hexafluorophosphate (PyBOP), benzotriazol-1-yloxytris(dimethylamino)phosphonium hexafluorophosphate (BOP), diphenylphosphoryl azide (DPPA) or 1-ethyl-3-[3-(dimethylamino)propyl]carbodiimide (WSC) is used in a solvent such as tetrahydrofuran, dimethylformamide or dichloromethane in the presence of a tertiary amine such as triethylamine, diisopropylethylamine or N-methylmorpholine. The amount of such a condensing agent to be added is not restricted, and is usually about 1 to 3 equivalents with respect to Formula IX. Addition of an additive such as 1-hydroxybenzotriazole (HOBT) may be advantageous in the proceeding of the reaction in some cases. While the mixing ratio of Formula IX and Formula XXX is not particularly restricted, it is usually about 1:1 to 50:1, preferably 1:1 to 20:1. While the reaction of Formula IX and Formula XXX is not particularly restricted, it may be carried out at a temperature of, usually, between 0 and 50°C, for about 1 to 48 hours.

Formula XXX can be produced by reacting Formula XXXI 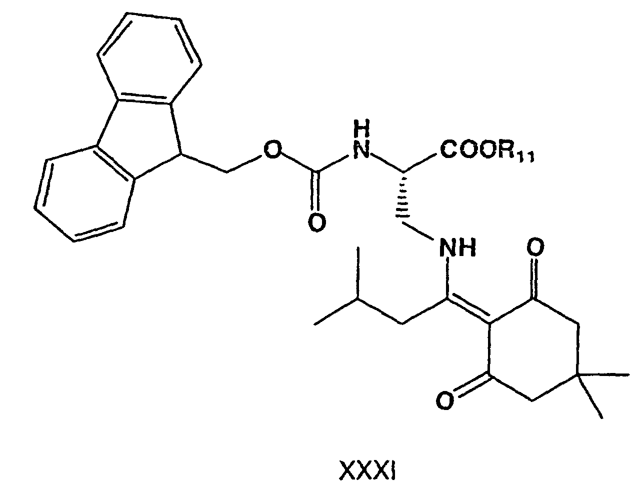 (wherein R₁₁ has the same definition as that described above) with 10 to 30% piperidine in a solvent such as dimethylformamide or methylene chloride. The reaction temperature is not particularly restricted, and is usually between about 0 and 50°C. The reaction time may be appropriately selected depending on the reaction temperature and the like, and usually, is about 0.1 to 10 hours.

Formula XXXI can be produced by employing a condensing agent to react a resin used in ordinary solid-phase synthesis, such as a Wang resin, with commercially available Formula XXXII 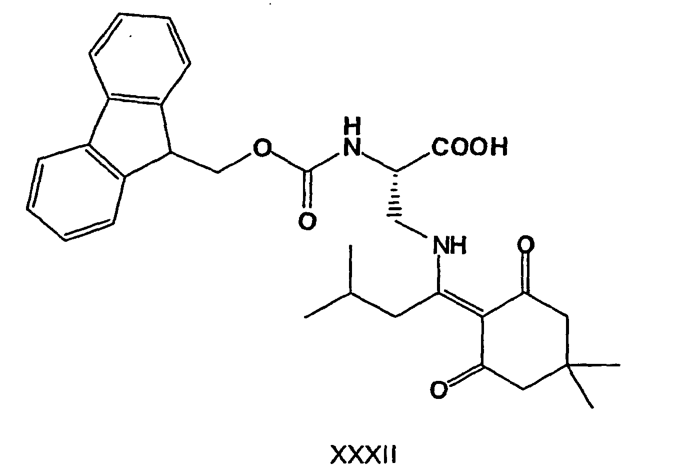 in a solvent such as dimethylformamide or tetrahydrofuran. A combination of diisopropylcarbodiimide and dimethylaminopyridine or combination of diethyl azodicarboxylate and triphenylphosphine can be used as the condensing agent. A Wang resin is preferably used as the resin in the solid-phase synthesis. While the reaction temperature is not particularly restricted, it is preferably between 0 and 50°C. The reaction time may be appropriately selected depending on the reaction temperature and the like, and is preferably from I to 48 hours.

When the novel spiro derivatives used in the present invention have one or more asymmetric carbon atoms, there exist racemic modifications, diasteromers and optical isomers. In the present invention, any of these may be used.

The reaction products obtained by the above-described processes may be isolated and purified in the form of a free compound, a salt or a solvate such as hydrate thereof. The salt may be produced by a usual salt-producing treatment. Isolation and purification may be carried out by ordinary chemical processes such as extraction, concentration, evaporation, crystallization, filtration, recrystallization and various types of chromatography.

Various isomers may be isolated by conventional methods utilizing the differences in the physicochemical properties between the isomers. Optical isomers may be separated by a general optical resolution method such as fractional crystallization or chromatography. Optically active substance may also be produced by an appropriate optically active compound as the starting material.

Examples of the pharmaceutically acceptable salts of the compounds represented by Formula I include inorganic salts such as ammonium salt, alkaline metal salts (e.g., sodium salt and potassium salt), alkaline earth metal salts (e.g., calcium salt and magnesium salt); organic salts such as dicyclohexylamine salt, N-methyl-D-glucamine salt, ethanolamine salt, diethanolamine salt, triethanolamine salt, diisopropanolamine salt and tris(hydroxymethyl)aminomethane salt; and lysine salt and arginine salt.

Further, various hydrates, solvates and crystalline polymorphs of the compounds (1) of the present invention and the salts thereof are included within the scope of the present invention.

The fact that the compounds according to the present invention have adhesion molecule inhibitory action can be confirmed by, for example, measuring adhesion inhibitory activity against VLA-4, which is one member of the integrin family.

The inhibitory activity of the compound according to the present invention against the adhesion of VLA-4 may be determined by using an adhesion-measuring system in which the adhesion between VLA-4-expressing cells such as Ramos cells or Jurkat cells and fibronectin or fibronectin fragment such as a peptide containing CS-1 sequence (Gly Pro Glu IIe Leu Asp Val Pro Ser Thr) (hereinafter referred to as "CS-1 peptide") immobilized on an immunoplate is measured. Alternatively, a binding-measuring system in which the adhesion between VLA-4 protein and fibronectin or fibronectin fragment such as CS-1 peptide immobilized on an immunoplate is measured may be used. In the present invention, it is preferred to evaluate the inhibitory activity of a compound using a binding-measuring system of soluble VLA-4 with CS-1 peptide (WO 98/32771), but the method is not restricted thereto. When testing the inhibitory effect of a compound, it is preferred to mix the soluble VLA-4 and the test compound beforehand.

Since the compounds according to the present invention have inhibitory activities against adhesion molecules, especially adhesion of VLA-4, and so inhibit accumulation of leukocytes at the inflammatory site, they may be used as therapeutic drugs for inflammatory diseases, in particular, chronic inflammatory diseases. Examples of such diseases include allergic diseases such as asthma, dermatitis and rhinitis, autoimmune diseases such as arthritis, multiple sclerosis, Crohn's disease and ulcerative colitis, hepatitis, nephritis, graft rejections after organ transplantation and type I diabetes. More preferred is use against allergic inflammatory diseases such as bronchial asthma, atopic dermatitis and allergic rhinitis, autoimmune diseases such as rheumatoid arthritis, multiple sclerosis, Crohn's disease and ulcerative colitis, hepatitis, nephritis, graft rejections after organ transplantation and type I diabetes. In addition to these, the compounds may be used as a therapeutic drug for the prevention of postoperative restenosis, arteriosclerosis and the like.

In the present invention, the inflammatory disease suppressing effect of the compounds obtained in accordance with the above-described method will be illustrated using a mouse inflammatory model, although such effect is not limited thereto.

Various models have been reported as allergic inflammatory models. For example, in the peritonitis model sensitized with antigens of ascaris or ragweed pollen, or the ear edema model that has been sensitized or induced using dinitrofluorobenzene or oxazolone as hapten antigens, the effects of the subject compound are examined by measuring the suppressing effects on the number of leukocytes accumulating at an inflammatory site or swelling. Although in the ear edema model the use of dinitrofluorobenzene as an antigen is more preferred, it is not limited thereto.

When using the compound of the present invention as a therapeutic drug against the above-mentioned diseases, the compound represented by Formula I or a base addition salt thereof may be administered as it is in the form of powder, or may be administered as a pharmaceutical composition in the form of an appropriate formulation, orally or parenterally (e.g., percutaneous administration, intravenous administration, rectal administration, inhalation, nasal drip or eye drip) to mammals.

Examples of the formulation for administration include tablets, powders, pills, capsules, granules, syrups, liquid preparations, injections, emulsions, suspensions and suppositories. These formulations may be prepared by the methods which per se are known, and contain various carriers usually used in the field of formulation. Examples thereof include vehicles, lubricants, binders and disintegrators for solid formulations; and solvents, solubilizers, suspending agents and soothing agents for liquid formulations. Additives such as antiseptics, antioxidants, coloring agents, sweeteners, absorbents, and wetting agents may be used as required.

Examples of the vehicles include lactose, sucrose, D-mannitol, starch, cornstarch, crystalline cellulose and light anhydrous silicic acid. Examples of the lubricants include magnesium stearate, calcium stearate, talc and colloidal silica. Examples of the binders include crystalline cellulose, saccharose, D-mannitol, dextrin, hydroxypropylcellulose, hydroxypropylmethylcellulose, polyvinylpyrrolidone, starch, sucrose, gelatin, methylcellulose and carboxymethylcellulose sodium. Examples of the disintegrators include starch, carboxymethylcellulose, carboxymethylcellulose calcium, cross carmelose sodium, sodium carboxymethyl starch and L-hydroxypropylcellulose. Examples of the solvents include water for injection, alcohol, propylene glycol, Macrogol, sesame oil and corn oil. Examples of the solubilizers include polyethylene glycol, propylene glycol, D-mannitol, benzyl benzoate, ethanol, cholesterol, triethanolamine, sodium carbonate and sodium citrate. Examples of the suspending agents include surfactants such as stearyl triethanolamine, sodium lauryl sulfate, lauryl aminopropionate, lecithin, benzalkonium chloride, benzethonium chloride and glycerin monostearate, and hydrophilic macromolecules such as polyvinyl alcohol, polyvinyl pyrrolidone, methylcellulose, hydroxymethylcellulose, hydroxyethylcellulose and hydroxypropylcellulose. Examples of the isotonicities include glucose, sodium chloride, D-sorbitol and D-mannitol. Examples of the buffering agents include buffering solutions containing phosphate, acetate, carbonate or citrate. An example of the soothing agents is benzyl alcohol. Examples of the antiseptics include p-hydroxybenzoates, chlorobutanol, benzyl alcohol, phenethyl alcohol, dehydroacetic acid and sorbic acid. Examples of the antioxidants include sulfites and ascorbic acid.

The effective dose and the number of times of administration of the compounds represented by Formula I and pharmaceutically acceptable salts thereof differ depending on the administration form, age and bodyweight of the patient, the type and severity of the disease to be treated, and usually, 0.1 to 1000 mg, preferably 1 to 300 mg of the compound may be administered once or in several times per day per adult.

The above-mentioned formulations may contain other active ingredients for therapy so long as the combination with the compounds, or salts thereof represented by Formula I does not cause an undesirable interaction. Examples thereof include steroid drugs, nonsteroidal anti-inflammatory drug, lipoxygenase inhibitors, leucotriene inhibitors, bronchodilators, thromboxane synthesis inhibitors, thromboxane antagonists, histamine antagonists, histamine release inhibitors, platelet activating factor (PAF) antagonists, serotonin antagonists, adenosine receptor antagonists, adrenalin β receptor stimulators, immunosuppressors and immunomodulators.

This specification includes part or all of the contents as disclosed in the specification of Japanese Patent Application No. 2002-80697, which is a priority document of the present application.

### Best Mode for Carrying Out the Invention

The effect of the present invention will now be specifically described by way of examples thereof. It should be noted that the present invention is not restricted to the examples.

### Example 1

### Methyl 2-((t-butoxy)carbonylamino)-3-((2,4,8-triaza-2-methyl-1-oxo-4-phenylspiro [4.5]dec-8-yl)carbonylamino)propanoate (1)

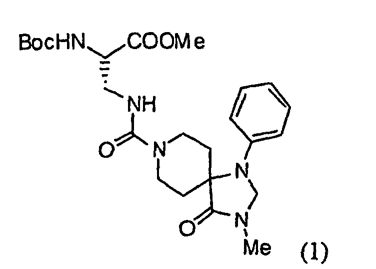

Under argon atmosphere, 12.9 g of methyl 3-amino-2-((t-butoxy)carbonylamino)propanoate was dissolved in 700 ml of dichloromethane, and then 7.9 g of saturated sodium hydrogen carbonate and 14.3 g of p-nitrophenyl chloroformate were added thereto at 0°C, followed by stirring the resulting mixture at room temperature for 5.5 hours. To the reaction mixture, 21.7 g of 2,4,8-triaza-2-methyl-4-phenylspiro[4.5]decane-1-one and 41.1 ml of triethylamine were added, and the resulting mixture was stirred at room temperature for 13 hours. After concentrating the reaction mixture, saturated aqueous sodium hydrogen carbonate solution was added, and the resulting mixture was extracted with chloroform. The organic layers were combined, washed with 0.1 N hydrochloric acid and with saturated saline, dried over anhydrous sodium sulfate and concentrated. The residue was purified by column chromatography (dichloromethane/methanol=60:1) to obtain 21.6 g of methyl 2-((t-butoxy)carbonylamino)-3-((2,4,8-triaza-2-methyl-1-oxo-4-phenylspiro[4.5]dec-8-yl)carbonylamino)propanoate (yield: 75%).
LR-MS(m/z):490(M+H)⁺
¹H-NMR (300MHz, CDCl₃, δppm):1.43 (9H, s), 1.63-1.72 (2H, m), 2.49-2.60 (2H, m), 3.01 (3H, s), 3.49-3.90 (6H, m), 3.75 (3H, s), 4.38 (1H, s), 4.68 (2H, s), 5.29 (1H, m), 5.84 (1H, m), 6.86 (1H, m), 7.26-7.31 (2H, m).

### Example 2

### Methyl 2-((t-butoxy)carbonylamino)-3-((2,4,8-triaza-1-oxo-4-phenylspiro[4.5]dec-8-yl)carbonylamino)propanoate (2)

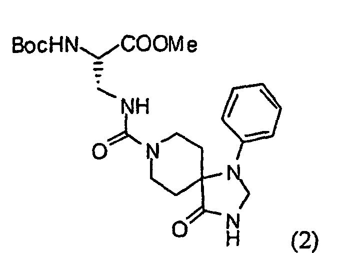

Under argon atmosphere, 564 mg of 1,1-carbonyldiimidazole was dissolved in 8 ml of tetrahydrofuran, and into this solution 10 ml of tetrahydrofuran containing 760 mg of methyl 3-amino-2-((t-butoxy)carbonylamino)propanoate was dropped at 0°C over 25 minutes. The resulting solution was then stirred for 0.5 hours. To the reaction mixture, 805 mg of 2,4,8-triaza-4-phenylspiro[4.5]decane-1-one was added, and the resulting mixture was stirred at room temperature for 13 hours. To the reaction mixture, 10% aqueous citric acid was added, and the resulting mixture was extracted with ethyl acetate. The organic layers were combined, washed with 0.1 N hydrochloric acid and with saturated saline, dried over anhydrous sodium sulfate and concentrated to obtain 1.55 g of methyl 2-((t-butoxy)carbonylamino)-3-((2,4,8-triaza-1-oxo-4-phenylspiro[4.5] dec-8-yl)carbonylamin o)propanoate (yield: 94%).
LR-MS(m/z):476(M+H)⁺
¹H-NMR (300MHz, CDCl₃, δppm):1.43 (9H, s), 1.64-1.76 (2H, m), 2.48-2.59 (2H, m), 3.51-3.87 (6H, m), 3.75 (3H, s), 4.38 (1H, m), 4.75 (2H, s), 5.30 (1H, m), 5.85 (1H, m), 6.74-6.77 (2H, m), 6.87 (1H, m), 7.26-7.31 (2H, m).

### Example 3

### Methyl 2-((phenylsulfonyl)amino)-3-((2,4,8-triaza-2-methyl-1-oxo-4-phenylspiro[4.5] dec-8-yl)carbonylamino)propanoate (3)

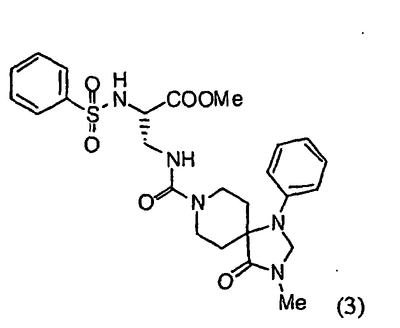

In 2 ml of dichloromethane, 362 mg of methyl 2-((t-butoxy)carbonylamino)-3-((2,4,8-triaza-2-methyl-1-oxo-4-phenylspiro[4.5]dec-8-yl)carbonylamino) propanoate was dissolved, and 1 ml of trifluoroacetic acid was added thereto, followed by stirring the resulting mixture at room temperature for 12 hours. The reaction mixture was concentrated then dissolved in chloroform, washed with aqueous potassium carbonate (0.5 M) and with saturated saline, dried over anhydrous sodium sulfate, and concentrated. The residue was dissolved in 4 ml of dichloromethane, and then 205 µl of triethylamine and 95 µl of benzenesulfonyl chloride were added thereto, followed by stirring the resulting mixture at room temperature overnight. Saturated aqueous sodium hydrogen carbonate solution was added to the reaction mixture, and the resulting mixture was extracted with chloroform. Organic layers were combined, washed with 10% aqueous citric acid solution and with saturated saline, dried over anhydrous sodium sulfate, and concentrated. The residue was purified by column chromatography (chloroform:methanol=60:1) to obtain 295 mg of methyl 2-((phenylsulfonyl)amino)-3-((2,4,8-triaza-2-methyl-1-oxo-4-phenylspiro[4.5]dec-8-yl)-carbonylamino)propanoate (yield: 75%).
LR-MS(m/z):530(M+H)⁺
¹H-NMR (300MHz, CDCl₃, δppm): 1.65-1.70 (2H, m), 2.50-2.61 (2H, m), 3.01 (3H, s), 3.48 (1H, m), 3.59 (3H, s), 3.63-3.88 (5H, m), 3.97 (1H, m), 4.68 (2H, s), 5.06 (1H, m), 6.09 (1H, m), 6.76 (2H, d, J=7.90), 6.84 (1H, t, J=7.32), 7.26 (2H, t, J=7.32), 7.47-7.61 (3H, m), 7.83-7.86 (2H, m).

### Example 4

### 2-((Phenylsulfonyl)anvno)-3-((2,4,8-triaza-2-methyl-1-oxo-4-phenylspiro[4.5]dec-8-yl) carbonylamino)propanoic acid (4)

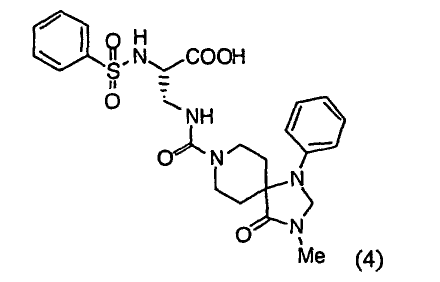

In 4 ml of tetrahydrofuran, 191 mg of methyl 2-«phenylsulfonyl)amino)-3-((2,4,8-triaza-2-methyl-1-oxo-4-phenylspiro[4.5]dec-8-yl)carbonylamino)propanoate was dissolved, and 4 ml of 0.1 N aqueous sodium hydroxide was added thereto, followed by stirring the resulting mixture at 0°C for 18 hours. Hydrochloric acid (0.1 N) was added to the reaction mixture, and the resulting mixture was extracted with ethyl acetate. Organic phases were combined, washed with saturated saline, dried over anhydrous sodium sulfate, and concentrated. The residue was reprecipitated from chloroform/diethyl ether to obtain 139 mg of 2-((phenylsulfonyl)amino)-3-((2,4,8-triaza-2-methyl-2-oxo-4-phenylspiro[4.5]dec-8-yl)-carbonylamino)propanoic acid (yield: 75 %).
LR-MS(m/z):516 (M+H)⁺
¹H-NMR (300MHz, CDCl₃, δppm): 1.67-1.72 (2H, m), 2.44-2.53 (2H, m), 3.01 (3H, s), 3.54-3.79 (7H, m), 4.68 (2H, s), 5.49 (1H, m), 6.41 (1H, m), 6.75 (2H, d, J=8.20), 6.87 (1H, t, J=7.03), 7.24-7.29 (2H, m), 7.45-7.58 (3H, m), 7.82 (2H, d, J=7.61).

### Example 5

### Methyl 3-((2,4,8-triaza-2-methyl-1-oxo-4-phenylspiro[4.5]dec-8-yl)carbonylamino)-2-(((2,4,6-trichlorophenyl)sulfonyl)amino)propanoate (5)

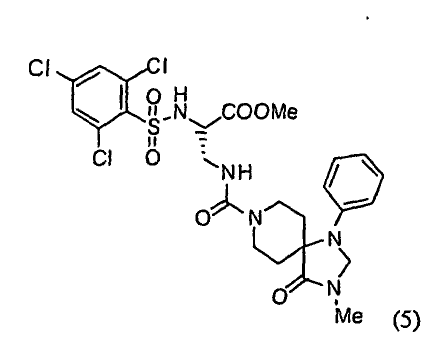

In 2.0 ml of dichloromethane, 220 mg of methyl 2-((t-butoxy)carbonylamino)-3-((2,4,8-triaza-2-methyl-1-oxo-4-phenylspiro[4.5]dec-8-yl)carbonylamino)propanoate was dissolved, and 1 ml of trifluoroacetic acid was added thereto, followed by stirring the resulting mixture at room temperature for 5 hours. After the reaction mixture was concentrated, the resulting residue was dissolved in chloroform, washed with aqueous potassium carbonate (0.5 M) and with saturated saline, dried over anhydrous sodium sulfate, and concentrated. The residue was dissolved in 4 ml of dichloromethane, and then 250 µl of triethylamine and 151 mg of 2,4,6-trichlorobenzenesulfonyl chloride were added thereto, followed by stirring the resulting mixture overnight at room temperature. Saturated aqueous sodium hydrogen carbonate solution was added to the reaction mixture, and the resulting mixture was extracted with chloroform. Organic phases were combined, washed with 10% aqueous citric acid solution and with saturated saline, dried over anhydrous sodium sulfate, and concentrated. The residue was purified by column chromatography (chloroform:methanol=50:1) to obtain 251 mg of methyl 3-((2,4,8-triaza-2-methyl-1-oxo-4-phenylspiro[4.5]dec-8-yl)-carbonylamino)-2-(((2,4,6-trichlorophenyl)sulfonyl)amino)propanoate (yield: 88%).
LR-MS(m/z):632(M+H)⁺
¹H-NMR (300MHz, CDCl₃, δppm): 1.65-1.70 (2H, m), 2.49-2.59 (2H, m), 3.01 (3H, s), 3.64-3.89 (6H, m), 3.61 (3H, s), 4.28 (1H, m), 4.68 (2H, s), 5.09 (1H, m), 6.74 (2H, d, J=8.20), 6.85 (1H, t, J=7.32), 7.22-7.28 (2H, m), 7.45 (2H, s).

### Example 6

### 3-((2,4,8-Triaza-2-methyl-1-oxo-4-phenylspiro[4.5]dec-8-yl)carbonylamino)-2-(((2,4,6 -trichlorophenyl)sulfonyl)amino)propanoic acid (6)

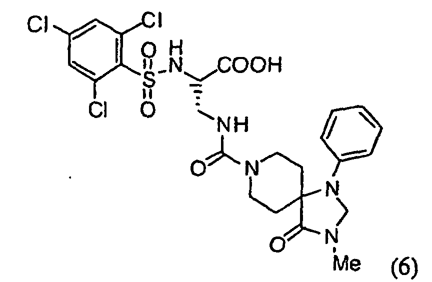

In 3 ml of tetrahydrofuran, 146 mg of methyl 3-((2,4,8-triaza-2-methyl-1-oxo-4-phenylspiro[4.5]dec-8-yl)carbonylamino)-2-(((2,4,6-trichlorophenyl)sulfonyl)amino)-propanoate was dissolved, and 2.8 ml of 0.1 N aqueous sodium hydroxide was added thereto at 0°C, followed by stirring the resulting mixture for 17 hours. Hydrochloric acid (0.1 N) was added to the reaction mixture, and the resulting mixture was extracted with ethyl acetate. Organic phases were combined, washed with saturated saline, dried over anhydrous sodium sulfate, and concentrated. The residue was reprecipitated from chloroform/diethyl ether, n-hexane to obtain 89.1 mg of 3-((2,4,8-triaza-2-methyl-1-oxo-4-phenylspiro[4.5]dec-8-yl)-carbonylamino)-2-(((2,4,6-trichlorophenyl)sulfonyl)amino)propanoic acid (yield: 63%).
LR-MS(m/z):618 (M+H)⁺
¹H-NMR (300MHz, CDCl₃, δppm): 1.68-1.73 (2H, m), 2.44-2.55 (2H, m), 3.01 (3H, s), 3.61-3.08 (7H, m), 4.68 (2H, s), 5.56 (1H, m), 6.73 (2H, d, J=8.20), 6.87 (1H, t, J=7.32), 7.09 (1H, m), 7.26 (2H, d, J=8.20), 7.43 (2H, s).

### Example 7

### Methyl 2-(((2-chloro-6-methylphenyl)sulfonyl)amino)-3-((2,4,8-triaza-2-methyl-1-oxo-4-phenylspiro[4.5]dec-8-yl)carbonylamino)propanoate (7)

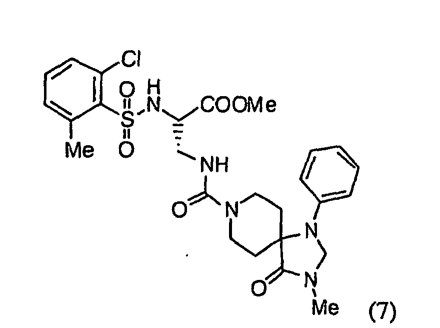

In 2 ml of dichloromethane, 220 mg of methyl 2-((t-butoxy)carbonylamino)-3-((2,4,8-triaza-2-methyl-1-oxo-4-phenylspiro[4.5]dec-8-yl)carbonylamino)propanoate was dissolved, and 1 ml of trifluoroacetic acid was added thereto, followed by stirring the resulting mixture at room temperature for 5 hours. After the reaction mixture was concentrated, the resulting residue was dissolved in chloroform, washed with aqueous potassium carbonate (0.5 M) and with saturated saline, dried over anhydrous sodium sulfate, and concentrated. The residue was dissolved in 4 ml of dichloromethane, and then 250 µl of triethylamine and 122 mg of 2-chloro-6-methylbenzenesulfonyl chloride were added thereto, followed by stirring the resulting mixture overnight at room temperature. Saturated aqueous sodium hydrogen carbonate solution was added to the reaction mixture, and the resulting mixture was extracted with chloroform. Organic layers were combined, washed with 10% aqueous citric acid solution and with saturated saline, dried over anhydrous sodium sulfate, and concentrated. The residue was purified by column chromatography (chloroform:methanol=50:1) to obtain 238 mg of methyl 2-(((2-chloro-6-methylphenyl)sulfonyl)amino)-3-((2,4,8-triaza-2-methyl-1-oxo-4-phenylspiro[4.5]dec-8-yl)carbonylamino)propanoate (yield: 92%).
LR-MS(m/z):578 (M+H)⁺
¹H-NMR (300MHz, CDCl₃, δppm): 1.65-1.70 (2H, m), 2.51-2.64 (2H, m), 2.67 (3H, s), 3.01 (3H, s), 3.50-3.77 (4H, m), 3.58 (3H, s), 3.84-3.88 (2H, m), 4.10 (1H, m), 4.68 (2H, s), 5.07 (1H, m), 6.68 (1H, m), 6.77 (2H, d, J=8.20), 6.84 (1H, t, J=7.32), 7.19-7.39 (5H, m).

### Example 8

### 2-(((2-Chloro-6-methylphenyl)sulfonyl)amino)-3-((2,4,8-triaza-2-methyl-1-oxo-4-phenylspiro[4.5]dec-8-yl)carbonylamino)propanoic acid (8)

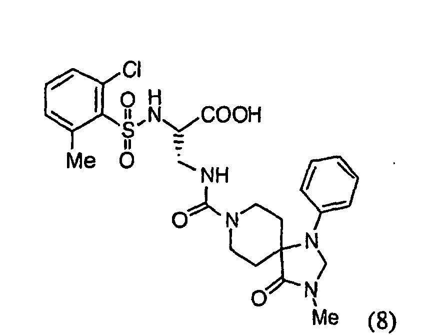

In 3 ml of tetrahydrofuran, 147 mg of methyl 2-(((2-chloro-6-methylphenyl)sulfonyl)amino)-3-((2,4,8-triaza-2-methyl-1-oxo-4-phenylspiro[4.5]dec-8-yl)c arbonylamino)propanoate was dissolved, and 3 ml of 0.1 N aqueous sodium hydroxide was added thereto at 0°C, followed by stirring the resulting mixture for 12 hours. Hydrochloric acid (0.1 N) was added to the reaction mixture, and the resulting mixture was extracted with ethyl acetate. Organic phases were combined, washed with saturated saline, dried over anhydrous sodium sulfate, and concentrated. The residue was reprecipitated from chloroform/diethyl ether, n-hexane to obtain 99.9 mg of 2-(((2-chloro-6-methylphenyl)sulfonyl)amino)-3-((2,4,8-triaza-2-methyl-1-oxo-4-phenylspiro[4.5]dec-8-yl)c arbonylamino)propanoic acid (yield: 71%).
LR-MS(m/z):564 (M+H)⁺
¹H-NMR (300MHz, CDCl₃, δppm): 1.68-1.72 (2H, m), 2.44-2.54 (2H, m), 2.64 (3H, s), 3.01 (3H, s), 3.61-3.81 (7H, m), 4.68 (2H, s), 5.52 (1H, m), 6.75 (2H, d, J=7.90), 6.84-6.89 (2H, m), 7.18 (1H, d, J=6.44), 7.24-7.37 (4H, m).

### Example 9

### Methyl 2-(((2S)-5-oxo-1-benzylpyrrolidine-2-yl)carbonylamino)-3-((2,4,8-triaza-2-methyl-1-oxo-4-phenylspiro[4.5]dec-8-yl)carbonylamino)propanoate (9)

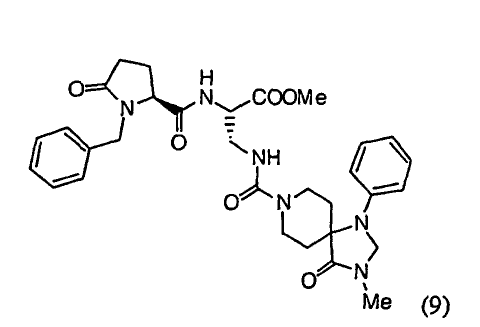

In 2 ml of dichloromethane, 183 mg of methyl 2-((t-butoxy)carbonylamino)-3-((2,4,8-triaza-2-methyl-1-oxo-4-phenylspiro[4.5]dec-8-yl)carbonylamino)propanoate was dissolved, and 1 ml of trifluoroacetic acid was added thereto, followed by stirring the resulting mixture at room temperature for 2.5 hours. After the reaction mixture was concentrated, the resulting residue was dissolved in chloroform, washed with aqueous potassium carbonate (0.5 M) and with saturated saline, dried over anhydrous sodium sulfate, and concentrated. The residue was dissolved in 3 ml of dichloromethane, and then 90 mg of (2S)-5-oxo-1-benzylpyrrolidine-2-carboxylic acid, 195 mg of BOP reagent and 259 µl of N,N-diisopropylethylamine were added thereto, followed by stirring the resulting mixture overnight at room temperature. Hydrochloric acid (0.1 N) was added to the reaction mixture, and the resulting mixture was extracted with ethyl acetate. Organic phases were combined, washed with saturated aqueous sodium hydrogen carbonate solution and with saturated saline, dried over anhydrous sodium sulfate, and concentrated. The residue was purified by column chromatography (chloroform:methanol=30:1) to obtain 96.9 mg of methyl 2-(((2S)-5-oxo-1-benzylpyrrolidine-2-yl)carbonylamino)-3-((2,4,8-triaza-2-methyl-1-oxo-4-phenylspiro [4.5]dec-8-yl)carbonylamino)propanoate (yield: 44%).
LR-MS(m/z):591 (M+H)⁺
¹H-NMR (300MHz, CD₃OD, δppm): 1.63-1.67 (2H, m), 2.20 (1H, m), 2.20-2.62 (5H, m), 2.98 (3H, s), 3.51-3.75 (4H, m), 3.72 (3H, s), 3.84-4.01 (4H, m), 4.47 (1H, t, J=6.44), 4.72 (2H, s), 4.99 (1H, d, J=14.9), 6.87-6.82 (3H, m), 7.18-7.34 (7H, m).

### Example 10

### 2-(((2S)-5-Oxo-1-benzylpyrrolidine-2-yl)carbonylamino)-3-((2,4,8-triaza-2-methyl-1-oxo-4-phenylspiro[4.5]dec-8-yl)carbonylamino)propanoic acid (10)

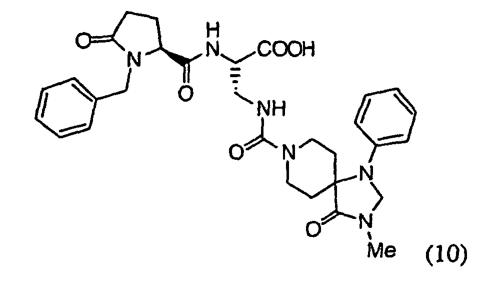

In 2 ml of tetrahydrofuran, 85 mg of methyl 2-(((2S)-5-oxo-1-benzylpyrrolidine-2-yl)carbonylamino)-3-((2,4,8-triaza-2-methyl-1-oxo-4-phenylspiro[4.5]dec-8-yl)-carbonylamino)propanoate was dissolved, and 1.5 ml of 0.1 N aqueous sodium hydroxide was added thereto at 0°C, followed by stirring the resulting mixture for 3.5 hours. Hydrochloric acid (0.1 N) was added to the reaction mixture, and the resulting mixture was extracted with ethyl acetate. Organic phases were combined, washed with saturated saline, dried over anhydrous sodium sulfate, and concentrated. The residue was reprecipitated from tetrahydrofuran/chloroform/diethyl ether/n-hexane to obtain 64.9 mg of 2-(((2S)-5-oxo-1-benzylpyrrolidine-2-yl)carbonylamino)-3-((2,4,8-triaza-2-methyl-1-oxo-4-phenylspiro[4.5] dec-8-yl)carbonylamino)propanoic acid (yield: 80%).
LR-MS(m/z):577 (M+H)⁺
¹H-NMR (300MHz, CD₃OD, δppm): 1.62-1.67 (2H, m), 2.03 (1H, m), 2.17-2.63 (5H, m), 2.98 (3H, s), 3.52-3.74 (4H, m), 3.81-3.99 (4H, m), 4.48 (1H, m), 4.71 (2H, s), 4.99 (1H, d, J=14.9), 6.77-6.81 (3H, m), 7.18-7.41 (7H, m).

### Example 11

### Methyl 2-(((2S)-5-oxo-1-benzylpyrrolidine-2-yl)carbonylamino)-3-((2,4,8-triaza-1-oxo-4-phenylsgiro[4.5]dec-8-yl)carbonylamino)propanoate (11)

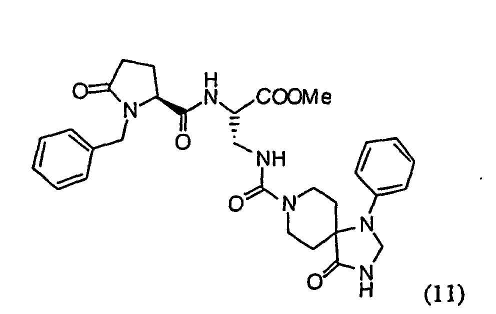

In 2 ml of dichloromethane, 114 mg of methyl 2-((t-butoxy)carbonylamino) -3-((2,4,8-triaza-1-oxo-4-phenylspiro[4.5]dec-8-yl)carbonylamino)propanoate was dissolved, and 1 ml of trifluoroacetic acid was added thereto, followed by stirring the resulting mixture at room temperature for 1.5 hours. After the reaction mixture was concentrated, the resulting residue was dissolved in chloroform, washed with aqueous potassium carbonate (0.5 M) and with saturated saline, dried over anhydrous sodium sulfate, and concentrated. The residue was dissolved in a mixed solvent of 2.5 ml of dichloromethane and 1.5 ml of tetrahydrofuran, and then 57 mg of (2S)-5-oxo-1-benzylpyrrolidine-2-carboxylic acid, 128 mg of BOP reagent and 168 µl of N,N-diisopropylethylamine were added thereto, followed by stirring the resulting mixture overnight at room temperature. Hydrochloric acid (0.1 N) was added to the reaction mixture, and the resulting mixture was extracted with ethyl acetate. Organic layers were combined, washed with saturated aqueous sodium hydrogen carbonate solution and with saturated saline, dried over anhydrous sodium sulfate, and concentrated. The residue was purified by column chromatography (chloroform:methanol=30:1) to obtain 54.4 mg of methyl 2-(((2S)-5-oxo-1-benzylpyrrolidine-2-yl)carbonylamino)-3-((2,4,8-triaza-1-oxo-4-phenylspiro [4.5]dec-8-yl)carbonylamino)propanoate (yield: 39%).
LR-MS(m/z):577 (M+H)⁺
¹H-NMR (300MHz, CDCl₃, δppm):1.73-1.80 (2H, m), 2.06 (1H, m), 2.10-2.67 (5H, m), 3.57-3.95 (7H, m), 3.77 (3H, s), 4.55 (1H, m), 4.73 (2H, s), 5.11-5.16 (2H, m), 6.74 (2H, d, J=8.49), 6.83-6.91 (2H, m), 7.21-7.32 (7H, m), 8.22 (1H, m).

### Example 12

### 2-(((2S)-5-Oxo-1-benzylpyrrolidine-2-yl)carbonylamino)-3-((2,4,8-triaza-1-oxo-4-phenylspiro[4.5]dec-8-yl)carbonylamino)propanoic acid (12)

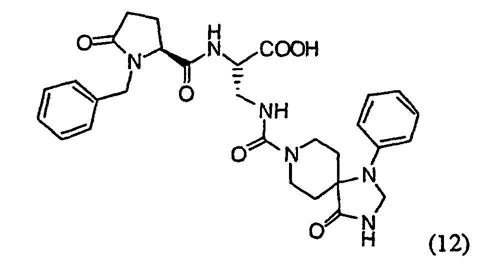

In 1 ml of tetrahydrofuran, 51.9 mg of methyl 2-(((2S)-5-oxo-1-benzylpyrrolidine-2-yl)carbonylamino)-3-((2,4,8-triaza-1-oxo-4-phenylspiro(4.5]dec-8-yl)carbonylamino)-propanoate was dissolved, and 1 ml of 0.1 N aqueous sodium hydroxide was added thereto at 0°C, followed by stirring the resulting mixture for 16 hours. Hydrochloric acid (0.1 N) was added to the reaction mixture, and the resulting mixture was extracted with ethyl acetate. Organic phases were combined, washed with saturated saline, dried over anhydrous sodium sulfate, and concentrated. The residue was reprecipitated from methanol/ethyl acetate/n-hexane to obtain 34.3 mg of 2-(((2S)-5-oxo-1-benzylpyrrolidine-2- yl)carbonylamino)-3-((2,4,8-triaza-1-oxo-4-phenylspiro[ 4.5]dec-8-yl)carbonylamino)propanoic acid (yield: 68 %).
LR-MS(m/z):563 (M+H)⁺
¹H-NMR (300MHz, CD₃OD δpm):1.66-1.71 (2H, m), 2.02 (1H, m), 2.19-2.62 (5H, m), 3.52-3.69 (4H, m), 3.81-4.00 (4H, m), 4.47 (1H, m), 4.69 (2H, s), 4.99 (1H, d, J=14.9), 6.76-6.81 (3H, m), 7.18-7.33 (7H, m).

### Example 13

### Methyl 2-(oxolane-2-ylcarbonylamino)-3-((2,4,8-triaza-1-oxo-4-phenylspiro[4.5]dec-8-yl)carbonylamino)propanoate (13)

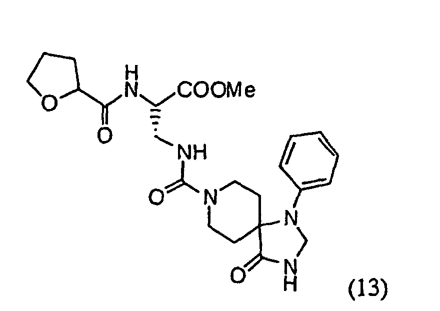

In 5 ml of dichloromethane, 150 mg of methyl 2-((t-butoxy)carbonylamino)-3-((2,4,8-triaza-1-oxo-4-phenylspiro[4.5]dec-8-yl)carbonylamino)propanoate was dissolved, and 1.5 ml of trifluoroacetic acid was added thereto, followed by stirring the resulting mixture at room temperature for 1.5 hours. After the reaction mixture was concentrated, the resulting residue was dissolved in chloroform, washed with aqueous potassium carbonate (0.5 M) and with saturated saline, dried over anhydrous sodium sulfate, and concentrated. The residue was dissolved in a mixed solvent of 2.5 ml of dichloromethane and 1.5 ml of tetrahydrofuran, and then 34 µl of oxolane-2-carboxylic acid, 167 mg of BOP reagent and 220 µl of N,N-diisopropylethylamine were added thereto, followed by stirring the resulting mixture overnight at room temperature. Hydrochloric acid (0.1 N) was added to the reaction mixture, and the resulting mixture was extracted with ethyl acetate. Organic layers were combined, washed with saturated aqueous sodium hydrogen carbonate solution and with saturated saline, dried over anhydrous sodium sulfate, and concentrated. The residue was purified by column chromatography (chloroform/methanol=80:1) to obtain 52.6 mg of methyl 2-(oxolane-2-ylcarbonylamino)-3-((2,4,8-triaza-1-oxo-4-phenylspiro[4.5]dec-8-yl)-carbonylamino)propanoate (yield: 34%).
LR-MS(m/z):474 (M+H)⁺
¹H-NMR (300MHz, CDCl₃, δppm):1.73-2.11 (5H, m), 2.26 (1H, m), 2.42-2.59 (2H, m), 3.55-4.03 (8H, m), 3.76 (3H, s), 4.38 (1H, m), 4.60 (1H, m), 4.74 (2H, s), 5.47 (1H, m), 6.74-6.77 (2H, m), 6.88 (1H, m), 7.24-7.33 (2H, m), 7.92 (1H, m).

### Example 14

### 2-(Oxolane-2-ylcarbonylamino)-3-((2,4,8-triaza-1-oxo-4-phenylspiro[4.5]dec-8-yl)-carbonylamino)propanoic acid (14)

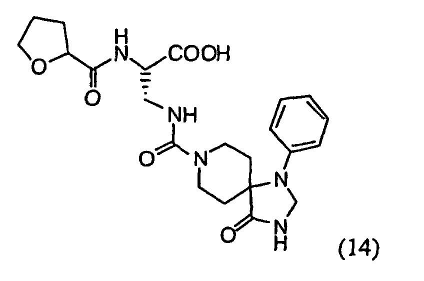

In 1 ml of tetrahydrofuran, 45.2 mg of methyl 2-(oxolane-2-ylcarbonylamino)-3-((2,4,8-triaza-1-oxo-4-phenylspiro[4.5]dec-8-yl)carbonylamino)propanoate was dissolved, and 1 ml of 0.1 N aqueous sodium hydroxide was added thereto at 0°C, followed by stirring the resulting mixture for 7.5 hours. Hydrochloric acid (0.1 N) was added to the reaction mixture, and the resulting mixture was extracted with ethyl acetate. Organic phases were combined, washed with saturated saline, dried over anhydrous sodium sulfate, and concentrated. The residue was reprecipitated from methanol/diethyl ether to obtain 27.5 mg of 2-(oxolane-2-ylcarbonylamino)-3-((2,4,8-triaza-1-oxo-4-phenylspiro[4.5]dec-8-yl)-carbonylamino)propanoic acid (yield: 63%).
LR-MS(m/z):460 (M+H)⁺
¹H-NMR (300MHz, CD₃OD, δppm):1.64-1.74 (2H, m), 1.80-2.09 (3H, m), 2.21 (1H, m), 2.43-2.64 (2H, m), 3.55-4.06 (8H, m), 4.32 (1H, m), 4.44 (1H, m), 4.70 (2H, s), 6.78-6.82 (3H, m), 7.22-7.27 (2H, m).

### Example 15

### Methyl 2-(((2S)-1-acetylpyrrolidine-2-yl)carbonylamino)-3-((2,4,8-triaza-1-oxo-4-phenylspiro[4.5]dec-8-yl)carbonylamino)propanoate (15)

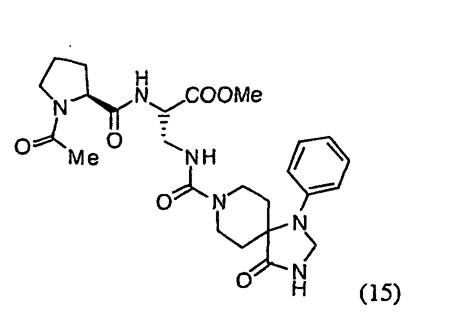

In 2 ml of dichloromethane, 114 mg of methyl 2-((t-butoxy)carbonylamino)-3-((2,4,8-triaza-1-oxo-4-phenylspiro[4.5]dec-8-yl)carbonylamino)propanoate was dissolved, and 1 ml of trifluoroacetic acid was added thereto, followed by stirring the resulting mixture at room temperature for 1.5 hours. After the reaction mixture was concentrated, the resulting residue was dissolved in chloroform, washed with aqueous potassium carbonate (0.5 M) and with saturated saline, dried over anhydrous sodium sulfate, and concentrated. The residue was dissolved in a mixed solvent of 2.5 ml of dichloromethane and 1.5 ml of tetrahydrofuran, and then 40.9 mg of (2S)-1-acetylpyrrolidine-2-carboxylic acid, 128 mg of BOP reagent and 168 µl of N,N-diisopropylethylamine were added thereto, followed by stirring the resulting mixture overnight at room temperature. Hydrochloric acid (0.1 N) was added to the reaction mixture, and the resulting mixture was extracted with ethyl acetate. Organic phases were combined, washed with saturated aqueous sodium hydrogen carbonate solution and with saturated saline, dried over anhydrous sodium sulfate, and concentrated. The residue was purified by column chromatography (chloroform:methanol=30:1) to obtain 38.8 mg of methyl 2-(((2S)-1-acetylpyrrolidine-2-yl)carbonylamino)-3-((2,4,8-triaza-1-oxo-4-phenylspiro[4.5] dec-8-yl)carbonylamino)propanoate (yield: 31%).
LR-MS(m/z):515 (M+H)⁺
¹H-NMR (300MHz, CDCl₃, δppm):1.66-2.19 (6H, m), 2.01 (3H, s), 2.53-2.64 (2H, m), 3.41-3.82 (6H, m), 3.79 (3H, s), 3.90-4.03 (2H, m), 4.26 (1H, m), 4.57 (1H, m), 4.74 (2H, s), 5.86 (1H, m), 6.72-7.00 (4H, m), 7.21-7.35 (3H, m).

### Example 16

### 2-(((2S)-1-Acetylpyrrolidine-2-yl)carbonylamino)-3-((2,4,8-triaza-1-oxo-4-phenylspiro [4.5]dec-8-yl)carbonylamino)propanoic acid (16)

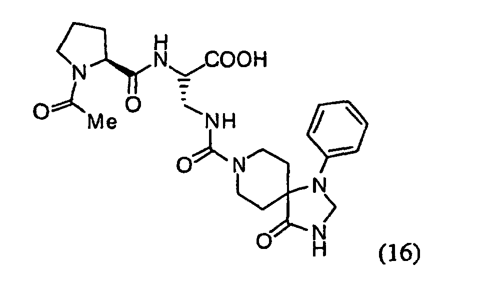

In 1 ml of tetrahydrofuran, 36.4 mg of methyl 2-(((2S)-1-acetylpyrrolidine-2-yl)carbonylamino)-3-((2,4,8-triaza-1-oxo-4-phenylspiro[4.5]dec-8-yl)carbonylamino)-propanoate was dissolved, and 0.8 ml of 0.1 N aqueous sodium hydroxide was added thereto at 0°C, followed by stirring the resulting mixture for 16 hours. Hydrochloric acid (0.1 N) was added to the reaction mixture, and the resulting mixture was extracted with ethyl acetate. Organic phases were combined, washed with saturated saline, dried over anhydrous sodium sulfate, and concentrated. The residue was reprecipitated from methanol/ethyl acetate/n-hexane to obtain 9.4 mg of 2-(((2S)-1-acetylpyrrolidine-2-yl)carbonylamino)-3-((2,4,8-triaza-1-oxo-4-phenylspiro[4.5]dec-8-yl)carbonylamino)propanoic acid (yield: 27%).
LR-MS(m/z):501 (M+H)⁺
¹H-NMR (300MHz, CD₃OD, δppm):1.65-1.69 (2H, m), 1.86-2.32 (4H, m), 2.00 (3H, s), 2.44-2.58 (2H, m), 3.44-3.80 (6H, m), 3.89-3.95 (2H, m), 4.38-4.53 (2H, m), 4.85 (2H, s), 6.78-6.82 (3H, m), 7.22-7.27 (2H, m).

### Example 17

### Methyl 2-((1-acetyl(2-piperidyl))carbonylamino)-3-((2,4,8-triaza-1-oxo-4-phenylspiro [4.5]dec-8-yl)carbonylamino)propanoate (17)

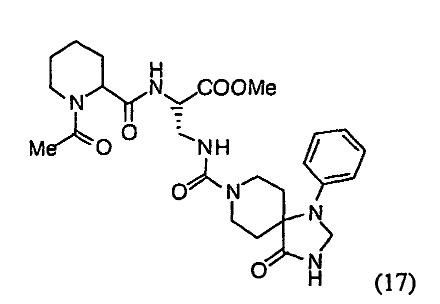

In 2 ml of dichloromethane, 114 mg of methyl 2-((t-butoxy)carbonylamino)-3-((2,4,8-triaza-1-oxo-4-phenylspiro[4.5]dec-8-yl)carbonylamino)propanoate was dissolved, and 1 ml of trifluoroacetic acid was added thereto, followed by stirring the resulting mixture at room temperature for 1.5 hours. After the reaction mixture was concentrated, the resulting residue was dissolved in chloroform, washed with aqueous potassium carbonate (0.5 M) and with saturated saline, dried over anhydrous sodium sulfate, and concentrated. The residue was dissolved in a mixed solvent of 2.5 ml of dichloromethane and 1.5 ml of tetrahydrofuran, and then 53.1 mg of 1-acetylpiperidine-2-carboxylic acid, 128 mg of BOP reagent and 168 µl of N,N-diisopropylethylamine were added thereto, followed by stirring the resulting mixture overnight at room temperature. Hydrochloric acid (0.1 N) was added to the reaction mixture, and the resulting mixture was extracted with ethyl acetate. Organic layers were combined, washed with saturated aqueous sodium hydrogen carbonate solution and with saturated saline, dried over anhydrous sodium sulfate, and concentrated. The residue was purified by column chromatography (chloroform:methanol=30:1) to obtain 40.5 mg of methyl 2-((1-acetyl(2-piperidyl))carbonylamino)-3-((2,4,8-triaza-1-oxo-4-phenylspiro[4.5]dec-8-yl)-carbonylamino)propanoate (yield: 32%).
LR-MS(m/z):529 (M+H)⁺
¹H-NMR (300MHz, CDCl₃, δppm):1.30-1.77 (7H, m), 2.10-2.20 (3H, m), 2.27-2.60 (3H, m), 3.28 (1H, m), 3.60-3.96 (9H, m), 4.50 (1H, m), 4.74 (2H, s), 5.14-5.37 (2H, m), 6.75 (2H, d, J=8.49), 6.84-6.94 (2H, m), 7.25-7.32 (2H, m).

### Example 18

### 2-((1-Acetyl(2-piperidyl))carbonylamino)-3-((2,4,8-triaza-1-oxo-4-phenylspiro[4.5] dec-8-yl)carbonylanuno)propanoic acid (18)

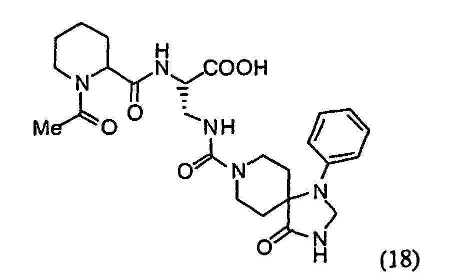

In 1 ml of tetrahydrofuran, 36.8 mg of methyl 2-((1-acetyl(2-piperidyl))carbonylamino)-3-((2,4,8-triaza-1-oxo-4-phenylspiro[4.5]dec-8-yl)-carbonylamino)propanoate was dissolved, and 0.8 ml of 0.1 N aqueous sodium hydroxide was added thereto at 0°C, followed by stirring the resulting mixture for 16 hours. Hydrochloric acid (0.1 N) was added to the reaction mixture, and the resulting mixture was extracted with ethyl acetate. Organic phases were combined, washed with saturated saline, dried over anhydrous sodium sulfate, and concentrated. The residue was reprecipitated from methanol/ethyl acetate/n-hexane to obtain 18.7 mg of 2-((1-acetyl(2-piperidyl))-carbonylamino)-3-((2,4,8-triaza-1-oxo-4-phenylspiro[4.5]dec-8-yl)carbonylamino)propanoic acid (yield: 52%).
LR-MS(m/z):515 (M+H)⁺
¹H-NMR (300MHz, CD₃OD, δppm):1.47-1.70 (7H, m), 2.07-2.22 (3H, m), 2.32 (1H, m), 2.46-2.65 (2H, m), 3.38-3.79 (6H, m), 3.88-4.05 (2H, m), 4.39 (1H, m), 4.69 (2H, s), 5.23 (1H, m), 6.75-6.82 (3H, m), 7.22-7.27 (2H, m).

### Example 19

### Wang resin loaded with 2-[(9H-fluorene-9-ylmethoxy)carbonylamino]-3-[1-(4,4-dimethyl-2,6-dioxocyclohex-1-ylidene)-3-methylbutylamino]propanoic acid (101)

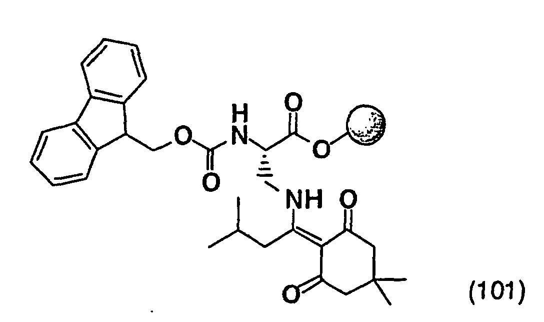

### (wherein in the structural formula, • represents a Wang resin; hereinafter the same unless otherwise noted)

Into a 50 ml syringe reaction vessel equipped with a filter, 2 g of a Wang resin (load: 1.3 mmol/g) was charged, then N-α-[(9H-fluorene-9-ylmethoxy)carbonyl]-N-ε-1-(4,4-dimethyl-2,6-dioxocyclohex-1-ylidene)-3-methylbutyl-L-diaminopropanoic acid (3.2 g, 6 mmol), dimethylaminopyridine (73 mg, 0.6 mmol), dimethylformamide (20 ml) and dicyclohexylcarbodiimide (1.0 ml, 6.6 mmol) were added thereto, and the resulting mixture was shaken for 4 days. The resin was filtered, then washed with dimethylformamide, methanol, tetrahydrofuran and methylene chloride (respectively 20 ml, 5 times each), and vacuum dried to obtain a Wang resin loaded with 2-[(9H-fluorene-9-ylmethoxy)-carbonylamino]-3-[1-(4,4-dimethyl-2,6-dioxohex-1-ylidene)-3-methylbutylamino]propanoic acid (3.2 g, maximum load 0.81 mmol/g).

Example compound 102 was obtained in the same manner.

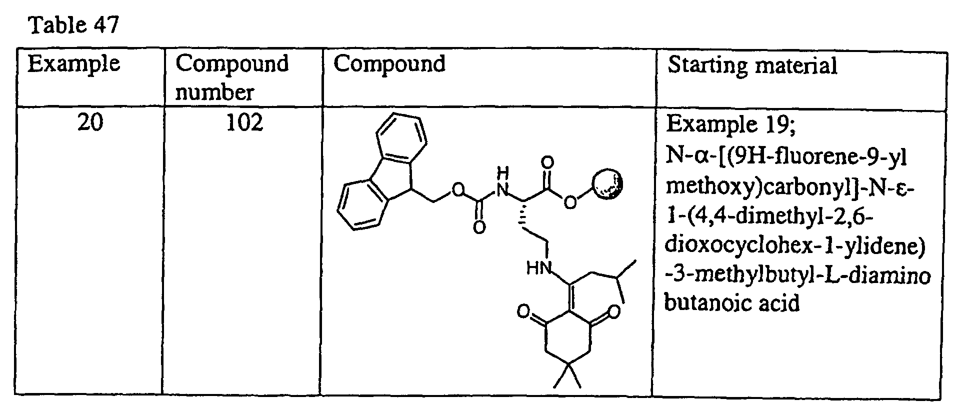

### Example 21

Wang resin loaded with 2-(2,4,6-trichlorobenzoylamino)-3-[1-(4,4-dimethyl-2,6-dioxocyclohex-1-ylidene)-3-methylbutylamino]propanoic acid (103)

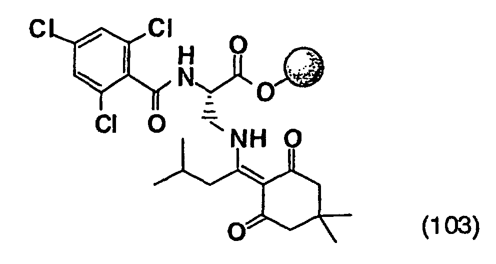

A Wang resin loaded with 2-[(9H-fluorene-9-ylmethoxy)carbonylamino]-3-[1-(4,4-dimethyl-2,6-dioxocyclohex-1-ylidene)-3-methylbutylamino]propanoic acid was charged into 96 MicroKans (20 mg each, ca 16 µmol). The 96 MicroKans and a solution of 10% piperidine in methylene chloride (100 ml) were charged into a 500 ml flask, and the resulting mixture was shaken at room temperature for 1 hour. The resin was filtered, then washed with dimethylformamide, methanol, tetrahydrofuran and methylene chloride (respectively 100 ml, 5 times each), and vacuum dried. Methylene chloride (12 ml), 2,4,6-trichlorobenzoyl chloride (560 µl, 3.6 mmol) and triethylamine (830 µl, 5.4 mmol) were charged into 12 MicroKans. The reaction mixture was shaken at room temperature for 16 hours, and filtered. The resin was washed with dimethylformamide, methanol, tetrahydrofuran and methylene chloride (respectively 10 ml, 5 times each), and vacuum dried to obtain a Wang resin loaded with 2-(2,4,6-trichlorobenzoylamino)-3-[1-(4,4-dimethyl-2,6-dioxocyclohex-1-ylidene)-3-methylbutylamino]propanoic acid.

### Examples 22 to 27

The compounds 104 to 109 given in Table 48 were obtained in the same manner as in Example 21.

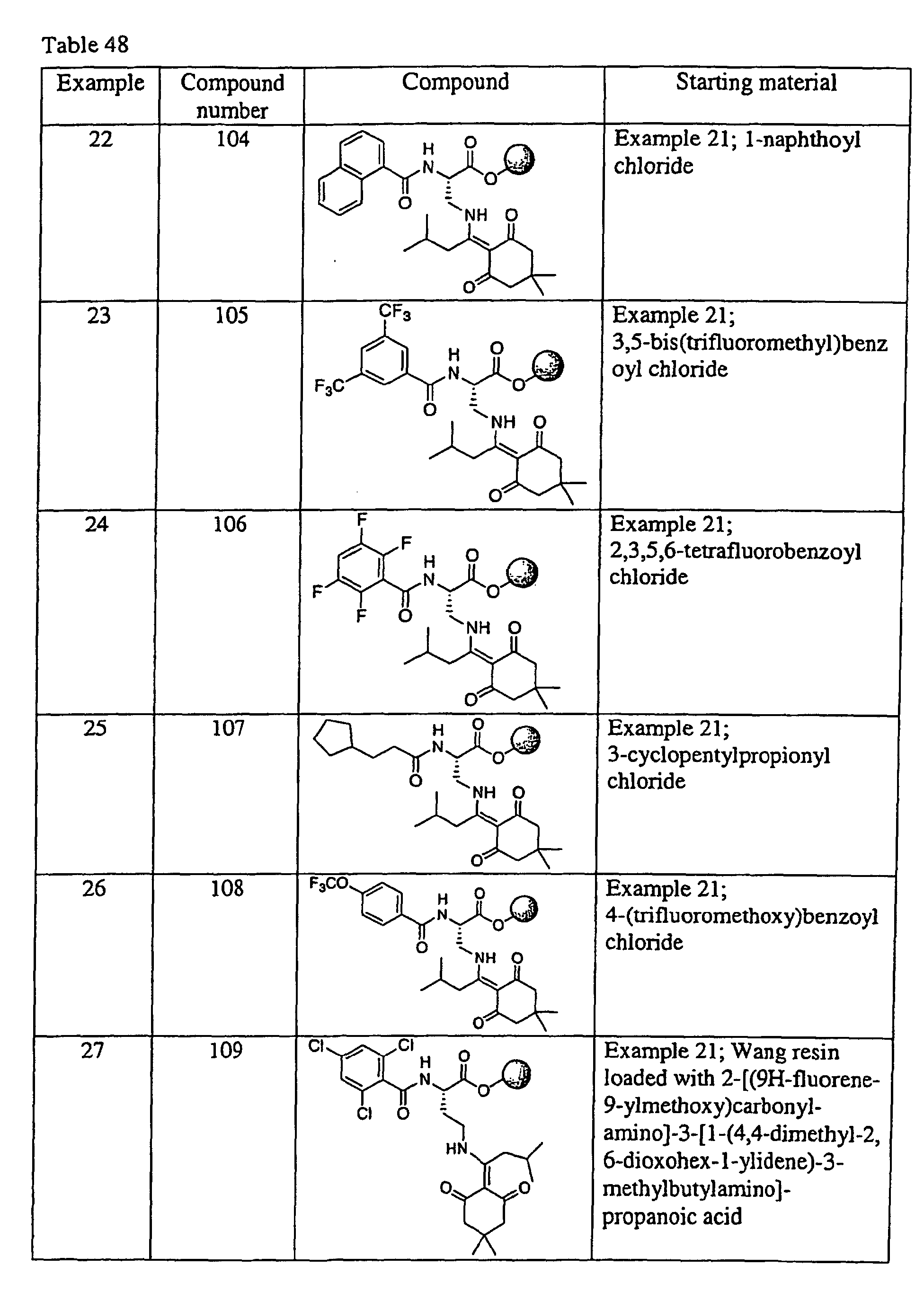

### Example 28

### 2-(2,4,6-trichlorobenzoylamino)-3-((2,4,8-triaza-2-methyl-1-oxo-4-phenylspiro[4.5] dec-8-yl)carbonylamino)propanoic acid (110)

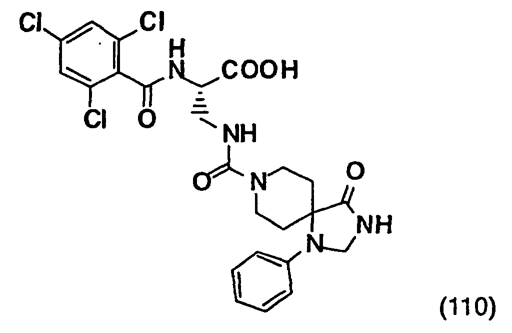

A 2% hydrazine/dimethylformamide solution (1 ml) was charged into a MicroKan (one) that was filled with a Wang resin loaded with 2-(2,4,6-trichlorobenzoylamino)-3-[1-(4,4-dimethyl-2,6-dioxocyclohex-1-ylidene)-3-methylbutylamino]propanoate, and the mixture was shaken at room temperature for 1 hour. The resin was filtered, then washed with dimethylformamide, methanol, tetrahydrofuran and methylene chloride (respectively 2 ml, 5 times each), and vacuum dried. A solution of 4-nitrophenyl chloroformate (60 mg, 0.3 mmol) and diisopropylethylamine (52 µl, 0.3 mmol) in methylene chloride-tetrahydrofuran (1:1 mixing solvent, 1 ml) was added to the resin and shaken for 20 minutes at room temperature. The resulting mixture was filtered, then washed with a solution of methylene chloride-tetrahydrofuran (1:1 mixing solvent, 2 ml, 3 times), and vacuum dried. A solution of 1-phenyl-1,3,8-triazaspiro[4.5]decan-4-one (48 mg, 0.2 mmol) and triethylamine (28 µl, 0.2 mmol) in dimethylformamide (1 ml) was added to the resin, and the mixture was shaken for 20 minutes at room temperature. After filtering, the resin was washed with dimethylformamide, methanol, tetrahydrofuran and methylene chloride (respectively 2 ml, 5 times each), and vacuum dried. A 10% solution of trifluoroacetic acid/methylene chloride (2 ml) was added to the resin, and shaken for 30 minutes. After filtering, the resin was washed with methylene chloride (1 ml, 2 times). The filtrate was collected, concentrated, then vacuum dried to obtain 2-(2,4,6-trichlorobenzoylamino)-3-((2,4,8-triaza-2-methyl-1-oxo-4-phenylspiro[4.5] dec-8-yl)carbonylamino)propanoic acid.
LC-MS Data
Column: Xterra ODS 5 µm, 4.6 × 50 mm (Waters)
Developing solvent: 0.1% aqueous formic acid solution:.0.1% formic acid/acetonitrile solution =90:10 (0 minutes) → 10:90 (3-5 minutes)
MS (m/z) (M+H)⁺ = 568
Retention time: 4.06 minutes

### Examples 29 to 34

The compounds 111 to 116 given in Table 49 were obtained in the same manner as in Example 28.

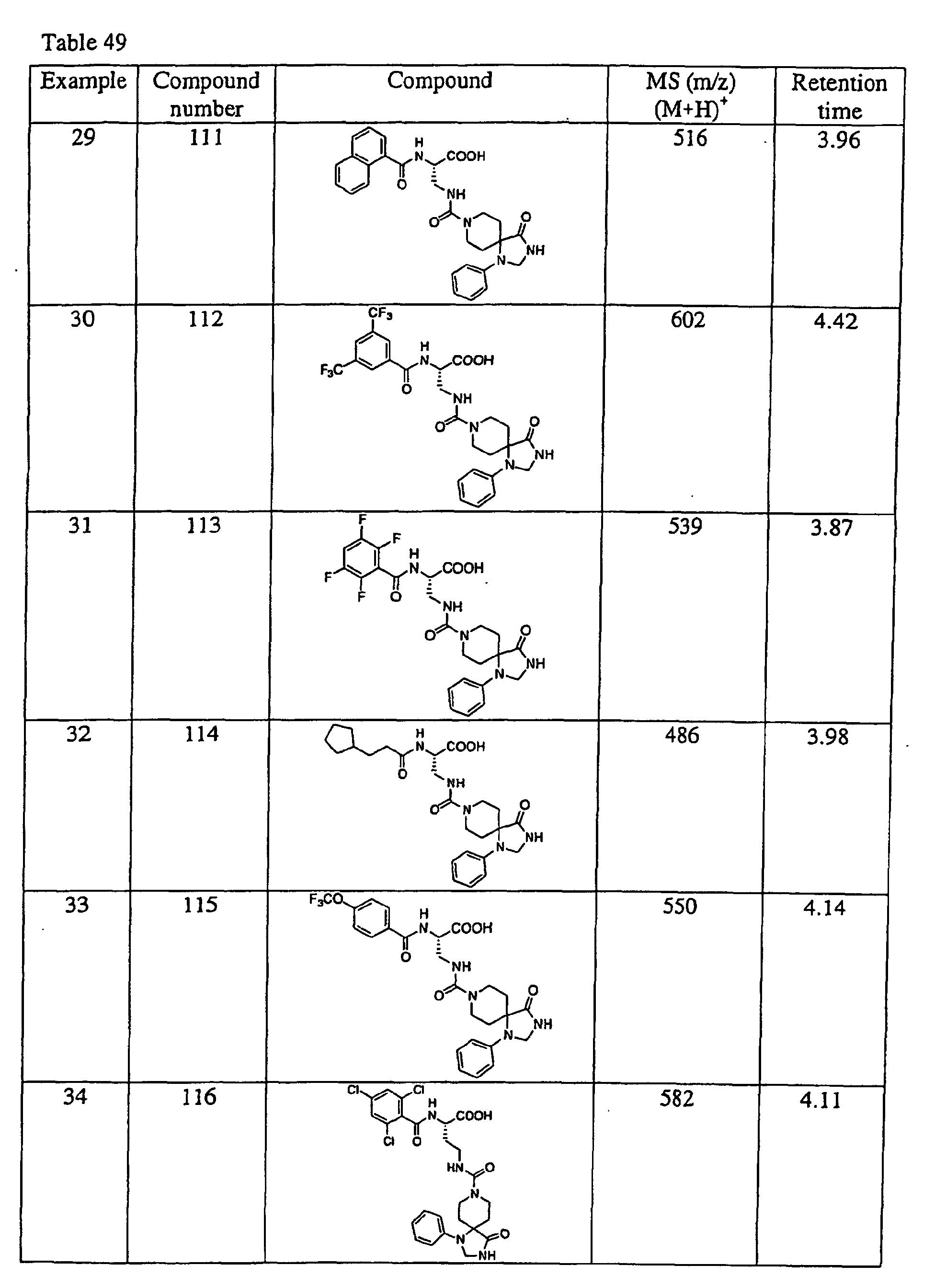

### Example 35

### Inhibitory Effect of Compounds Against Binding between CS-1 Peptide and Soluble VLA-4

The test compounds were evaluated in accordance with the method described in WO 98/32771. That is, in accordance with the teaching of a report (J. Bio. Chem., 262, 6886 (1987)), a conjugate between a peptide (Gys Leu His Gly Pro Glu Glu De Leu Asp Val Pro Ser Thr) containing CS-1 sequence and rabbit IgG (Sigma) was prepared. This was diluted with a phosphate buffer (hereinafter abbreviated as "PBS(-)"), and the obtained solution was placed in the wells of a 96-well immunoplate (NUNC) in an amount of 100 µl/well, followed by leaving to stand the immunoplate at 4°C for 16 hours to immobilize the conjugate.

The wells were then washed twice with PBS(-), and 1% BSA solution in PBS, which BSA was heated at 80°C for 10 minutes, was placed in each well in an amount of 300 µl/well. The immunoplate was left to stand at 4°C for 3 hours, and then the solution in each well was removed by suction.

Each compound and soluble VLA-4 (100 µl) were preliminarily reacted at room temperature for 20 minutes, and then the resulting mixture was allowed to react with the CS-1 peptide in each well at 30°C for 3 hours. Thereafter, non-bound soluble VLA-4 was removed by suction, and each well was washed twice with 0.1% BSA-containing TBS buffer (150 mM NaCl, 25 mM Tris-HCl, 1 mM MnCl₂, pH 7.4). To the bound soluble VLA-4, avidin-labelled horseradish peroxidase (Sigma)-labelled antibody was added, thereby allowing the reactions. Then o-phenylenediamine as a substrate was added to color the reaction solution, and the absorbance at 490 nm was measured. From this absorbance, the binding inhibitory activity of each compound was determined. The inhibitory activities of the representative compounds are shown in Table 50.

**Table 50**

| Compound number | Inhibitory activity (IC₅₀:nM) |
|---|---|
| 8 | 110 |
| 16 | 110 |
| 18 | 65 |

### Example 36

### Inhibitory Effect of Compounds Against Mouse Allergic Inflammation

Using a seven-week old male Balb/c mouse (Charles River Japan, Inc.), sensitization was carried out by intravenously administering anti-DNP mouse IgE antibody. Twenty-four hours after sensitization, 0.38% DNFB (2,4-dinitrofluorobenzene) which was dissolved in a mixed solution of acetone:olive oil at 4:1 was applied to the right auricle of the mouse to induce inflammation. The thickness of both auricles was measured each hour after induction with a thickness gauge to calculate the ear swelling ratio (%) after induction versus pre-induction. The test compounds were dissolved in ethanol for applying to the auricle before induction.

Compound 16 suppressed the increase in the ratio of ear swelling in a dose-dependent manner from DNFB induction, wherein the ED 50 value thereof was 0.8 mg/kg.

All publications, patents and patent applications cited herein are incorporated herein by reference in their entirety.

### Industrial Applicability

According to the present invention, a novel substance is provided which inhibits cell infiltration via adhesion molecules, especially adhesion molecule VLA-4, thereby making it possible to prevent and treat inflammatory diseases caused by infiltration of leukocytes such as monocytes, lymphocytes and eosinophils.

## Claims

1. A spiro derivative or a pharmaceutically acceptable salt thereof represented by Formula I, 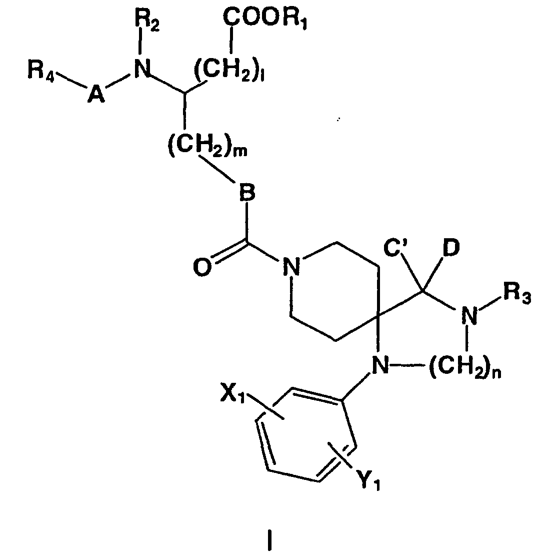 wherein I and m each independently represent an integer of 0 to 2;
n represents an integer of 1 to 3;
A represents -C(O)- or -S(O)₂-;
B represents -CH₂- or -NH-;
C' and D both represent a hydrogen atom, or C' and D represent together =O;
X₁ and Y₁ independently represent hydrogen, halogen, C₁₋₈ alkyl, trifluoromethyl, C₁₋₈ alkoxy, cyano, nitro, hydroxyl, amino, or tetrazolyl;
R₁ represents hydrogen, C₁₋₆ linear alkyl, C₃₋₈ branched alkyl, benzyl or -CH₂OC(O)C(CH₃)₃;
R₂ represents hydrogen or C₁₋₆ linear alkyl;
R₃ represents hydrogen, C₁₋₆ linear alkyl, C₃₋₈ branched alkyl, allyl, homoallyl, C₃₋₈ cycloalkyl-C₁₋₈ alkyl, unsubstituted phenyl or phenyl substituted with at least one substituent of substituent group E, unsubstituted benzyl or benzyl substituted with at least one substituent of substituent group E, unsubstituted phenethyl or phenethyl substituted with at least one substituent of substituent group E, unsubstituted styryl or styryl substituted with at least one substituent of substituent group E, unsubstituted naphthyl or naphthyl substituted with at least one substituent of substituent group E, or unsubstituted naphthylmethyl or naphthylmethyl substituted with at least one substituent of substituent group E (wherein substituent group E consists of halogen, C₁₋₈ alkyl, C₁₋₈ alkoxy, trifluoromethyl, trifluoromethoxy, C₁₋₈ alkylthio, cyano, nitro, hydroxyl, amino, C₁₋₈ alkylacyl, C₁₋₈ alkylacylamino and tetrazolyl);
R₄ represents C₁₋₈ alkyl, C₂₋₈ alkenyl, C₂₋₈ alkynyl, Cy, Cy-C₁₋₈ alkyl, Cy-C₂₋₈ alkenyl, Cy-C₂₋₈ alkynyl, Ar, Ar-C₁₋₈ alkyl, Ar-C₂₋₈ alkenyl or Ar-C₂₋₈ alkynyl, wherein the alkyl, alkenyl and alkynyl may be linear or branched, and may be substituted with 1 to 4 of R₅ independently selected, wherein
Cy is C₃₋₈ cycloalkyl that may be substituted with 1 to 4 substituents of R₆ or 3- to 8-membered monocyclic or bicyclic heterocycle that includes 1 to 4 nitrogen atoms, oxygen atoms or sulfur atoms independently selected, which heterocycle may be substituted with 1 to 4 substituents of R₆ (with the proviso that the hetero atoms do not bond directly with A),
Ar is phenyl that may be substituted with 1 to 5 substituents of R₇, naphthyl that may be substituted with 1 to 5 substituents of R₇, or 5- to 8-membered monocyclic or bicyclic heteroaryl that includes I to 4 nitrogen atoms, oxygen atoms or sulfur atoms independently selected, which heterocycle may be substituted with 1 to 5 substituents of R₇ (wherein the hetero atoms do not directly bond with A),
R₅ is halogen, trifluoromethyl, -OR^{a} or -SR^{a},
R^{a} is hydrogen, C₁₋₈ alkyl, allyl, homoallyl, trifluoromethyl, phenyl or benzyl,
R₆ and R₇ are, each independently, R₅, C₁₋₈ alkyl, C₂₋₈ alkenyl, C₂₋₈ alkynyl, C₃₋₈ cycloalkyl-C₁₋₈ alkyl, cyano, nitro, =O, -SO₂R^{b}, -SO₂NR^{c}R^{d}, -C(O)R^{b}, -C(O)OR^{b}, -C(O)NR^{c}R^{d}, -NR^{c}R^{d}, -NR^{c}C(O)R^{b}, -NR^{c}SO₂R^{b}, unsubstituted phenyl or phenyl substituted with at least one substituent of substituent group E or unsubstituted benzyl or benzyl substituted with at least one substituent of substituent group E,
R^{b}, R^{c} and R^{d} are each independent, and are hydrogen, C₁₋₈ alkyl, C₃₋₈ cycloalkyl, C₃₋₈ cycloalkyl-C₁₋₈ alkyl, unsubstituted phenyl or phenyl substituted with at least one substituent of substituent group E, unsubstituted benzyl or benzyl substituted with at least one substituent of substituent group E, unsubstituted phenethyl or phenethyl substituted with at least one substituent of substituent group E, unsubstituted styryl or styryl substituted with at least one substituent of substituent group E, unsubstituted naphthyl or naphthyl substituted with at least one substituent of substituent group E, or unsubstituted naphthylmethyl or naphthylmethyl substituted with at least one substituent of substituent group E
(with the proviso that excluded are the compounds in which, when A is -C(O)-, R₄ is C₁₋₇ linear alkyl, C₃₋₉ branched alkyl, adamantyl, benzyl or phenethyl substituted with 0 to 2 substituents of halogen, C₁₋₈ alkyl, C₁₋₈ alkoxy, cyano, nitro, hydroxyl, amino, or tetrazolyl; Formula II, 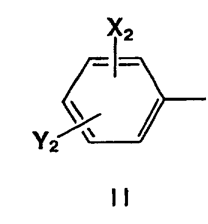 wherein X₂ and Y₂ each independently represent hydrogen, halogen, alkyl containing 1 to 8 carbon atoms, alkoxy containing 1 to 8 carbon atoms, cyano, nitro, hydroxyl, amino, or tetrazolyl; or
Formula III 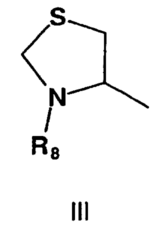 wherein R₈ represents linear alkyl containing 1 to 6 carbon atoms, branched alkyl containing 3 to 8 carbon atoms, linear alkylacyl containing 1 to 6 carbon atoms, branched alkylacyl containing 3 to 8 carbon atoms, cycloalkylacyl containing 5 to 7 carbon atoms, linear alkylsulfonyl containing 1 to 6 carbon atoms or branched alkylsulfonyl containing 3 to 8 carbon atoms, or
benzoyl substituted with 0 to 2 substituents of halogen, alkyl containing 1 to 8 carbon atoms, alkoxy containing 1 to 8 carbon atoms, cyano, nitro, hydroxyl, amino or tetrazolyl, phenylsulfonyl substituted with 0 to 2 substituents of halogen, alkyl containing 1 to 8 carbon atoms, alkoxy containing 1 to 8 carbon atoms, cyano, nitro, hydroxyl, amino or tetrazolyl, benzyl substituted with 0 to 2 substituents of halogen, alkyl containing 1 to 8 carbon atoms, alkoxy containing 1 to 8 carbon atoms, cyano, nitro, hydroxyl, amino or tetrazolyl).

2. A spiro derivative or a pharmaceutically acceptable salt thereof represented by Formula I, 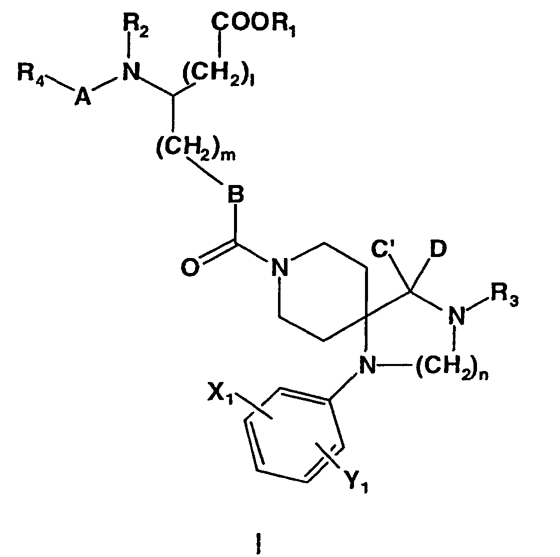 wherein I and m each independently represent an integer of 0 to 2;
n represents an integer of 1 to 3;
A represents -C(O)- or -S(O)₂-;
B represents -CH₂- or -NH-;
C' and D both represent a hydrogen atom, or C' and D represent together =O;
X₁ and Y₁ independently represent hydrogen, halogen, C₁₋₈ alkyl, trifluoromethyl, C₁₋₈ alkoxy, cyano, nitro, hydroxyl, amino, or tetrazolyl;
R₁ represents hydrogen, C₁₋₆ linear alkyl, C₃₋₈ branched alkyl, benzyl or -CH₂OC(O)C(CH₃)₃;
R₂ represents hydrogen or C₁₋₆ linear alkyl;
R₃ represents hydrogen, C₁₋₆ linear alkyl, C₃₋₈ branched alkyl, allyl, homoallyl, C₆₋₁₀ cycloalkylalkyl, unsubstituted phenyl or phenyl substituted with at least one substituent of substituent group E, unsubstituted benzyl or benzyl substituted with at least one substituent of substituent group E, unsubstituted phenethyl or phenethyl substituted with at least one substituent of substituent group E, unsubstituted styryl or styryl substituted with at least one substituent of substituent group E, or unsubstituted naphthylmethyl or naphthylmethyl substituted with at least one substituent of substituent group E (wherein substituent group E consists of halogen, C₁₋₈ alkyl, C₁₋₈ alkoxy, trifluoromethyl, trifluoromethoxy, C₁₋₈ alkylthio, cyano, nitro, hydroxyl, amino, C₁₋₈ alkylacyl, C₁₋₈ alkylacylamino and tetrazolyl);
R₄ represents C₃₋₈ cycloalkyl that may be substituted with 1 to 4 substituents of R₆ or 3- to 8-membered monocyclic or bicyclic heterocycle that includes 1 to 4 nitrogen atoms or oxygen atoms independently selected, which heterocycle may be substituted with 1 to 4 substituents of R₆ (with the proviso that the hetero atoms do not bond directly with A), tetrahydrothiophene or tetrahydrothiopyran that may be substituted with 1 to 4 substituents of R₆, phenyl that may be substituted with 3 to 5 substituents of R₇, naphthyl that may be substituted with 1 to 4 substituents of R₇, 5- to 8-membered monocyclic or bicyclic heteroaryl that includes 1 to 4 nitrogen atoms, oxygen atoms or sulfur atoms independently selected, which heterocycle may be substituted with 1 to 4 substituents of R₇, C₃₋₈ cycloalkyl-C₁₋₈ alkyl, or 3- to 8-membered monocyclic heterocycle-C₁₋₈ alkyl, the heterocycle including 1 to 4 nitrogen atoms or oxygen atoms independently selected and which may be substituted with 1 to 4 substituents of R₆, wherein
R₆ and R₇ are, independently, halogen, trifluoromethyl, -OR^{a}, -SR^{a}, C₁₋₈ alkyl, C₂₋₈ alkenyl, C₂₋₈ alkynyl, C₃₋₈ cycloalkyl-C₁₋₈ alkyl, cyano, nitro, =O, -SO₂R^{b}, -SO₂NR^{c}R^{d}, -C(O)R^{b}, -C(O)OR^{b}, -C(O)NR^{c}R^{d}, -NR^{c}R^{d}, -NR^{c}C(O)R^{b}, -NR^{c}SO₂R^{b}, unsubstituted phenyl or phenyl substituted with at least one substituent of substituent group E, or unsubstituted benzyl or benzyl substituted with at least one substituent of substituent group E,
R³ is hydrogen, C₁₋₈ alkyl, allyl, homoallyl, trifluoromethyl, phenyl or benzyl,
R^{b}, R^{c} and R^{d} are each independent, and are hydrogen, C₁₋₈ alkyl, C₃₋₈ cycloalkyl, C₃₋₈ cycloalkyl-C₁₋₈ alkyl, unsubstituted phenyl or phenyl substituted with at least one substituent of substituent group E, unsubstituted benzyl or benzyl substituted with at least one substituent of substituent group E, unsubstituted phenethyl or phenethyl substituted with at least one substituent of substituent group E, unsubstituted styryl or styryl substituted with at least one substituent of substituent group E, unsubstituted naphthyl or naphthyl substituted with at least one substituent of substituent group E, or unsubstituted naphthylmethyl or naphthylmethyl substituted with at least one substituent of substituent group E.

3. A spiro derivative or a pharmaceutically acceptable salt thereof, wherein in the above-described Formula I,
l, m, n, B, C', D, X₁, Y₁, R₁, R₂, and R₃ are defined in the same manner as in claim 2,
A represents -C(O)- or -S(O)₂-,
R₄ represents C₃₋₈ cycloalkyl that may be substituted with 1 to 4 substituents of R₆ (wherein R₆ is defined in the same manner as in claim 2), phenyl that may be substituted with 3 to 5 substituents of R₇ (wherein R₇ is defined in the same manner as in claim 2), naphthyl that may be substituted with 1 to 4 substituents of R₇ (wherein R₇ is defined in the same manner as in claim 2), Formula IV that may be substituted with 1 to 4 substituents of R₆, 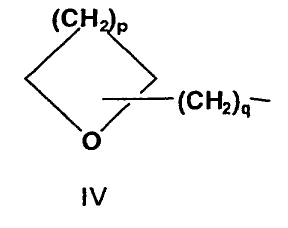 wherein R₆ is defined in the same manner as in claim 2, and p represents an integer of 0 to 5 and q represents an integer of 0 to 2,
Formula V that may be substituted with 1 to 4 substituents of R₆, 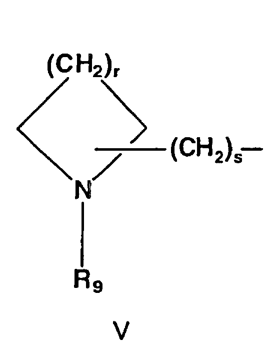 wherein R₆ is defined in the same manner as in claim 2, r represents an integer of 0 to 5 and s represents an integer of 0 to 2, R₉ represents hydrogen, C₁₋₈ alkyl, -SO₂R^{b}, -SO₂NR^{c}R^{d}, -C(O)R^{b}, -C(O)OR^{b}, -C(O)NR^{c}R^{d}, unsubstituted phenyl or phenyl substituted with at least one substituent of substituent group E, unsubstituted benzyl or benzyl substituted with at least one substituent of substituent group E, unsubstituted phenethyl or phenethyl substituted with at least one substituent of substituent group E, unsubstituted styryl or styryl substituted with at least one substituent of substituent group E, unsubstituted naphthyl or naphthyl substituted with at least one substituent of substituent group E, or unsubstituted naphthylmethyl or naphthylmethyl substituted with at least one substituent of substituent group E, and R^{b}, R^{c}, R^{d} and substituent group E are defined in the same manner as in claim 2, or
Formula VI that may be substituted with 1 to 4 substituents of R₆, 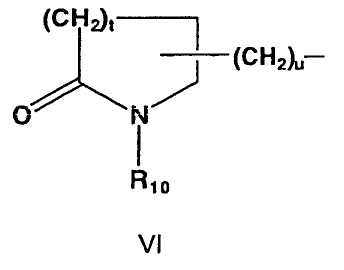 wherein R₆ is defined in the same manner as in claim 2, t represents an integer of 0 to 4 and u represents an integer of 0 to 2, R₁₀ represents hydrogen, C₁₋₈ alkyl, unsubstituted phenyl or phenyl substituted with at least one substituent of substituent group E, unsubstituted benzyl or benzyl substituted with at least one substituent of substituent group E, unsubstituted phenethyl or phenethyl substituted with at least one substituent of substituent group E, unsubstituted styryl or styryl substituted with at least one substituent of substituent group E, unsubstituted naphthyl or naphthyl substituted with at least one substituent of substituent group E, or unsubstituted naphthylmethyl or naphthylmethyl substituted with at least one substituent of substituent group E, and substituent group E is defined in the same manner as in claim 2.

4. A spiro derivative or a pharmaceutically acceptable salt thereof, wherein in the Formula I,
l, m, n, A, B, C', D, X₁, Y₁, R₁, R₂, and R₃ are defined in the same manner as in claim 2, and
R₄ represents Formula IV, Formula V or Formula VI (wherein Formula IV, Formula V and Formula VI are defined in the same manner as in claim 3).

5. A spiro derivative or a pharmaceutically acceptable salt thereof, wherein in the Formula I,
l, m, n, A, B, C', D, X₁, Y₁, R₁, R₂, and R₃ are defined in the same manner as in claim 2, and
R₄ represents Formula IV (wherein p and q are defined in the same manner as in claim 3), Formula V (wherein r and s are defined in the same manner as in claim 3, R₉ represents hydrogen, C₁₋₈ alkyl, -SO₂R^{b}, or -C(O)R^{b}, R^{b} represents C₁₋₈ alkyl, substituted phenyl or phenyl substituted with at least one substituent of substituent group E, substituted benzyl or benzyl substituted with at least one substituent of substituent group E, substituent group E being defined in the same manner as in claim 2), or Formula VI (wherein t and u are defined in the same manner as in claim 3, R₁₀ represents hydrogen, C₁₋₈ alkyl, substituted phenyl or phenyl substituted with at least one substituent of substituent group E, substituted benzyl or benzyl substituted with at least one substituent of substituent group E, substituent group E being defined in the same manner as in claim 2).

6. The spiro derivative or pharmaceutically acceptable salt thereof according to claim 1, wherein in Formula I, A is -C(O)-.

7. The spiro derivative or pharmaceutically acceptable salt thereof according to claim 1, wherein in Formula I, B is -NH-.

8. The spiro derivative or pharmaceutically acceptable salt thereof according to claim 1, wherein in Formula I, C' and D represent together =O.

9. The spiro derivative or pharmaceutically acceptable salt thereof according to claim 1, wherein in Formula I, X₁ and Y₁ are both hydrogen.

10. The spiro derivative or pharmaceutically acceptable salt thereof according to claim 1, wherein in Formula I, n is 1.

11. The spiro derivative or pharmaceutically acceptable salt thereof according to claim 1, wherein in Formula I, is 0.

12. The spiro derivative or pharmaceutically acceptable salt thereof according to claim 1, wherein in Formula I, m is 1 or 2.

13. The spiro derivative or pharmaceutically acceptable salt thereof, according to claims 1, wherein in Formula I, R₃ is hydrogen, C₁₋₆ linear alkyl, C₃₋₈ branched alkyl or benzyl.

14. The spiro derivative or pharmaceutically acceptable salt thereof according to claim 1, wherein in Formula I, R₁ is hydrogen or C₁₋₆ linear alkyl.

15. An adhesion molecule inhibitor comprising the spiro derivative or pharmaceutically acceptable salt thereof according to claim 1 as an active ingredient.

16. The adhesion molecule inhibitor according to claim 15, wherein the adhesion molecule is integrin family.

17. The adhesion molecule inhibitor according to claim 16, wherein the integrin family is VLA-4.

18. A drug comprising the spiro derivative or pharmaceutically acceptable salt thereof according to claim 1 as an active ingredient.

19. An inflammatory disease therapeutic agent comprising the spiro derivative or pharmaceutically acceptable salt thereof according to claim 1 as an active ingredient.

20. The inflammatory disease therapeutic agent according to claim 19, wherein the inflammatory disease is an allergic disease or an autoimmune disease.

21. The inflammatory disease therapeutic agent according to claim 20, wherein the allergic disease is asthma, rhinitis or dermatitis.

22. The inflammatory disease therapeutic agent according to claim 20, wherein the autoimmune disease is multiple sclerosis, ulcerative colitis, arthritis or nephritis.
